(19) Europäisches Patentamt
European Patent Office
Office européen des brevets

(11) EP 2 398 388 B1

(12) **EUROPÄISCHE PATENTSCHRIFT**

(45) Veröffentlichungstag und Bekanntmachung des Hinweises auf die Patenterteilung:
**08.04.2020 Patentblatt 2020/15**

(21) Anmeldenummer: **10705281.3**

(22) Anmeldetag: **12.02.2010**

(51) Int Cl.:
***A61B 5/155*** *(2006.01)* ***A61B 5/157*** *(2006.01)*
***A61B 5/1455*** *(2006.01)* ***G01N 33/487*** *(2006.01)*
***A61B 5/1468*** *(2006.01)*

(86) Internationale Anmeldenummer:
**PCT/EP2010/000865**

(87) Internationale Veröffentlichungsnummer:
**WO 2010/094427 (26.08.2010 Gazette 2010/34)**

(54) **PLATZSPARENDE MAGAZINIERUNG ANALYTISCHER HILFSMITTEL**

COMPACT STORAGE OF AUXILIARY ANALYTICAL DEVICES IN A CARTRIDGE

STOCKAGE PEU ENCOMBRANT DE MOYENS D’ANALYSE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **19.02.2009 EP 09153211**
**20.08.2009 EP 09168335**

(43) Veröffentlichungstag der Anmeldung:
**28.12.2011 Patentblatt 2011/52**

(73) Patentinhaber:
- **Roche Diabetes Care GmbH**
  **68305 Mannheim (DE)**
  Benannte Vertragsstaaten:
  **DE**
- **F. Hoffmann-La Roche AG**
  **4070 Basel (CH)**
  Benannte Vertragsstaaten:
  **AT BE BG CH CY CZ DK EE ES FI FR GB GR HR HU IE IS IT LI LT LU LV MC MK MT NL NO PL PT RO SE SI SK SM TR**

(72) Erfinder:
- **KUHR, Hans-Jürgen**
  **68219 Mannheim (DE)**
- **LIST, Hans**
  **64754 Hesseneck-Kailbach (DE)**
- **LEICHNER, Wilhelm**
  **68307 Mannheim (DE)**
- **KRÄMER, Uwe**
  **68549 Ilvesheim (DE)**

(74) Vertreter: **Altmann Stößel Dick Patentanwälte PartG mbB**
**Dudenstrasse 46**
**68167 Mannheim (DE)**

(56) Entgegenhaltungen:
**EP-A- 2 042 098**
**DE-A1-102005 035 461**
**US-A1- 2004 138 543**
**US-A1- 2006 008 389**
**US-A1- 2007 129 650**
**US-A1- 2007 167 872**
**US-A1- 2008 094 804**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 398 388 B1

**Beschreibung**

Gebiet der Erfindung

Die Erfindung betrifft ein analytisches Magazin zur Aufnahme einer Mehrzahl analytischer Hilfsmittel sowie ein analytisches System, welches eingerichtet ist, um mit einem erfindungsgemäßen analytischen Magazin zusammenzuwirken. Derartige analytische Magazine und analytische Systeme werden in den Naturwissenschaften, der Technik, der Medizin und/oder der medizinischen Diagnostik eingesetzt, um einen oder mehrere Analyte qualitativ oder quantitativ in einer Probe nachzuweisen. Beispielsweise kann es sich bei diesen Proben um Proben von Körperflüssigkeit handeln, wie beispielsweise Urin, Blut, interstitielle Flüssigkeit oder ähnliches. Bei den nachzuweisenden Analyten kann es sich beispielsweise um Metabolite handeln. Ohne Beschränkung weiterer möglicher Einsatzgebiete wird die Erfindung im Folgenden unter Bezugnahme auf den Nachweis von Blutglukose beschrieben. Grundsätzlich sind jedoch auch andere Analyte alternativ oder zusätzlich messbar, wobei der Begriff eines Analyten auch andere messbare Eigenschaften der Probe umfassen kann, wie beispielsweise Koagulate oder ähnliches.

Stand der Technik

Insbesondere aus dem Bereich der medizinischen Diagnostik, jedoch auch aus anderen Bereichen, sind Systeme bekannt, mittels derer sich Analyte in Proben nachweisen lassen. In der medizinischen Diagnostik umfasst ein derartiger Nachweis, beispielsweise von Blutglukose, in der Regel ein Generieren einer Probe von Körperflüssigkeit, beispielsweise Blut oder interstitieller Flüssigkeit, gefolgt von einer Aufnahme dieser Probe und einer qualitativen und/oder quantitativen Analyse. Zu diesem Zweck werden in der Regel ein oder mehrere analytische Hilfsmittel verwendet, welche beispielsweise eine Lanzette und/oder Testelemente umfassen können, mittels derer die Probe generiert und/oder aufgenommen und/oder analysiert werden kann.

Insbesondere die Integration verschiedener Systemfunktionen hat bei derartigen analytischen Systemen, beispielsweise Blutzucker-Messsystemen, in der Vergangenheit zu kommerziellen Lösungen mit auswechselbaren Magazinen für die analytischen Hilfsmittel, beispielsweise Teststeifen-Magazinen, geführt. Typische Vertreter dieser Produktgruppe sind die Systeme Accu-Chek Compact® sowie Ascensia Breeze, welche kommerziell erhältliche Systeme darstellen. Typische zugehörige Magazine fassen in der Regel 17 bzw. 10 Teststeifen.

In neueren Systemen werden dabei in vielen Fällen unterschiedliche Systemfunktionen integriert zusammengefasst, beispielsweise die Systemfunktion einer Probengenerierung (beispielsweise durch Perforation einer Hautpartie) und die Systemfunktion einer Probenaufnahme sowie optional auch die Systemfunktion einer Analyse. Bei der Konzeption höher integrierter Systeme können beispielsweise eine Blutgewinnung und eine Testfunktion zusammengeführt werden. Zu diesem Zweck sind beispielsweise so genannte "Microsampler" bekannt, welche die Funktion einer Lanzette und die Funktion eines Transports der Probe, beispielsweise hin zu einem Testelement, zusammenfassen können. Eine separate Stechhilfe zur Blutentnahme, beispielsweise aus einer Fingerbeere, einem Ohrläppchen oder einer sonstigen Hautpartie eines Probanden, kann somit eingespart werden.

Dabei können mehrere derartiger Microsampler in einem Wechselmagazin unterschiedlichster Bauart untergebracht werden. Grundsätzlich lassen sich, unabhängig von der Art des analytischen Hilfsmittels, drei Haupttypen von Magazinen unterscheiden, nämlich Rundmagazine (beispielsweise in Form von Trommeln und/oder Scheiben), Linearmagazine (beispielsweise in Form von Stapelmagazinen, Zick-Zack-Magazinen oder ähnlichem) und Bandmagazine, bei welchen die analytischen Hilfsmittel auf einem Band oder einer anderen Form von zumindest teilweise flexiblem Träger angeordnet sind. Diese Magazintypen lassen sich grundsätzlich auch im Rahmen der im Folgenden beschriebenen Erfindung einsetzen, bzw. modifizieren. Im Stand der Technik werden Rundmagazine beispielsweise in US 2006/0008389, US 2007/0292314, US 2006/0184064, US 2003/0212347, US 2002/0087056, US2004/0138543 oder US2007/0129650 beschrieben. Linearmagazine sind beispielsweise in US 6,036,924 oder in US 2003/0191415 beschrieben. Bandmagazine werden beispielsweise offenbart in US 2002/0188224, in US 2008/0103415, in EP 1360935 A1 oder in DE 19819407 A1.

Eine Triebkraft für die Integration mehrerer Systemfunktionen ist insbesondere der Wunsch nach einer einfachen und sicheren Bedienung sowie einer Miniaturisierung des gesamten Systems. Da ein häufiger Wechsel des Magazins eher unbequem ist und das Risiko in sich birgt, im entscheidenden Moment kein Reservemagazin zur Hand zu haben, sollte ein Magazin mindestens 25 Testelemente, bevorzugt sogar 50 Testelemente oder mehr, enthalten.

Die aus dem Stand der Technik bekannten Systeme und Magazine beinhalten technisch jedoch einige Herausforderungen. So stellt beispielsweise die Forderung nach einer möglichst geringen Systemgröße, bei gleichzeitig möglichst hoher Anzahl an analytischen Hilfsmitteln je Magazin, beispielsweise Testelementen, einen Zielkonflikt dar. Gerätevolumina unterhalb von 130 ml sind vom Anwender besonders bevorzugt. Kommerziell ist es jedoch bislang nicht gelungen, diese Baugröße mit einem Magazin zu kombinieren, welches beispielsweise 50 Microsampler umfasst.

Rundmagazine, beispielsweise der oben beschriebenen Art, weisen weiterhin den Nachteil auf, dass sich die

Anzahl an analytischen Hilfsmitteln in der Regel unmittelbar auf den Außendurchmesser dieser Rundmagazine auswirkt. Eine hohe Magazinkapazität führt somit entweder zu einem sehr flächigen Gerät (beispielsweise bei Scheibenmagazinen) oder zu einem besonders dicken Gerät (beispielsweise bei Trommelmagazinen). Derartige Geräte entsprechen jedoch in vielen Fällen nicht den Kundenvorstellungen. Bei Linearmagazinen auf der anderen Seite geht die Anzahl der verfügbaren analytischen Hilfsmittel in der Regel unmittelbar in die Länge der Magazine ein. Zudem ist bei diesem Konzept in der Regel eine Magazinverschiebung nach jedem Test erforderlich, so dass der doppelte Platzbedarf im System für das Magazin vorgehalten werden muss. Am ehesten wird die gewünschte Systemgröße durch Bandmagazine erreicht, da die Magazingröße nichtlinear abhängig ist von der Magazinkapazität. Nachteilig an derartigen Systemen ist jedoch in vielen Fällen die hohe mechanische Komplexität, insbesondere bezüglich einer Bandführung und/oder Bandsteuerung.

Aus dem Stand der Technik sind daher Ansätze bekannt, Magazine in Form von so genannten Wendemagazinen vorzuhalten. So wird in US 2006/0264996 ein Linearmagazin für Lanzetten beschrieben, welches verdoppelt ausgestaltet ist, indem zwei derartiger Magazine endseitig aneinandergefügt sind. Nachdem alle Lanzetten eines ersten Endabschnitts des derart erzeugten gesamten Magazins aufgebraucht sind, wird das Magazin ausgeworfen, umgedreht, und die Lanzetten in einem zweiten Endabschnitt werden verwendet.

Nachteilig an derartigen, aus dem Stand der Technik bekannten Wendemagazinen ist jedoch, dass diese ausschließlich für die Verwendung in Stechhilfen ausgestaltet sind. Einem Integrationsgedanken, also einer Verwendung in Systemen, in welchen mehrere Systemfunktionen integriert werden, werden die bekannten Wendemagazine nicht gerecht. Derartige integrierte Systeme, insbesondere Systeme mit so genannten Microsampler-Magazinen, weisen jedoch eine Reihe besonderer Anforderungen auf. So muss der Analyt in der Regel im Magazin detektierbar sein, beispielsweise optisch und/oder elektrochemisch. Weiterhin sollen integrierte Testelemente, beispielsweise Testchemien, hinsichtlich ihrer Beständigkeit eine Gewährleistung aufweisen, so dass eine zuverlässige Aufbrauchfrist angebbar ist. Weiterhin sollte eine Kreuzkontamination der analytischen Hilfsmittel, insbesondere der Microsampler, vermieden werden. Diese Anforderungen sind insgesamt bei bekannten analytischen Systemen und bekannten analytischen Magazinen bislang nicht oder nur unzureichend erfüllt.

Aufgabe der Erfindung

Es ist daher eine Aufgabe der vorliegenden Erfindung, ein analytisches Magazin und ein analytisches System bereitzustellen, welche die Nachteile bekannter Magazine bzw. Systeme zumindest weitgehend vermeiden. Insbesondere soll das analytische Magazin für den Einsatz in Microsampler-Systemen eingerichtet sein.

Offenbarung der Erfindung

Diese Aufgabe wird durch ein analytisches Magazin sowie durch ein analytisches System mit den Merkmalen des Anspruchs 1 gelöst. Vorteilhafte Weiterbildungen der Erfindung, welche einzeln oder in Kombination realisierbar sind, sind in den abhängigen Ansprüchen 2-13 dargestellt.

Es wird ein analytisches Magazin vorgeschlagen, welches eine Mehrzahl von in Kammern aufgenommenen analytischen Hilfsmitteln umfasst, vorzugsweise mindestens 10 analytische Hilfsmittel, insbesondere mindestens 20, mindestens 30, mindestens 40 und besonders bevorzugt mindestens 50 analytische Hilfsmittel oder mehr. Das analytische Magazin dient der Verwendung in einem analytischen System. Unter einem analytischen System ist dabei allgemein ein System zu verstehen, welches mindestens eine analytische Funktion erfüllt. Unter einer analytischen Funktion ist dabei der Nachweis mindestens eines Analyten in einer Probe zu verstehen. Ohne Beschränkung wird dabei im Folgenden davon ausgegangen, dass es sich bei der Probe um eine Probe einer Körperflüssigkeit handelt. Grundsätzlich wären jedoch auch weitere Proben analysierbar. Auch der Begriff des mindestens einen Analyten ist grundsätzlich weit zu fassen und umfasst neben nachzuweisenden Substanzen, beispielsweise eines oder mehrerer Metaboliten, grundsätzlich auch andere messbare Eigenschaften der Probe. Besonders bevorzugt wird die Erfindung jedoch eingesetzt für den Nachweis mindestens eines Metaboliten in einer Körperflüssigkeit, beispielsweise für den Nachweis von Blutglukose, Cholesterin, (Anti-) Koagulantien oder ähnlichem.

Entsprechend ist unter einem analytischen Magazin ein Magazin zu verstehen, welches für den Einsatz in einem derartigen analytischen System ausgestaltet ist. Beispielsweise kann das Magazin zu diesem Zweck ein oder mehrere Gehäuse umfassen, welche in ein oder mehrere Gehäuse des analytischen Systems eingefügt werden können. Zu diesem Zweck können beispielsweise entsprechende Orientierungshilfen, Verbindungselemente, Schienen, Haken, Nuten, Vorsprünge, Transportelemente oder Kombinationen der genannten und/oder anderer Elemente an dem analytischen Magazin vorgesehen sein, welche ein Zusammenwirken des analytischen Magazins mit dem analytischen System ermöglichen oder zumindest vereinfachen. Beispielsweise können Transportelemente und/oder Positionierungshilfen vorgesehen sein, welche eine Positionierung bzw. korrekte Ausrichtung des Magazins innerhalb des analytischen Systems ermöglichen.

Unter einem analytischen Hilfsmittel ist dementsprechend allgemein ein Hilfsmittel zu verstehen, welches eingerichtet ist, um dem analytischen System den Nachweis des mindestens einen Analyten in einer Körperflüssigkeit zu ermöglichen und/oder mit Komponenten des analytischen Systems beim Nachweis des mindestens eines Analyten in der Körperflüssigkeit zumindest teilweise zusammenzuwirken. Insbesondere kann es sich bei den analytischen Hilfsmitteln um Einweg-Hilfsmittel handeln (Disposables), also Hilfsmittel, welche für einen einmaligen Gebrauch eingerichtet sind. Das analytische Magazin umfasst eine Mehrzahl von Kammern, also mindestens zwei Kammern, in welchen jeweils mindestens eines der analytischen Hilfsmittel aufgenommen oder aufnehmbar ist. Beispielsweise kann in jeweils einer Kammer jeweils genau ein analytisches Hilfsmittel aufgenommen sein. Dies kann beispielsweise bei scheibenförmigen oder stangenförmigen analytischen Magazinen der Fall sein. Alternativ können auch mehrere analytische Hilfsmittel in einer Kammer aufgenommen oder aufnehmbar sein. Dies kann beispielsweise bei Bandmagazinen realisiert werden, bei welchen beispielsweise ein Gutwickel mit einer Mehrzahl von noch nicht benutzten analytischen Hilfsmitteln in einer ersten Kammer aufgenommen ist und ein Schlechtwickel, auf dem eine Mehrzahl von benutzten analytischen Hilfsmitteln aufnehmbar ist, in einer zweiten Kammer. Die analytischen Hilfsmittel können gegebenenfalls wiederum aus Teil-Hilfsmitteln zusammengesetzt sein, welche zusammenhängend ausgestaltet sein können, oder welche gegebenenfalls auch unabhängig voneinander ausgebildet sein können, beispielsweise unabhängig voneinander betätigt, bzw. eingesetzt werden können. Die Gesamtheit aller Teile-Hilfsmittel in einer Kammer, welche umgangssprachlich häufig als ein "Test" bezeichnet wird, kann dann insgesamt als ein analytisches Hilfsmittel im Sinne der obigen Definition aufgefasst werden.

Insbesondere können die analytischen Hilfsmittel eine oder mehrere der im Folgenden beschriebenen Arten analytischer Hilfsmittel umfassen, gegebenenfalls auch als Teil-Hilfsmittel. So können die analytischen Hilfsmittel beispielsweise mindestens ein Testelement umfassen, beispielsweise jeweils mindestens ein Testelement, wobei das mindestens eine Testelement mindestens eine Testchemie aufweist, die eingerichtet ist, um bei Anwesenheit eines nachzuweisenden Analyten mindestens eine Eigenschaft zu ändern. Diese Testchemie kann beispielsweise in Form eines oder mehrerer Testfelder ausgestaltet sein. Dabei kann die Testchemie beispielsweise zumindest teilweise in ein Gehäuse des analytischen Magazins integriert sein, insbesondere in eine Wand der Kammern. Beispielsweise kann die Testchemie derart ausgestaltet sein, dass jeweils mindestens ein Testfeld einem Innenraum einer Kammer zuweist. Die Testchemie kann dabei auch für mehrere oder alle Kammern gleichzeitig ausgestaltet sein, beispielsweise in Form eines gemeinsamen Testfeldes für mehrere oder alle Kammern des analytischen Magazins. Beispielsweise kann eine Testchemiescheibe vorgesehen sein, beispielsweise in Form eines Testchemieringes, wobei jeweils mindestens ein Teil der Oberfläche dieser Testchemiescheibe, beispielsweise des Testchemierings, zu den Innenräumen der Kammern weist, so dass diese Bereiche jeweils für sich separate Testfelder in den einzelnen Kammern bilden.

Die mindestens eine Eigenschaft, aus deren Änderung sich auf die Anwesenheit und/oder Abwesenheit des mindestens einen Analyten qualitativ und/oder quantitativ schließen lässt, kann beispielsweise mindestens eine chemisch und/oder physikalisch messbare Eigenschaft umfassen. Beispielsweise kann es sich hierbei um eine elektrochemisch und/oder optisch messbare Eigenschaft handeln, beispielsweise eine Farbänderung oder ähnliches. Beispiele derartiger Testchemien, welche grundsätzlich auch im Rahmen der vorliegenden Erfindung einsetzbar sind, sind insbesondere in J. Hönes et al., Diabetes Technology & Therapeutics, Volume 10, Supplement 1, 2008, Seiten S-10 bis S-26, beschrieben. Weiterhin kann beispielsweise auf WO 2007/012494 A1 verwiesen werden, in welcher besonders feuchtigkeitsstabile Testchemien beschrieben werden. Die in diesen Druckschriften genannten Testchemien können, einzeln oder in Kombination, auch im Rahmen der vorliegenden Erfindung eingesetzt werden. Insbesondere können stark spezifische Testchemien eingesetzt werden, bei welchen der Nachweis spezifisch auf den mindestens einen Analyten reagiert. Auch andere Arten von Testchemien sind grundsätzlich einsetzbar.

Alternativ oder zusätzlich zu Testelementen können die analytischen Hilfsmittel auch eine oder mehrere Lanzetten zum Erzeugen mindestens einer Öffnung in einer Haut eines Probanden umfassen. Unter einer Lanzette ist dabei allgemein eine Vorrichtung zu verstehen, welche, beispielsweise durch einen Einstich und/oder einen Einschnitt, eine derartige Öffnung erzeugt, durch welche einem Gewebe des Probanden die Körperflüssigkeit entnehmbar ist. Beispielsweise können zu diesem Zweck die Lanzetten mindestens eine Spitze und/oder mindestens eine Schneide umfassen. Insbesondere kann es sich bei diesen Lanzetten um runde Lanzetten und/oder um Flachlanzetten handeln, also um Lanzetten, welche aus einem flachen Bauelement, beispielsweise einem Metallblech, herausgearbeitet sind, beispielsweise herausgeätzt sind.

Alternativ oder zusätzlich zu den genannten Hilfsmitteln kann das mindestens eine analytische Hilfsmittel mindestens ein Transferelement zum Aufnehmen einer Probe einer Körperflüssigkeit und/oder zum Transferieren der Probe umfassen, insbesondere zum Transferieren der Probe zu mindestens einem Testelement, beispielsweise der oben beschriebenen Art. Unter einem Transferelement kann dabei beispielsweise ein Element verstanden werden, welches die Probe aufnimmt und dann unter Durchführung einer Bewegung zu dem Testelement transferiert. Alternativ oder zusätzlich können jedoch auch andere Transfermechanismen eingesetzt werden, beispielsweise Transfermechanismen, welche auf Kapillarkräften beruhen. So können, ebenfalls wiederum alternativ oder zusätzlich, die analytischen Hilfsmittel auch ein oder mehrere Kapillarelemente zum Aufnehmen und/oder Transferieren einer Probe einer Körperflüssigkeit umfassen, insbesondere zu dem mindestens einen Testelement. Unter einem Kapillarelement ist dabei allgemein ein

Element zu verstehen, welches unter Ausnutzung einer Kapillarkraft eine Transportwirkung und/oder eine Sammelwirkung vornimmt.

Die genannten Möglichkeiten können auch einzeln oder gruppenweise kombiniert werden. So können die analytischen Hilfsmittel beispielsweise auch einen oder mehrere Microsampler umfassen. Derartige Microsampler können eine räumlich enge Verzahnung einer saugenden Nadel und eines Testelements umfassen. So kann beispielsweise das zu analysierende Blut ohne Zutun eines Anwenders aus einer Wunde bzw. Öffnung auf ein Testelement transferiert werden. Beispielsweise kann hierfür wiederum eine Lanzette, beispielsweise eine Nadel, mit einem saugenden Kapillarkanal verwendet werden. Allgemein sollen die Microsampler mindestens eine Lanzette zum Erzeugen mindestens einer Öffnung in einer Haut eines Probanden sowie mindestens ein Kapillarelement zum Aufnehmen und/oder Transferieren einer Probe einer Körperflüssigkeit umfassen, wobei der Transfer wiederum vorzugsweise zumindest teilweise hin zu mindestens einem Testelement erfolgen kann. Der Transfer kann jedoch zusätzlich durch eine räumliche Bewegung des Microsamplers unterstützt werden, beispielsweise mittels mindestens eines Aktors.

Der Inhalt wird im Folgenden unter Bezugnahme auf die bevorzugte Möglichkeit beschrieben, bei welcher die analytischen Hilfsmittel zumindest teilweise eingerichtet sind, um bei einer Probennahmebewegung eine Probe einer Körperflüssigkeit aufzunehmen und vollständig oder teilweise zu einem Testelement zu transferieren. Unter einer Probennahmebewegung wird dabei allgemein eine Bewegung des analytischen Hilfsmittels und/oder eines Teil-Hilfsmittels verstanden, welche zunächst von dem analytischen System aus hin zur Hautoberfläche des Probanden erfolgt, gefolgt von einer Rückbewegung zum System. Bei der Hinbewegung kann dabei beispielsweise eine Perforation der Haut durchgeführt werden, beispielsweise mittels einer oder mehrerer der genannten Lanzetten. Bereits während der Perforation und/oder der Perforation nachgelagert, kann eine Probenaufnahme erfolgen, beispielsweise eine Probensammlung, beispielsweise durch einfaches Aufbringen der Probe auf ein Transferelement und/oder ein Einsaugen der Probe in das Transferelement, beispielsweise ein Kapillarelement. Anschließend kann eine Rückbewegung der analytischen Hilfsmittel erfolgen, welche ebenfalls noch Bestandteil der Probennahmebewegung sein kann. Bei der Probennahmebewegung, insbesondere bei oder nach der Rückbewegung, kann die Probe zu einem Testelement transferiert werden, welches Bestandteil des analytischen Hilfsmittels sein kann, welches an der Probennahmebewegung teilnimmt und/oder welches auch als zumindest teilweise unbewegliches Teil-Hilfsmittel in den Kammern aufgenommen sein kann. Beispiele werden unten näher beschrieben. Der Transfer der Probe auf das mindestens eine Testelement kann beispielsweise bei, während oder nach einer im Folgenden noch näher beschriebenen Remagazinierung des analytischen Hilfsmittels erfolgen, also einem Vorgang, bei welchem das analytische Hilfsmittel vollständig oder teilweise wieder in einer Kammer, insbesondere der Kammer, welcher es zuvor entnommen worden war, aufgenommen wird.

Bei der Aufnahme der analytischen Hilfsmittel in den Kammern sind grundsätzlich mindestens zwei Prinzipien der Aufnahme in den Kammern denkbar. So kann beispielsweise pro Kammer jeweils ein analytisches Hilfsmittel aufgenommen sein, insbesondere ein analytisches Hilfsmittel, welches mindestens ein Teil-Hilfsmittel in Form eines Testelements mit einer Testchemie umfasst. Optional kann, vorzugsweise zusätzlich, pro Kammer mindestens ein weiteres Teil-Hilfsmittel in Form einer Lanzette und/oder eines Microsamplers vorgesehen sein. Bei diesem Prinzip können die analytischen Hilfsmittel insbesondere nach einer Verwendung in derselben Kammer remagaziniert werden. Alternativ ist jedoch auch eine Remagazinierung in einer anderen Kammer möglich. Dieses erste Prinzip ist insbesondere bei scheibenförmigen oder stangenförmigen Magazinen bevorzugt. Nach einem weiteren Prinzip können mindestens eine erste Kammer für unbenutzte analytische Hilfsmittel und mindestens eine zweite Kammer für benutzte analytische Hilfsmittel vorgesehen sein. In diesem Fall können beispielsweise für eine Benutzung die analytischen Hilfsmittel aus der ersten Kammer entnommen und nach der Benutzung in die zweite Kammer überführt werden, welche räumlich getrennt von der ersten Kammer ausgebildet sein kann. Dieses Prinzip kann beispielsweise für Bandmagazine eingesetzt werden, wobei beispielsweise in einer ersten Kammer ein Gutwickel zur Aufnahme unbenutzter analytischer Hilfsmittel vorgesehen ist und in einer zweiten Kammer ein Schlechtwickel zur Aufnahme benutzter analytischer Hilfsmittel.

In einem ersten Aspekt wird insbesondere vorgeschlagen, das analytische Magazin derart einzurichten, dass dieses in mindestens zwei Orientierungen in dem analytischen System aufgenommen werden kann. Insbesondere kann das analytische Magazin derart eingerichtet sein, dass dieses in den mindestens zwei Orientierungen an dieselbe Schnittstelle des analytischen Systems ankoppeln kann, beispielsweise eine Schnittstelle mit mindestens einer Transportfunktion und/oder mindestens einer Aktorfunktion, beispielsweise zum Durchführen der Probennahmebewegung, und/oder mindestens einer Messfunktion, beispielsweise einer optischen und/oder elektrochemischen Messfunktion.

Zu diesem Zweck kann das analytische Magazin beispielsweise zumindest teilweise mit einer Symmetrie ausgestaltet sein, insbesondere einer Symmetrie um mindestens eine Symmetrieachse. Dementsprechend kann das analytische Magazin beispielsweise als Wendemagazin ausgestaltet sein, welches in den mindestens zwei Orientierungen in das analytische System einfügbar ist und dort vorzugsweise in allen Orientierungen an dieselbe Schnittstelle ankoppeln kann.

Unter einer Orientierung ist dabei allgemein vorzugsweise eine Winkelorientierung des analytischen Magazins, beispielsweise relativ zu einem Koordinatensystem des analytischen Systems, zu verstehen, also eine Orientierung hinsichtlich einer oder mehrerer Winkelkoordinaten, beispielsweise Raumwinkelkoordinaten. Alternativ oder zusätzlich

kann unter einer Orientierung jedoch auch eine absolute Orientierung verstanden werden, beispielsweise unter Verwendung einer oder mehrerer kartesischer Koordinaten. Besonders bevorzugt ist jedoch die Ausgestaltung des Magazins als Wendemagazin, beispielsweise derart, dass das analytische Magazin in einer ersten Orientierung dem analytischen System entnehmbar ist, dann um einen oder mehrere Winkel gedreht werden kann, um dann in mindestens einer zweiten, von der ersten Orientierung verschiedenen Orientierung wieder in dem analytischen System aufgenommen werden zu können. Insbesondere kann es sich bei der Drehung um eine Drehung um 180° um eine Achse handeln, optional gefolgt von mindestens einer weiteren Drehung, beispielsweise einer Winkelversatz-Drehung um eine zur ersten Achse um 90° gedrehten Achse, beispielsweise um einen unten näher beschriebenen Versatzwinkel. Das Magazin kann beispielsweise vollständig als Wendemagazin ausgestaltet sein, so dass sich die beiden Orientierungen um 180° bezüglich einer Drehachse voneinander unterscheiden.

Insbesondere kann das analytische Magazin derart ausgestaltet sein, dass dieses, abgesehen von den gewünschten mindestens zwei voneinander verschiedenen Orientierungen, keine weiteren Orientierungen zulässt. Beispielsweise können, wie oben dargestellt, ein oder mehrere Elemente vorgesehen sein, welche das Einfügen des analytischen Magazins in genau den vorgesehenen Orientierungen in das analytische System ermöglichen, nicht jedoch in anderen Orientierungen. Beispielsweise kann ein Gehäuse des analytischen Magazins derart ausgestaltet sein, dass dieses ein Einfügen in das analytische System lediglich in den vorgesehenen Orientierungen ermöglicht, nicht hingegen in anderen Orientierungen. Zu diesem Zweck kann das Gehäuse beispielsweise eine entsprechende Form aufweisen, beispielsweise eine symmetrische Form im Sinne der obigen Definition, beispielsweise eine Symmetrie hinsichtlich einer oder mehrerer Drehachsen. Das Gehäuse kann auch beispielsweise entsprechende Elemente aufweisen, wie beispielsweise Nuten, Schienen, Vorsprünge, Rillen oder ähnliche Elemente, welche ein Einfügen des analytischen Magazins in einer nicht vorgesehenen Orientierung in das analytische System verhindern.

Das analytische Magazin ist also eingerichtet, um in den unterschiedlichen Orientierungen jeweils eine Mehrzahl analytischer Hilfsmittel an das analytische System bereitzustellen, beispielsweise indem in jeder Orientierung eine Mehrzahl von Kammern mit jeweils mindestens einem analytischen Hilfsmittel an das analytische System bereitgestellt werden können. Diese analytischen Hilfsmittel können beispielsweise nacheinander bereitgestellt werden, beispielsweise durch einen entsprechenden Transportmechanismus des analytischen Systems, welcher nacheinander die jeweiligen in der Orientierung verfügbaren Kammern in eine Applikationsposition bringt. Damit unterscheidet sich das analytische Magazin beispielsweise von einfachen Trommelmagazinen, welche nach einem Weitertakten der Trommel zur nächsten Kammer zwar ebenfalls in einer unterschiedlichen Orientierung angeordnet sind, allerdings in einer Orientierung, in welcher nur ein analytisches Hilfsmittel bereitgestellt wird.

Unter einer Orientierung ist somit eine Orientierung des analytischen Magazins als Ganzes zu verstehen, in welcher aus dem zu dieser Orientierung gehörigen Teil-Magazin analytische Hilfsmittel an das analytische System bereitgestellt werden können. Eine Orientierung ist dabei jeweils vorzugsweise genau einem Teil-Magazin zugeordnet. Jede Orientierung kann sich dabei in mehrere Unter-Orientierungen unterteilen, beispielsweise Winkelstellungen einer Magazinscheibe, welche beim Weitertakten nacheinander eingenommen werden können. Diese Unter-Orientierungen sind jedoch nach wie vor einer Orientierung des analytischen Magazins zugeordnet, beispielsweise einer Orientierung, in welcher sich ein Teil-Magazin in einer Applikationsebene und/oder einer Applikationsposition des analytischen Systems befindet. Erst nachdem die Orientierung gewechselt wurde, beispielsweise durch ein Wenden des analytischen Magazins durch eine Drehung um 180°, gelangt ein anderes Teil-Magazin in die Applikationsebene bzw. Applikationsposition und kann seine analytischen Hilfsmittel an das analytische System bereitstellen.

Mittels der analytischen Hilfsmittel ist mindestens eine Probennahmebewegung gemäß der obigen Definition durchführbar. Insbesondere kann das analytische System eine oder mehrere Applikationspositionen umfassen, wobei das analytische System eingerichtet sein kann, um jeweils die mindestens eine gerade in der Applikationsposition befindliche Kammer bzw. das mindestens eine analytische Hilfsmittel in dieser Kammer für die Probennahmebewegung zu verwenden. Beispielsweise kann das analytische System einen oder mehrere Aktoren umfassen, mittels derer die Probennahmebewegung durchführbar ist. Bei der Probennahmebewegung kann das ganze analytische Hilfsmittel bewegt werden und/oder auch ggf. nur mindestens ein Teil-Hilfsmittel. Umfassen die analytischen Hilfsmittel beispielsweise mindestens eine Lanzette und/oder mindestens einen Microsampler und/oder mindestens ein Transferelement, so können diese Lanzetten bzw. Microsampler bzw. Transferelemente für die Probennahmebewegung eingesetzt werden, wohingegen weitere Teil-Hilfsmittel der analytischen Hilfsmittel in der Kammer verbleiben können, beispielsweise ein oder mehrere Testelemente. Auch andere Ausgestaltungen sind jedoch möglich, beispielsweise Ausgestaltungen, bei welchen Testelemente ganz oder teilweise an der Probennahmebewegung teilnehmen. Im Folgenden wird jedoch eine Ausführung beschrieben, bei welcher die Testelemente innerhalb der Kammern verbleiben, wohingegen Microsampler die Probennahmebewegung durchführen und eine Probe der Körperflüssigkeit in die Kammern hinein zu den Testelementen transferieren.

Das analytische Magazin ist dabei in dem ersten Aspekt eingerichtet, um eine Remagazinierung der analytischen Hilfsmittel zu ermöglichen. Unter einer Remagazinierung ist dabei ein Vorgang zu verstehen, bei welchem die analytischen Hilfsmittel, vorzugsweise während oder nach einer vollständigen Durchführung der Probennahmebewegung, wieder in dieselbe Kammer, welcher diese auch entnommen worden sind, oder, alternativ oder zusätzlich, in eine separate Kammer, vollständig oder teilweise zurück überführt werden. Allgemein ist das analytische Magazin also derart ausgestaltet, dass eine derartige Rückführung technisch möglich ist und nicht beispielsweise durch Widerhaken oder ähnliche Gestaltungen verhindert wird.

Zum Zweck der Remagazinierung können beispielsweise die analytischen Hilfsmittel auch eingerichtet sein, um mit dem analytischen System derart zusammenzuwirken, dass eine derartige Remagazinierung ermöglicht wird. So können die analytischen Hilfsmittel beispielsweise mindestens ein Kopplungselement aufweisen, mittels dessen eine Remagazinierung der analytischen Hilfsmittel ermöglicht wird, insbesondere eine Remagazinierung in die Kammern, welcher die jeweiligen analytischen Hilfsmittel entnommen worden sind. Beispielsweise können diese Kopplungselemente ein oder mehrere Nuten, Haken, Vorsprünge, Vertiefungen, Ösen, Pilotöffnungen oder sonstige Kopplungselemente oder Kombinationen der genannten und/oder andere Kopplungselemente aufweisen. Derartige Kopplungselemente können beispielsweise dem Stand der Technik entnommen werden. So kann beispielsweise auf die in US 2006/0008389 A1 beschriebenen Kopplungselemente verwiesen werden, bei welchen ein Aktor mittels eines oder mehrerer Haken an die analytischen Hilfsmittel ankoppelt und anschließend wieder von diesen abkoppelt, beispielsweise für eine Probennahmebewegung und eine anschließende Remagazinierung. Derartige Kopplungselemente können auch im Rahmen der vorliegenden Erfindung eingesetzt werden. Auch andere Arten von Kopplungen können jedoch grundsätzlich eingesetzt werden, beispielsweise eine reibschlüssige oder formschlüssige Ankopplung eines Aktors an das analytische Hilfsmittel und/oder Teil-Hilfsmittel, beispielsweise mittels eines Greifers oder einer ähnlichen Vorrichtung.

An die Kopplungselemente können insbesondere einer oder mehrere Aktoren angekoppelt werden, wie beispielsweise Kopplungsstangen, welche Bestandteil des analytischen Systems sein können, und welche sowohl einen Vortrieb der analytischen Hilfsmittel als auch einen Rücktrieb der analytischen Hilfsmittel im Rahmen einer Probennahmebewegung, mit anschließender Remagazinierung, bewirken. Beispielsweise können entsprechende Kopplungsstangen, Stößel oder ähnliches vorgesehen sein.

Das analytische Magazin ist in dem ersten Aspekt eingerichtet, um die analytischen Hilfsmittel beziehungsweise Teil-Hilfsmittel nach der Remagazinierung in den Kammern zu haltern. Unter einer Halterung ist dabei ein Vorgang zu verstehen, bei welchem ein Herausfallen oder Herausgleiten der analytischen Hilfsmittel aus den Kammern, beispielsweise durch bei der Probennahmebewegung geschaffene Öffnungen in einer Versiegelung der Kammern, zumindest weitgehend verhindert wird. Zu diesem Zweck können beispielsweise in dem analytischen Magazin und/oder an dem analytischen Hilfsmittel bzw. Teil-Hilfsmittel ein oder mehrere Halteelemente vorgesehen seien, beispielsweise jeweils mindestens ein Halteelement pro Kammer, welche eine derartige Halterung nach der Remagazinierung bewirken. Beispielsweise können diese Halteelemente Haken, Engstellen, Vorsprünge, Ösen, Sperren oder ähnliche Elemente oder Kombinationen der genannten und/oder anderer Elemente umfassen. Dabei können auch jeweils Teil-Halteelemente an den analytischen Hilfsmitteln oder Teil-Hilfsmitteln vorgesehen sein, welche mit entsprechenden Teil-Halteelementen der Kammern zusammenwirken.

Alternativ oder zusätzlich zur Verwendung von einem oder mehreren Halteelementen kann die Halterung der analytischen Hilfsmittel nach der Remagazinierung auch auf andere Weise realisiert werden. Beispielsweise können die analytischen Hilfsmittel auch zumindest teilweise formschlüssig und/oder kraftschlüssig gehaltert werden. Dieser Formschluss und/oder Kraftschluss kann beispielsweise auch erst bei der Remagazinierung und/oder nach der Remagazinierung erfolgen. So kann beispielsweise bei der Remagazinierung das analytische Hilfsmittel und/oder Teil-Hilfsmittel in eine Remagazinierungsposition in dem Magazin, beispielsweise in der Kammer, gebracht werden, in welcher erst dieser Kraftschluss und/oder Formschluss auftritt. Diese Remagazinierungsposition kann identisch oder verschieden zu einer Position sein, in welcher die analytischen Hilfsmittel und/oder Teil-Hilfsmittel vor der Probennahmebewegung sind. Der Formschluss und/oder Kraftschluss kann beispielsweise auch mit einer Verformung der analytischen Hilfsmittel und/oder Teil-Hilfsmittel verbunden sein. Beispielsweise kann die Kammer derart gestaltet sein, dass das analytische Hilfsmittel und/oder Teil-Hilfsmittel, beispielsweise eine Lanzette und/oder ein Microsampler, in der Kammer gebogen wird und vorzugsweise durch eine Rückstellkraft gegen eine Kammerwand gepresst wird. Auf diese Weise kann ein Kraftschluss, insbesondere ein Reibschluss, realisiert werden. Beispielsweise kann die Kammer gekrümmt ausgestaltet sein, so dass das analytische Hilfsmittel und/oder Teil-Hilfsmittel in der Kammer gebogen wird. Die Lanzette bzw. der Microsampler kann beispielsweise ganz oder teilweise aus einem elastischen Material hergestellt sein, beispielsweise einem metallischen Werkstoff, beispielsweise einem Metallblech, welches die oben genannte Rückstellkraft bereitstellen kann. Alternativ oder zusätzlich kann auch die Kammer beispielsweise mit einer elastischen Wand ausgestaltet sein, welche bei der Remagazinierung durch das analytische Hilfsmittel und/oder Teil-Hilfsmittel verformt wird, wobei Rückstellkräfte der Kammerwand den genannten Kraftschluss, insbesondere den Reibschluss, bewirken können. Auch auf diese Weise kann also durch ein Anpassen der Kammergeometrie und/oder der Geometrie des analytischen Hilfsmittels und/oder eine geeignete Auswahl von Materialien des analytischen Hilfsmittels und/oder des Gehäuses eine entsprechende Halterung des remegazinierten analytischen Hilfsmittels bewirkt werden. Auch Kombinationen der genannten Möglichkeiten und/oder anderer Möglichkeiten zur Halterung sind denkbar.

Das analytische Magazin umfasst in dem ersten Aspekt mindestens zwei im Wesentlichen gleiche Teil-Magazine, wobei jedes Teil-Magazin eine Mehrzahl analytischer Hilfsmittel, beispielsweise eine Mehrzahl gleichartiger analytischer Hilfsmittel, umfasst, welche beispielsweise jeweils in einzelnen Kammern aufgenommen sein können. Unter im Wesentlichen gleichen Teil-Magazinen sind dabei Magazine zu verstehen, welche derart gleich ausgestaltet sind, dass diese Teil-Magazine in den oben genannten möglichen unterschiedlichen Orientierungen des analytischen Magazins einander ersetzen können. Jedes Teil-Magazin kann beispielsweise eine bestimmte Orientierung des analytischen Magazins vorsehen, welche auch als Applikationsorientierung bezeichnet werden kann und in welcher das jeweilige Teil-Magazin mit dem analytischen System zusammenwirken kann. Beispielsweise kann das analytische System eine Applikationsposition aufweisen, wobei in der Applikationsorientierung jeweils eines der Teil-Magazine in der Applikationsposition und/oder Applikationsebene des analytischen Systems angeordnet ist und/oder analytische Hilfsmittel oder Teil-Hilfsmittel an das analytische System bereitstellen kann. Die Teil-Magazine weisen nicht notwendigerweise, jedoch vorzugsweise, jeweils dieselbe Anzahl an analytischen Hilfsmitteln auf.

Beispielsweise können zwei übereinander gestapelte Teil-Magazine vorgesehen sein. Die Teil-Magazine können beispielsweise nach einer Drehung um 180° und optional mindestens einer weiteren Drehung um eine andere Achse, beispielsweise eine Winkelversatzdrehung, ein Wiedereinfügen des Magazins in das analytische System ermöglichen. Die Teil-Magazine können beispielsweise in unterschiedlichen Ebenen angeordnet sein, beispielsweise in Form von übereinander gestapelten Teil-Magazinen. Diese Stapelung soll derart erfolgen, dass nach dem Wechsel von einer möglichen Orientierung in eine andere mögliche Orientierung das jeweils zu dieser Orientierung gehörige Teil-Magazin mit dem analytischen System zusammenwirken kann, beispielsweise mit ein und derselben, vorzugsweise für alle Orientierungen geeigneten Schnittstelle des analytischen Systems. Damit unterscheidet sich das analytische Magazin beispielsweise von Magazinen, bei denen einfache Testelemente nebeneinander angeordnet sind.

Das analytische Magazin kann auf verschiedene Weisen vorteilhaft weiter ausgestaltet werden. So kann das analytische Magazin, wie oben bereits beschrieben, zumindest teilweise mit einer Symmetrie ausgestaltet sein, insbesondere einer Symmetrie um mindestens eine Symmetrieachse.

Das analytische Magazin kann insbesondere mindestens ein Anzeigeelement aufweisen, vorzugsweise eine Mehrzahl von Anzeigeelementen, deren Anzahl beispielsweise der Anzahl der möglichen Orientierung entsprechen kann. Das Anzeigeelement kann ausgestaltet sein, um von dem analytischen System erfasst zu werden und mindestens eine Information über eine aktuelle Orientierung an das analytische System zu liefern. Das Anzeigeelement kann beispielsweise mindestens ein einfaches, unveränderliches Element umfassen, welches von dem analytischen System erfasst werden kann. Alternativ oder zusätzlich kann das Anzeigeelement jedoch auch ein oder mehrere veränderliche Elemente umfassen, welche beispielsweise von dem analytischen System verändert werden können, beispielsweise Folienelemente, welche nach Verwendung einer Kammer durchstoßen werden können, von dem analytischen System beschreibbare magnetische Speicherelemente oder ähnliches.

Die analytischen Hilfsmittel können in dem analytischen Magazin in mindestens zwei Hilfsmittelebenen angeordnet sein, wobei nach einem Wechsel der Orientierung in dem analytischen System jeweils eine neue Hilfsmittelebene zumindest teilweise in einer Applikationsebene des analytischen Systems angeordnet ist. Unter einer Applikationsebene kann dabei eine Ebene des analytischen Systems verstanden werden, welche mindestens eine Applikationsposition aufweist, also eine Position, in welcher ein analytisches Hilfsmittel von dem analytischen System eingesetzt werden kann, beispielsweise für eine Lanzettenbewegung, eine Probennahmebewegung, eine Messung einer Analytkonzentration oder ähnliche Zwecke. Unter einer Hilfsmittelebene ist eine gemeinsame Ebene einer Ansammlung von in derselben Orientierung verwendbaren analytischen Hilfsmitteln zu verstehen, welche beispielsweise tatsächlich in derselben Ebene angeordnet sein können oder auch leicht zu dieser Ebene versetzt.

Das analytische Magazin kann dabei grundsätzlich eine oder mehrere der oben bereits beschriebenen Grundformen und/oder auch andere Formen aufweisen, beispielsweise eine Rundmagazin-Form (beispielsweise in Form einer Trommel und/oder einer Scheibe), eines Linearmagazins (beispielsweise eines Stapelmagazins und/oder eines Zick-Zack-Magazins) und/oder eines Bandmagazins. Diesbezüglich kann beispielsweise auf den Stand der Technik verwiesen werden, wobei die dort beschriebenen Systeme zu Wendemagazinen bzw. zu analytischen Magazinen, welche in mindestens zwei Orientierungen in dem analytischen System aufgenommen werden können, modifiziert werden können. Besonders bevorzugt ist es, wenn das analytische Magazin in Form eines Rundmagazins ausgestaltet ist, insbesondere in Form einer runden Scheibe, wobei die analytischen Hilfsmittel vorzugsweise in dem analytischen Magazin zumindest näherungsweise in radialer Anordnung ausgerichtet sind. Bei derartigen Scheibenmagazinen, welche beispielsweise die analytischen Hilfsmittel in mindestens zwei Hilfsmittelebenen umfassen können, machen sich die Vorteile der Wendemagazine besonders ausgeprägt bemerkbar, da, ohne dass ein Durchmesser der runden Scheibe vergrößert werden müsste, die Magazinkapazität erhöht werden kann. Da in der Regel die Dicke der Scheibe im Vergleich zu deren Durchmesser erheblich kleiner ist, machen sich die Vergrößerungen der Dicke, beispielsweise durch das Vorsehen der mehreren Hilfsmittelebenen, unter Design-Aspekten weniger stark bemerkbar als die Vergrößerung eines Radius der Scheibe. Auch andere analytische Magazine als scheibenförmige Magazine lassen sich jedoch grundsätzlich entsprechend ausgestalten, beispielsweise Stangenmagazine und/oder Reihenmagazine.

Wird das analytische Magazin vollständig oder teilweise als Rundmagazin ausgestaltet, insbesondere als Rundmagazin in Form einer runden Scheibe, so ist es besonders bevorzugt, die analytischen Hilfsmittel in unterschiedlichen Hilfsmittelebenen winkelversetzt zueinander anzuordnen. Beispielsweise können in einer ersten Hilfsmittelebene die analytischen Hilfsmittel äquidistant radial ausgerichtet sein, ebenso wie in einer zweiten, von der ersten Hilfsmittelebene verschiedenen Hilfsmittelebene. In diesem Fall ist es besonders bevorzugt, wenn der genannte Winkelversatz stattfindet, so dass beispielsweise zwischen zwei benachbarten Hilfsmitteln einer ersten Hilfsmittelebene in einer Projektion auf die zweite Hilfsmittelebene ein analytisches Hilfsmittel winkelversetzt genau zwischen den beiden analytischen Hilfsmitteln der ersten Hilfsmittelebene angeordnet ist. Auf diese Weise kann beispielsweise gewährleistet sein, dass sämtliche Hilfsmittel aller Hilfsmittelebenen aus zwei Richtungen senkrecht zu den Hilfsmittelebenen, welche vorzugsweise parallel zueinander angeordnet sind, zugänglich sind, ohne dass dieser Zugang durch analytische Hilfsmittel in anderen Hilfsmittelebenen blockiert wäre.

Allgemein ist es also besonders bevorzugt, die unterschiedlichen Hilfsmittelebenen parallel zueinander anzuordnen. Weiterhin ist es allgemein, auch bei anderen als den beschriebenen Ausführungsformen, bevorzugt, wenn das analytische Magazin derart eingerichtet ist, dass in allen möglichen Orientierungen des analytischen Magazins in dem analytischen System ein Zugang zu einer in einer Applikationsposition des analytischen Systems befindlichen Kammer und/oder mindestens eines in dieser Kammer aufgenommenen analytischen Hilfsmittels aus mindestens zwei Richtungen, insbesondere voneinander gegenüberliegenden Richtungen aus, ermöglicht ist, insbesondere aus Richtungen senkrecht zu den Hilfsmittelebenen.

Insbesondere mittels einer oder mehrerer der oben beschriebenen Ausgestaltungen, jedoch auch auf andere Weisen, lassen sich analytische Magazine mit einer hohen Packungsdichte herstellen. Insbesondere lassen sich derartig hohe Packungsdichten bei den genannten Rundmagazinen, insbesondere den genannten scheibenförmigen Magazinen, insbesondere mit radialer Ausrichtung, erzeugen. Im Gegensatz zum Stand der Technik, in welchem derartige Erhöhungen der Packungsdichten in der Regel mit einer Erhöhung des Radius verbunden sind, kann dies bei gleich bleibendem Radius erfolgen. Insbesondere können auf diese Weise analytische Magazine erzeugt werden, welche eine oder mehrere der folgenden Eigenschaften aufweisen: Ein Gesamtvolumen von nicht mehr als 10 $cm^3$, vorzugsweise von nicht mehr als 8 $cm^3$ und besonders bevorzugt von nicht mehr als 7,5 $cm^3$; einen Außenradius von nicht mehr als 5 cm, vorzugsweise von nicht mehr als 3 cm und besonders bevorzugt von nicht mehr als 2,5 cm; einen Innenradius zwischen 0,5 cm und 2 cm, insbesondere zwischen 1,0 cm und 1,5 cm und besonders bevorzugt von etwa 1,2 cm; eine Dicke von nicht mehr als 1 cm, besonders bevorzugt von nicht mehr als 0,5 cm; eine Anzahl an analytischen Hilfsmitteln zwischen 10 und 100, insbesondere zwischen 20 und 70 und besonders bevorzugt von 50; ein Volumen zwischen 3 $cm^3$ und 30 $cm^3$, insbesondere zwischen 5 $cm^3$ und 10 $cm^3$ und besonders bevorzugt von 7,5 $cm^3$; eine Packungsdichte der analytischen Hilfsmittel von mehr als 5/$cm^3$, insbesondere zwischen 5/$cm^3$ und 10/$cm^3$, beispielsweise zwischen 6/$cm^3$ und 7/$cm^3$ und besonders bevorzugt von 6,7/$cm^3$. Beispielsweise kann das analytische Magazin als kreisscheibenförmiges Magazin ausgestaltet sein, mit einem Außenradius von circa 2,5 cm, einen Innenradius von circa 1,2 cm, einer Dicke von circa 0,5 cm, einer Anzahl an analytischen Hilfsmitteln von 50 und damit einem Volumen von 7,5 $cm^3$ und einer Packungsdichte von 6,7/$cm^3$.

Unter einer Packungsdichte ist dabei ein Verhältnis aus der Anzahl der analytischen Hilfsmittel und/oder einer Anzahl der Kammern zum Bauvolumen des analytischen Magazins zu verstehen. Beispielsweise kann die Packungsdichte bei einer Wendescheibe mit 50 mm Durchmesser bei 50 analytischen Hilfsmitteln / 5,97 $cm^3$ (d.h. beispielsweise 50 Kammern) liegen, im Vergleich zu einer einfachen Scheibe mit 50 mm Durchmesser und ca. 3 mm Dicke, welche beispielsweise eine Packungsdichte von 50 analytischen Hilfsmitteln / 5,42 $cm^3$ aufweisen kann. Die analytischen Hilfsmittel werden dabei häufig auch als "Tests" bezeichnet, unabhängig von deren Funktion und Ausgestaltung. Unter einem Test kann dabei also allgemein mindestens ein analytisches Hilfsmittel verstanden werden, welches für einen Testvorgang genutzt werden kann. Dabei kann es sich beispielsweise um ein Testelement oder um eine Lanzette oder auch um ein Paar aus einem Testelement und einer Lanzette handeln, welche zusammengehören und für eine gemeinsame Probennahme mit anschließender Analyse genutzt werden können, wobei vorzugsweise genau ein Test in genau einer Kammer gelagert ist. Ein analytisches Hilfsmittel bzw. ein Test kann also beispielsweise mehrere zusammengehörige Teil-Hilfsmittel umfassen. Besonders bevorzugt ist es, wenn jedes analytische Hilfsmittel mindestens ein analytisches Teil-Hilfsmittel in Form eines Testelements mit einer Testchemie umfasst. Alternativ oder vorzugsweise zusätzlich kann jedes analytische Hilfsmittel weiterhin ein analytisches Teil-Hilfsmittel in Form einer Lanzette und/oder eines Microsamplers umfassen. Jeweils ein analytisches Hilfsmittel bzw. ein Test kann beispielsweise in genau einer Kammer aufgenommen sein. Es wird hier und im Folgenden jedoch zwischen einem Test und einem analytischen Hilfsmittel sprachlich und inhaltlich nicht weiter unterschieden, einschließlich der Möglichkeit, dass ein Test bzw. ein analytisches Hilfsmittel mehrere Teil-Hilfsmittel umfassen kann, beispielsweise jeweils ein Testelement und eine Lanzette. Die Wendescheibe kann beispielsweise eine Dicke von 5 mm und einen Durchmesser von 42 mm aufweisen. In den genannten Fällen sind jeweils die zentralen Löcher im Magazin mit berücksichtigt, da dieser Bauraum auch für eine Gerätemechanik mitverwendet werden kann.

Wie oben dargestellt, können die analytischen Hilfsmittel insbesondere zumindest teilweise als so genannte

Microsampler ausgestaltet sein, also als analytische Hilfsmittel, welche sowohl eine Erzeugung als auch eine Aufnahme der Probe sowie optional auch eine Analyse der Probe ermöglichen sollen. Zu diesem Zweck können die Microsampler beispielsweise mindestens eine Lanzette und mindestens ein Kapillarelement umfassen. Allgemein können die analytischen Hilfsmittel zumindest teilweise eingerichtet sein, um bei der Probennahmebewegung eine Probe einer Körperflüssigkeit aufzunehmen und zu einem Testelement zu transferieren, wobei der Transfer der Probe insbesondere zumindest teilweise während der Remagazinierung des analytischen Hilfsmittels erfolgen kann.

Die analytischen Hilfsmittel können, wie oben dargestellt, insbesondere eine Testchemie aufweisen, welche eingerichtet sein kann, um bei Anwesenheit eines nachzuweisenden Analyten mindestens eine Eigenschaft zu ändern. Beispielsweise kann auf die oben beschriebenen Arten von bekannten Testchemien verwiesen werden, welche auch im Rahmen der vorliegenden Erfindung einsetzbar sind. Insbesondere kann diese Testchemie in Form mindestens eines Testfeldes enthalten sein. Insbesondere können derartige Testfelder zumindest teilweise in den Kammern aufgenommen sein. Beispielsweise können die Testfelder ganz oder teilweise in die Kammern eingebracht sein und/oder auch in die Kammern integriert sein, beispielsweise in die Kammerwände. Beispielsweise können in den Kammerwänden und/oder auf und/oder in in den Kammerwänden aufgenommenen Öffnungen Testfelder aufgenommen sein, deren Oberflächen jeweils dem Inneren der Kammern zuweisen bzw. vom Inneren der Kammern aus zugänglich sind. Das analytische System kann den Transfer der Probe auf diese Testfelder unterstützen, beispielsweise indem ein oder mehrere Aktoren vorgesehen sind, welche Microsampler und/oder Lanzetten und/oder Transferelemente, nachdem diese die Probe aufgenommen haben, auf das mindestens eine Testfeld drücken und/oder in die Nähe dieses mindestens einen Testfeldes bringen, so dass die Probe auf dieses mindestens eine Testfeld übertragen wird. Verschiedene Ausführungsbeispiele werden unten näher beschrieben.

Das analytische Magazin kann insbesondere derart ausgestaltet sein, dass dieses in jeder der möglichen Orientierungen eine Mehrzahl analytischer Hilfsmittel, beispielsweise eine Mehrzahl gleichartiger analytischer Hilfsmittel, bereitstellt, beispielsweise in jeder Orientierung eine gleiche Anzahl analytischer Hilfsmittel. Wie oben dargestellt, ist das analytische Magazin vorzugsweise derart ausgestaltet, dass die unterschiedlichen Teil-Magazine in ihrer jeweiligen Applikationsorientierung mit derselben Schnittstelle des analytischen Systems zusammenwirken können, so dass für die unterschiedlichen Teil-Magazine keine unterschiedlichen Schnittstellen im analytischen System vorgehalten werden müssen. Die Schnittstelle kann beispielsweise eine mechanische Schnittstelle, beispielsweise für ein Weitertakten der einzelnen Kammern jedes Teil-Magazins (beispielsweise durch eine Drehung und/oder eine lineare Verschiebung) und/oder für eine mechanische Ankopplung an das analytische Magazin und/oder die analytischen Hilfsmittel, beispielsweise zum Zweck einer Durchführung einer Probennahmebewegung, und/oder eine Messschnittstelle, beispielsweise eine optische und/oder eine elektrische Messschnittstelle, umfassen. Das analytische System, insbesondere die Schnittstelle, kann mindestens ein Messsystem aufweisen, welches eingerichtet sein kann, um mit den Testelementen für einen Nachweis des mindestens einen Analyten zusammenzuwirken. Das analytische Magazin kann dann derart eingerichtet sein, dass die Testelemente nach einem Wechsel der Orientierung mit demselben Messsystem zusammenwirken, ohne dass dieses an das neu orientierte Magazin wesentlich angepasst werden müsste. Beispielsweise kann eine bestimmte Applikationsposition und/oder Applikationsebene des analytischen Systems vorgesehen sein, wobei das Messsystem mit dem analytischen Hilfsmittel (beispielsweise einem Testelement und/oder Testfeld) der Kammer desjenigen Teil-Magazins, welches gerade in der Applikationsposition ist, zusammenwirken kann. So kann beispielsweise ein und derselbe Detektor für alle Testelemente und/oder Testfelder sämtlicher Kammern sämtlicher Teil-Magazine verwendet werden.

Das Messsystem kann der Ausgestaltung des analytischen Magazins und/oder der Testelemente angepasst sein. Handelt es sich bei diesen Testelementen um optische Testelemente, also Testelemente, welche unter Zusammenwirkung mit dem mindestens einen Analyten mindestens eine optisch nachweisbare Eigenschaft ändern, so kann es sich beispielsweise um ein optisches Messsystem handeln. Ein derartiges optisches Messsystem kann beispielsweise mindestens einen optischen Detektor umfassen, mittels dessen die optische Eigenschaft nachgewiesen werden kann. Auch zusätzliche Elemente können umfasst sein, beispielsweise eine oder mehrere Beleuchtungsvorrichtungen und/oder ein oder mehrere optische Systeme. Alternativ oder zusätzlich können andere Arten des Nachweises verwendet werden, beispielsweise elektrochemische Nachweise. Zu diesem Zweck können beispielsweise entsprechende elektrische und/oder elektronische Messvorrichtungen vorgesehen sein, beispielsweise um amperometrische Messungen an den Testelementen vorzunehmen.

Das Messsystem kann beispielsweise eingerichtet sein, um in mindestens einer Applikationsposition mit dem mindestens einen Testelement, welches sich in der mindestens einen Applikationsposition befindet und/oder mit der jeweiligen Kammer in der Applikationsposition zusammenzuwirken. Diese Applikationsposition, in welcher die Messung erfolgt, kann ganz oder teilweise identisch sein zu der Applikationsposition, in welcher die Probennahmebewegung erfolgen kann. Auch andere Ausgestaltungen sind jedoch grundsätzlich möglich, beispielsweise Probennahmebewegungen und Messungen in unterschiedlichen Applikationspositionen.

In der beschriebenen Ausgestaltung kann das Messsystem insbesondere ausgestaltet sein, um mittels desselben Messsystems die Messungen durchzuführen, auch nachdem ein Wechsel der Orientierung durchgeführt worden

ist. Auf diese Weise lässt sich Bauraum des analytischen Systems einsparen und das analytische System lässt sich stark vereinfachen. Um dies zu ermöglichen, kann das analytische Magazin beispielsweise derart ausgestaltet sein, dass die unterschiedlichen Orientierungen, beispielsweise die analytischen Hilfsmittel in den unterschiedlichen Hilfsmittelebenen, auch nach Wechsel der Orientierung wieder im Wesentlichen gleich zueinander gelagert sind, so dass beispielsweise wieder eine Kammer und/oder ein analytisches Hilfsmittel in der mindestens einen Applikationsposition angeordnet ist.

Oben wurde bereits beschrieben, dass das analytische Magazin vorzugsweise eine Mehrzahl von Eigenschaften hinsichtlich seiner Dimensionierung, insbesondere hinsichtlich seiner Packungsdichte, aufweist. Die oben genannten Dimensionen oder noch kleinere Dimensionen, welche direkt oder indirekt mit der Packungsdichte in Zusammenhang stehen, lassen sich grundsätzlich auch ohne die oben beschriebene Ausgestaltung des analytischen Magazins als Wendemagazin mit mindestens zwei möglichen Orientierungen realisieren. Dementsprechend wird in einem zweiten, nebengeordneten Aspekt allgemein ein analytisches Magazin vorgeschlagen, welches insbesondere, jedoch nicht notwendigerweise, gemäß einer oder mehrerer der oben beschriebenen Ausgestaltungen ausgebildet sein kann. Auch andere Ausgestaltungen sind jedoch möglich, insbesondere Ausgestaltungen, bei welchen das analytische Magazin nicht als Wendemagazin bzw. als Magazin mit zwei möglichen Orientierungen ausgestaltet ist. Insbesondere können die zusätzlichen Merkmale der bevorzugten Ausgestaltungen des oben beschriebenen ersten Aspekts, wie sie insbesondere auch in den Unteransprüchen dargestellt sind, auch unabhängig von dem ersten Aspekt in dem zweiten Aspekt realisiert werden.

Das analytische Magazin gemäß dem zweiten Aspekt weist mindestens zwei Kammern auf, in denen analytische Hilfsmittel aufnehmbar sind. Die analytischen Hilfsmittel sind in mindestens einer der Kammern aufgenommen. Die analytischen Hilfsmittel umfassen jeweils mindestens ein Testelement mit mindestens einer Testchemie zum Nachweis mindestens eines Analyten in einer flüssigen Probe, insbesondere einer Körperflüssigkeit.

Bei herkömmlichen analytischen Magazinen und Testelementen, insbesondere für den Nachweis von Glukose, wird in der Regel eine Testchemie verwendet, welche empfindlich ist gegenüber Luftfeuchtigkeit und welche bei zu langer Exposition an Luft ihre Funktion verschlechtern oder sogar ganz einbüßen kann. Dementsprechend müssen beispielsweise herkömmliche Teststreifen in gegenüber Luftfeuchtigkeit dichten Behältern gelagert werden. Diese Behälter sind üblicherweise mit einem Trockenmittel, also einem Feuchtigkeit absorbierenden Material, beispielsweise Aktivkohle, teilbefüllt. Werden nun bei integrierten Systemen analytische Magazine und/oder analytische Hilfsmittel, beispielsweise Einweg-Hilfsmittel (Disposables), entwickelt, in denen Testelemente einzeln oder in Gruppen verpackt werden, so müssen auch diese Verpackungen feuchtigkeitsdicht ausgestaltet sein. Diese Forderung nach Dichtigkeit gegenüber Feuchtigkeit schränkt jedoch die Auswahl potentieller Materialien extrem ein, insbesondere potentieller Materialien für das Gehäuse. Dies ist insbesondere dadurch bedingt, dass in der Regel zusätzliche Anforderungen existieren, die gleichzeitig erfüllt sein müssen. So müssen die verwendeten Materialien in den meisten Fällen sterilisierbar sein, insbesondere mittels ionisierender Strahlung. Alternativ oder zusätzlich dürfen die verwendeten Materialien in der Regel nicht ausgasen, insbesondere nicht nach oder bei einer Strahlenbelastung durch einen Sterilisationsprozess. Wiederum alternativ oder zusätzlich müssen die verwendeten Materialien für den gewählten Herstellungsprozess geeignet sein, beispielsweise für ein Spritzgießverfahren und/oder einen anderen Formgebungsprozess. Wiederum alternativ oder zusätzlich sollten die verwendeten Materialien vorzugsweise biokompatibel sein und/oder sollten fügbar und/oder versiegelungsfähig sein. Weitere Anforderungen können existieren. Dabei ist insbesondere die Anforderungen einer Dichtigkeit gegenüber Feuchtigkeit eine in der Praxis schwer zu erfüllende Forderung, da die meisten Kunststoffe diffusionsoffen für Feuchtigkeit sind, insbesondere bei geringen Wandstärken, beispielsweise Wandstärken von weniger als einem Millimeter.

Es wird daher in dem zweiten Aspekt, wie auch in dem unten noch näher beschriebenen dritten Aspekt, vorgeschlagen, die Testchemie dabei derart auszugestalten, dass diese zumindest weitgehend stabil gegenüber Umwelteinflüssen, insbesondere gegenüber Feuchtigkeit, ist. Die Testchemie kann insbesondere als Trockenchemie vorliegen, insbesondere auf einem Teststreifen. Im Rahmen der vorliegenden Erfindung wird unter einer im Wesentlichen gegenüber Umwelteinflüssen stabilen Testchemie eine Testchemie verstanden, die gegenüber Luftfeuchtigkeit und vorteilhafterweise ebenfalls gegenüber Sterilisationsverfahren, insbesondere Sterilisationsverfahren unter Verwendung ionisierender Strahlung, stabil ist. Hierbei wird als stabil bezeichnet, wenn bei einer Lagerung bei 32 °C, einer relativen Luftfeuchtigkeit von 85% bei Normaldruck über eine Zeitdauer von drei Wochen die Aktivität, beispielsweise die Enzymaktivität der Testchemie des analytischen Hilfsmittels, um weniger als 50%, vorzugsweise um weniger als 30% und besonders bevorzugt um weniger als 20% abnimmt. Die Aktivität kann dabei grundsätzlich mittels eines beliebigen, aus dem Stand der Technik bekannten Verfahrens bestimmt werden, da im Rahmen der angegebenen Definition lediglich ein Verhältnis der Abnahme der mit diesem Verfahren gemessenen Aktivität zu einer mit diesem Verfahren gemessenen Aktivität vor der Lagerung bzw. unmittelbar nach der Herstellung des analytischen Hilfsmittels von Relevanz ist. Die Aktivität kann sich dabei insbesondere auf eine Enzymaktivität einer Trockenchemie beziehen, insbesondere in einem Teststreifen. Beispielsweise sind Verfahren bekannt, die zur Messung der Enzymaktivität das Enzym aus der Testchemie bzw. dem Teststreifen extrahieren und anschließend beispielsweise mittels einer Ultraviolett-Absorption die Aktivität

bestimmen. Diesbezüglich kann beispielsweise auf H. U. Bergmeyer: Methoden der enzymatischen Analyse, Verlag Chemie, 2. Auflage 1970, S. 417 oder auf Banauch et al.: A glucose dehydrogenase for the determination of glucose concentrations in body fluids, Z. Klin. Chem. Klin. Biochem. 1975 Mar; 13(3):101-7 verwiesen werden. Beispielsweise kann für den Test ein Teststreifen mit der Testchemie hergestellt werden, mit einem üblichen Verfahren die Enzymaktivität eines Enzyms der Testchemie gemessen werden, dann die oben beschriebene Lagerung durchgeführt werden und anschließend erneut dasselbe Verfahren zur Messung der Enzymaktivität durchgeführt werden. Diese Prozedur wird üblicherweise mit einem repräsentativen Kollektiv an Teststreifen bzw. Testchemien durchgeführt. Alternativ oder zusätzlich zu einer Stabilität gegenüber Umwelteinflüssen in Form von Luftfeuchtigkeit kann vorzugsweise auch eine hohe Stabilität der Testchemie gegenüber Umwelteinflüssen in Form von üblicherweise für eine Sterilisierung der analytischen Hilfsmittel und/oder der analytischen Magazine insgesamt verwendeten Strahlung gegeben sein, beispielsweise einer Gammastrahlung und/oder Betastrahlung und/oder einer anderen Art von ionisierender Strahlung.

Als Beispiel einer derartigen, gegenüber Umwelteinflüssen stabilen Testchemie kann auf die oben bereits zitierte WO 2007/012494 A1 verwiesen werden. Die dort dargestellte Testchemie ist auch im Rahmen der vorliegenden Erfindung einsetzbar, allein oder auch in Kombination mit einer oder mehreren anderen Testchemien. Alternativ oder zusätzlich kann die Testchemie auch ausgestaltet sein wie in der nachveröffentlichten europäischen Patentanmeldung mit der Nummer EP 08003054.7 oder in der nachveröffentlichten internationalen Patentanmeldung mit der Nummer PCT/EP2009/001206 aus dem Hause der Anmelderin der vorliegenden Patentanmeldung beschrieben.

So kann die Testchemie beispielsweise ein Enzym und ein stabiles Coenzym enthalten, welche gemeinsamen gelagert sind. Überraschenderweise wurde gefunden, dass mit Hilfe eines stabilen Coenzyms eine Langzeitstabiliserung von mehreren Wochen bzw. Monaten bei hoher relativer Feuchte oder sogar in flüssiger Phase und bei erhöhten Temperaturen möglich ist. Diese Erkenntnis ist überraschend, da bekannt ist, dass Enzyme in Gegenwart von nativem Coenzym zwar eine erhöhte Kurzzeitstabilität für einige Stunden besitzen, aber eine geringere Haltbarkeit über einen längeren Zeitraum aufweisen. Gegenüber diesen Erkenntnissen gegenüber dem Stand der Technik war es überraschend, dass ein Enzym in Gegenwart eines stabilen Coenzyms eine deutlich erhöhte Langzeitstabilität als ein Enzym in Gegenwart eines nativen Coenzyms besitzt, insbesondere da die stabilen Coenzyme eine niedrigere Bindungskonstante mit dem Enzym als das native Coenzym besitzen.

Das durch das Verfahren stabilisierte Enzym kann insbesondere ein Coenzym-abhängiges Enzym sein. Geeignete Enzyme sind z.B. Dehydrogenasen, ausgewählt aus einer Glucose-Dehydrogenase (E.C.1.1.1.47), Lactat-Dehydrogenase (E.C.1.1.1.27, 1.1.1.28), Malat-Dehydrogenase (E.C.1.1.1.37), Glycerin-Dehydrogenase (E.C.1.1.1.6), Alkohol-Dehydrogenase (E.C.1.1.1.1), alpha-Hydroxybutyrat-Dehydrogenase, Sorbitol-Dehydrogenase oder Aminosäure-Dehydrogenase, z. B. L-Aminosäure-Dehydrogenase (E.C.1.4.1.5). Weitere geeignete Enzyme sind Oxidasen, wie etwa Glucoseoxidase (E.C.1.1.3.4) oder Cholesterinoxidase (E.C.1.1.3.6) bzw. Aminotransferasen, wie z.B. Aspartat oder Alanin Aminotransferase, 5'-Nukleotidase oder Creatin-Kinase. Vorzugsweise ist das Enzym Glucose-Dehydrogenase.

Als besonders bevorzugt hat sich der Einsatz einer mutierten Glucose-Dehydrogenase erwiesen. Der Begriff "Mutante", wie er im Rahmen der vorliegenden Anmeldung verwendet wird, bezeichnet eine genetisch veränderte Variante eines nativen Enzyms, welche bei gleicher Anzahl an Aminosäuren eine gegenüber dem Wildtyp-Enzym veränderte Aminosäuresequenz besitzt, d.h. sich in mindestens einer Aminosäure vom Wildtyp-Enzym unterscheidet. Die Einführung der Mutation(en) kann ortsspezifisch oder nichtortsspezifisch, bevorzugt ortsspezifisch unter Verwendung von im Fachbereich bekannten rekombinanten Methoden, erfolgen, wobei entsprechend den jeweiligen Anforderungen und Bedingungen mindestens ein Aminosäureaustausch innerhalb der Aminosäuresequenz des nativen Enzyms resultiert. Besonders bevorzugt weist die Mutante eine gegenüber dem Wildtyp-Enzym erhöhte thermische oder hydrolytische Stabilität auf.

Die mutierte Glucose-Dehydrogenase kann die gegenüber der korrespondierenden Wildtyp-Glucose-Dehydrogenase veränderte(n) Aminosäure(n) grundsätzlich an einer beliebigen Position ihrer Aminosäuresequenz enthalten. Vorzugsweise umfasst die mutierte Glucose-Dehydrogenase eine Mutation an mindestens einer der Positionen 96, 170 und 252 der Aminosäuresequenz der Wildtyp-Glucose-Dehydrogenase, wobei Mutanten mit Mutationen an Position 96 und Position 170 bzw. Mutationen an Position 170 und Position 252 besonders bevorzugt sind. Als vorteilhaft hat es sich erwiesen, wenn die mutierte Glucose-Dehydrogenase neben diesen Mutationen keine weiteren Mutationen enthält.

Die Mutation an den Positionen 96, 170 und 252 kann grundsätzlich einen beliebigen Aminosäureaustausch umfassen, welcher zu einer Stabilisierung, z.B. einer Erhöhung der thermischen oder hydrolytischen Stabilität, des Wildtyp-Enzyms führt. Vorzugsweise umfasst die Mutation an Position 96 einen Aminosäureaustausch von Glutaminsäure gegen Glycin, während in Bezug auf Position 170 ein Aminosäureaustausch von Glutaminsäure gegen Arginin oder Lysin, insbesondere ein Aminosäureaustausch von Glutaminsäure gegen Lysin, bevorzugt ist. Was die Mutation an Position 252 anbetrifft, so umfasst diese bevorzugt einen Aminosäureaustausch von Lysin gegen Leucin.

Die mutierte Glucose-Dehydrogenase kann durch Mutation einer aus einer beliebigen biologischen Quelle stammenden Wildtyp-Glucose-Dehydrogenase erhalten werden, wobei der Begriff "biologische Quelle" sowohl Prokaryoten, wie beispielsweise Bakterien, als auch Eukaryoten, wie beispielsweise Säuger und andere Tiere, umfasst. Vorzugsweise entstammt die Wildtyp-Glucose-Dehydrogenase einem Bakterium, wobei es sich besonders bevorzugt um eine Glucose-Dehydrogenase aus Bacillus megaterium, Bacillus subtilis oder Bacillus thuringiensis, insbesondere aus Bacillus subtilis, handelt.

In einer besonders bevorzugten Ausführungsform der vorliegenden Erfindung handelt es sich bei der mutierten Glucose-Dehydrogenase um eine durch Mutation von Wildtyp-Glucose-Dehydrogenase aus Bacillus subtilis erhaltene Glucose-Dehydrogenase, welche die in SEQ ID NO: 1 (GlucDH_E96G_E170K) oder die in SEQ ID NO:2 (GlucDH_E170K_K252L) dargestellte Aminosäuresequenz besitzt.

Das stabile Coenzym ist vorzugsweise ein gegenüber dem nativen Coenzym chemisch verändertes Coenzym, welches eine im Vergleich zum nativen Coenyzm höhere Stabilität, (z.B. hydrolytische Stabilität) aufweist. Vorzugsweise ist das stabile Coenyzm unter Testbedingungen gegenüber Hydrolyse stabil. Im Vergleich zum nativen Coenzym kann das stabile Coenzym eine verringerte Bindungskonstante für das Enzym aufweisen, beispielsweise eine um den Faktor von 2 oder mehr verringerte Bindungskonstante.

Bevorzugte Beispiele für stabile Coenzyme sind stabile Derivate von Nicotinamid-Adenin-Dinukleotid (NAD/NADH) oder Nicotinamid-Adenin-Dinukleotidphosphat (NADP/NADPH), oder verkürzte NAD-Derivate, z.B. ohne AMP-Teil bzw. mit nicht nukleosidischen Resten, z.B. hydrophoben Resten. Ebenfalls bevorzugt ist als stabiles Coenzym im Sinne der vorliegenden Erfindung ist die Verbindung der Formel (I).

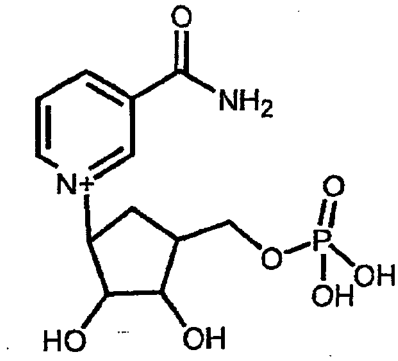

(I).

Bevorzugte stabile Derivate von NAD/NADH bzw. NADP/NADPH sind in den zuvor genannten Referenzen beschrieben, auf deren Offenbarung hiermit ausdrücklich Bezug genommen wird. Besonders bevorzugte stabilisierte Coenzyme sind in WO 2007/012494 bzw. US 11/460,366 beschrieben, auf deren Offenbarung hiermit ausdrücklich Bezug genommen wird. Das stabile Coenzym wird besonders bevorzugt aus Verbindungen mit der allgemeinen Formel (II) ausgewählt:

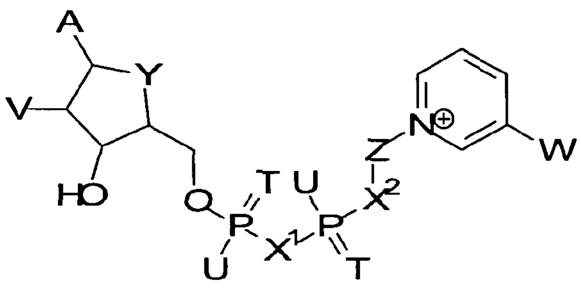

(II)

mit

A = Adenin oder ein Analog davon,
T = jeweils unabhängig O, S,

U = jeweils unabhängig OH, SH, $BH_3^-$, $BCNH_2^-$,
V = jeweils unabhängig OH oder eine Phosphatgruppe, oder zwei Gruppen, die eine cyclische Phosphatgruppe bilden;
W = COOR, $CON(R)_2$, COR, $CSN(R)_2$ mit R = jeweils unabhängig H oder $C_1$-$C_2$-Alkyl,
$X^1$, $X^2$ = jeweils unabhängig O, $CH_2$, $CHCH_3$, $C(CH_3)_2$, NH, $NCH_3$,
Y = NH, S, O, $CH_2$,
Z = ein linearer oder cyclischer organischer Rest ist,
mit der Maßgabe, dass Z und der Pyridin-Rest nicht durch eine glycosidische Verbindung verknüpft sind, oder ein Salz oder gegebenenfalls eine reduzierte Form davon.

In den Verbindungen der Formel (II) ist Z vorzugsweise ein linearer Rest mit 4-6 C-Atomen, vorzugsweise 4 C-Atomen, worin 1 oder 2 C-Atome gegebenenfalls durch ein oder mehrere Heteroatome ausgewählt aus O, S und N ersetzt sind, oder ein Rest umfassend eine cyclische Gruppe mit 5 oder 6 C-Atomen, die gegebenenfalls ein Heteroatom ausgewählt aus O, S und N sowie gegebenenfalls einen oder mehrere Substituenten enthält, und einen Rest $CR^4_2$, wobei $CR^4_2$ an die cyclische Gruppe und an $X^2$ gebunden ist, mit $R^4$ = jeweils unabhängig H, F, Cl, $CH_3$.
Besonders bevorzugt ist Z ein gesättiger oder ungesättigter carbocyclischer oder heterocyclischer Fünfring, insbesondere eine Verbindung der allgemeinen Formel (III)

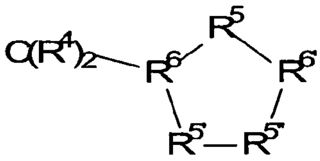

(III)

wobei zwischen $R^{5'}$ und $R^{5''}$ eine Einfach- oder Doppelbindung vorliegen kann, mit
$R^4$ = jeweils unabhängig H, F, Cl, $CH_3$,
$R^5$ =$CR^4_2$,
wobei $R^{5'}$ = O, S, NH, $NC_1$-$C_2$-Alkyl, $CR^4_2$, CHOH, $CHOCH_3$, und
$R^{5''}$ = $CR^4_2$, CHOH, $CHOCH_3$, wenn zwischen $R^{5'}$ und $R^{5''}$ eine Einfachbindung vorliegt, und
wobei $R^{5'}R^{5''}$=$CR^4$, wenn zwischen $R^{5'}$ und $R^{5''}$ eine Doppelbindung vorliegt, und
$R^6$, $R^{6'}$ = jeweils unabhängig CH oder $CCH_3$.

In einer bevorzugten Ausführungsform enthalten die Verbindungen Adenin oder Adenin-Analoga, wie z.B. $C_8$- und $N_6$-substituiertes Adenin, Deaza-Varianten wie 7-Deaza, Azavarianten wie 8-Aza oder Kombinationen wie 7-Deaza oder 8-Aza oder carbocyclische Analoga, wie Formycin, wobei die 7-Deazavarianten in der 7-Position mit Halogen, $C_1$-$C_6$-Alkinyl, -Alkenyl oder -Alkyl substituiert sein können.
In einer weiteren bevorzugten Ausführungsform enthalten die Verbindungen Adenosin-Analoga, welche statt Ribose z.B. 2-Methoxy-deoxyribose, 2'-Fluoro-deoxyribose, Hexitol, Altritol bzw. polycyclische Analoga, wie Bicyclo-, LNA- und Tricyclo-Zucker enthalten.
Insbesondere können in den Verbindungen der Formel (II) auch (Di-)-Phosphatsauerstoffe isotronisch ersetzt sein, wie z.B. $O^-$ durch $S^-$ bzw. $BH_3^-$, O durch NH, $NCH_3$ bzw. $CH_2$ und =O durch =S.
In den Verbindungen der Formel (II) ist W vorzugsweise $CONH_2$ oder $COCH_3$.
In den Gruppen der Formel (III) ist $R^5$ vorzugsweise $CH_2$. Weiterhin ist bevorzugt, dass $R^{5'}$ ausgewählt ist aus $CH_2$, CHOH und NH. In einer besonders bevorzugten Ausführungsform sind $R^{5'}$ und $R^{5''}$ jeweils CHOH. In noch einer weiteren bevorzugten Ausführungsform ist $R^{5'}$ NH und $R^{5''}$ $CH_2$.
In der am stärksten bevorzugten Ausführungsform handelt es sich bei dem stabilen Coenzym um carbaNAD.
Die bevorzugte Testchemie ist insbesondere derart ausgestaltet, dass in dieser enthaltene Enzyme Langzeit-stabilisiert sind. Dies bedeutet, dass man das mit einem stabilen Coenzym stabilisierte Enzym, z.B. als Trockensubstanz, beispielsweise über eine Dauer von mindestens 2 Wochen, vorzugsweise von mindestens 4 Wochen und besonders bevorzugt von mindestens 8 Wochen lagert und wobei die Enzymaktivität vorzugsweise um weniger als 50 %, besonders bevorzugt weniger als 30 % und am meisten bevorzugt um weniger als 20 % bezüglich des Ausgangswerts der Enzymaktivität abnimmt.
Weiterhin umfasst kann die Testchemie derart ausgestaltet werden, dass das mit einem stabilen Coenzym stabilisierte Enzym bei erhöhten Temperaturen, beispielsweise bei einer Temperatur von mindestens 20 °C, vorzugsweise von mindestens 25 °C und besonders bevorzugt von mindestens 30 °C gelagert wird. Die Enzymaktivität nimmt

dabei vorzugsweise um weniger als 50 %, besonders bevorzugt weniger als 30 % und am meisten bevorzugt weniger als 20 % bezüglich ihres Ausgangswerts ab.

Durch die Stabilisierung ist es möglich, das mit einem stabilen Coenzym stabilisierte Enzym auch ohne Trocknungsreagenz für eine lange Zeit, wie oben angegeben, und/oder bei hohen Temperaturen, wie oben angegeben, zu lagern. Weiterhin kann das stabilisierte Enzym auch bei einer hohen relativen Luftfeuchtigkeit, z.B. einer relativen Luftfeuchtigkeit von mindestens 50 % gelagert werden, wobei die Enzymaktivität vorzugsweise um weniger als 50 %, besonders bevorzugt weniger als 30 % und am meisten bevorzugt weniger als 20 % bezüglich des Ausgangswerts abnimmt.

Die Lagerung des mit einem stabilen Coenzym stabilisierten Enzyms kann einerseits als Trockensubstanz und andererseits in Flüssigphase erfolgen. Bevorzugt erfolgt die Lagerung des stabilisierten Enzyms auf oder in einem Testelement, das zur Bestimmung eines Analyten geeignet ist. Das mit einem stabilen Coenzym stabilisierte Enzym ist dabei Bestandteil der bevorzugten Testchemie, welche gegebenenfalls noch weitere Bestandteile wie etwa Salze, Puffer, etc. enthalten kann. Vorzugsweise ist die Testchemie dabei frei von einem Mediator.

Das mit einem stabilen Coenzym stabilisierte Enzym kann allgemein zum Nachweis von Analyten, beispielsweise Parametern in Körperflüssigkeiten wie etwa Blut, Serum, Plasma oder Urin bzw. in Abwasserproben oder Lebensmitteln eingesetzt werden.

Als Analyten können beliebige biologische oder chemische Substanzen bestimmt werden, die durch eine Redoxreaktion nachgewiesen werden können, z.B. Substanzen, bei denen es sich um Substrate eines Coenzym-abhängigen Enzyms handelt oder Coenzym-abhängige Enzyme selbst. Bevorzugte Beispiele für Analyten sind Glucose, Milchsäure, Äpfelsäure, Glycerin, Alkohol, Cholesterin, Triglyceride, Ascorbinsäure, Cystein, Glutathion, Peptide, Harnstoff, Ammonium, Salicylat, Pyruvat, 5'-Nukleotidase, Creatinkinase (CK), Lactat-Dehydrogenase (LDH), Kohlendioxid etc. Bevorzugt ist der Analyt Glucose. Besonders bevorzugt ist hierbei der Nachweis von Glucose mit Hilfe von Glucose-Dehydrogenase (GlucDH).

Die Veränderung des stabilen Coenzyms durch Reaktion mit dem Analyten kann grundsätzlich auf beliebige Art und Weise nachgewiesen werden. Hier können grundsätzlich alle aus dem Stand der Technik bekannten Methoden zum Nachweis enzymatischer Reaktionen eingesetzt werden. Vorzugsweise wird jedoch die Veränderung des Coenzyms durch optische Methoden nachgewiesen. Optische Nachweismethoden umfassen beispielsweise die Messung von Absorption, Fluoreszenz, Circulardichroismus (CD), optische Rotationsdispersion (ORD), Refraktometrie etc.

Ein optisches Nachweisverfahren, welches im Rahmen der vorliegenden Anmeldung bevorzugt Anwendung findet, ist die Photometrie. Zur photometrischen Messung einer Veränderung des Coenzyms infolge Umsetzung mit dem Analyten bedarf es indessen der zusätzlichen Anwesenheit mindestens eines Mediators, welcher die Reaktivität des reduzierten Coenzyms erhöht und eine Übertragung von Elektronen auf einen geeigneten optischen Indikator oder ein optisches Indikatorsystem ermöglicht.

Als Mediatoren, welche für die Zwecke der vorliegenden Erfindung geeignet sind, kommen u.a. Nitrosoaniline, wie beispielsweise [(4-Nitrosophenyl)imino]dimethanol-Hydrochlorid, Chinone, wie beispielsweise Phenanthrenchinone, Phenanthrolinchinone oder Benzo[h]-chinolinchinone, Phenazine, wie beispielsweise 1-(3-Carboxypropoxy)-5-ethylphenazinium-trifluormethansulfonat, oder/und Diaphorase (EC 1.6.99.2) in Frage. Bevorzugte Beispiele für Phenanthrolinchinone umfassen 1,10-Phenanthrolin-5,6-chinone, 1,7-Phenanthrolin-5,6-chinone, 4,7-Phenanthrolin-5,6-chinone sowie deren N-alkylierte oder N,N'-dialkylierte Salze, wobei im Falle N-alkylierter bzw. N,N'-dialkylierter Salze Halogenide, Trifluormethansulfonat oder andere die Löslichkeit erhöhende Anionen als Gegenion bevorzugt sind.

Als optischer Indikator oder als optisches Indikatorsystem kann eine beliebige Substanz verwendet werden, welche reduzierbar ist und bei Reduktion eine detektierbare Änderung ihrer optischen Eigenschaften, wie beispielsweise Farbe, Fluoreszenz, Remission, Transmission, Polarisation oder/und Brechungsindex, erfährt. Die Bestimmung des Vorhandenseins oder/und der Menge des Analyten in der Probe kann mit dem bloßen Auge oder/und mittels einer Detektionsvorrichtung unter Verwendung eines dem Fachmann geeignet erscheinenden photometrischen Verfahrens erfolgen. Vorzugsweise werden Heteropolysäuren, und insbesondere 2,18-Phosphormolybdänsäure, als optische Indikatoren verwendet, die zum entsprechenden Heteropolyblau reduziert werden.

Besonders bevorzugt wird die Veränderung des Coenzyms durch Messung der Fluoreszenz nachgewiesen. Die Fluoreszenzmessung ist hochsensitiv und ermöglicht den Nachweis selbst geringer Konzentrationen des Analyten in miniaturisierten Systemen.

Alternativ kann die Veränderung des Coenzyms auch elektrochemisch unter Verwendung eines geeigneten Testelements, wie beispielsweise eines elektrochemischen Teststreifens, nachgewiesen werden. Voraussetzung hierfür ist wiederum die Verwendung geeigneter Mediatoren, welche vom reduzierten Coenzym durch Übertragung von Elektronen in eine reduzierte Form überführt werden können. Die Bestimmung des Analyten erfolgt über eine Messung des zur Reoxidation des reduzierten Mediators benötigten Stromes, welcher mit der Konzentration des Analyten in der Probe korreliert. Beispiele für Mediatoren, welche für elektrochemische Messungen verwendet werden können, umfassen insbesondere die vorstehend erwähnten, für photometrische Messungen eingesetzten Mediatoren.

Zum Nachweis eines Analyten kann ein Flüssigtest verwendet werden, wobei das Reagenz z.B. in Form einer

Lösung oder Suspension in einer wässrigen oder nichtwässrigen Flüssigkeit oder als Pulver oder Lyophilisat vorliegt. Es kann jedoch auch ein Trockentest verwendet werden, wobei das Reagenz auf einem Träger, einem Teststreifen, aufgebracht ist. Der Träger kann beispielsweise einen Teststreifen, umfassend ein saugfähiges oder/und quellbares Material, umfassen, das von der zu untersuchenden Probeflüssigkeit benetzt wird.

Ein besonders bevorzugtes Testformat, umfasst die Verwendung des Enzyms Glucose-Dehydrogenase mit einem stabilen NAD-Derivat zum Nachweis von Glucose, wobei ein Derivat des reduzierten Coenzyms NADH gebildet wird. Der Nachweis von NADH erfolgt durch optische Methoden, z.B. durch photometrische oder fluorometrische Bestimmung nach UV-Anregung. Ein besonders bevorzugtes Testsystem ist in US 2005/0214891 beschrieben, auf das hier ausdrücklich Bezug genommen wird.

Die Testchemie kann insbesondere ein mit einem stabilen Coenzym stabilisiertes Enzym enthalten, wobei das stabilisierte Enzym bei einer Lagerung von vorzugsweise mindestens 2 Wochen, besonders bevorzugt mindestens 4 Wochen und am meisten bevorzugt mindestens 8 Wochen bei einer Temperatur von vorzugsweise mindestens 20 °C, besonders bevorzugt mindestens 25 °C und am meisten bevorzugt mindestens 30 °C, gegebenenfalls bei hoher Luftfeuchtigkeit und ohne Trockenreagenz eine Abnahme der enzymatischen Aktivität von weniger als 50 %, vorzugsweise weniger als 30 % und am meisten bevorzugt weniger als 20 % gegenüber dem Ausgangswert aufweist.

Auch andere Arten stabiler Testchemien können alternativ oder zusätzlich verwendet werden, beispielsweise die in WO 2007/012494 A1 beschriebene Testchemie. Die Testchemie kann grundsätzlich in beliebiger Weise in einem Testelement enthalten sein. Die Testchemie bzw. das Testelement können zur Durchführung von Trocken- oder Flüssigtests geeignet sein. Beispielsweise kann die Testchemie zu diesem Zweck auf einem geeigneten Trägermaterial aufgebracht sein, beispielsweise auf einem Kunststoff und/oder einem keramischen Material und/oder einem Papiermaterial.

Das analytische Magazin weist in dem zweiten Aspekt eine Packungsdichte der analytischen Hilfsmittel von mehr als 5 analytischen Hilfsmitteln pro $cm^3$ auf, vorzugsweise mindestens $10/cm^3$, insbesondere mindestens $16/cm^3$, vorzugsweise mindestens $25/cm^3$ und beispielsweise $32{,}3/cm^3$. Auch in den anderen Aspekten ist diese Packungsdichte bevorzugt. Weiterhin kann das analytische Magazin in diesem zweiten Aspekt, wie auch in anderen Aspekten, vorzugsweise eine oder mehrere der folgenden Eigenschaften aufweisen: ein Gesamtvolumen von nicht mehr als 10 $cm^3$; einen Außenradius von nicht mehr als 5 cm; einen Innenradius zwischen 0,5 cm und 2 cm; eine Dicke von nicht mehr als 1 cm; eine Anzahl an analytischen Hilfsmitteln von 10 bis 100; ein Volumen zwischen 3 $cm^3$ und 30 $cm^3$.

Besonders bevorzugt ist es, wenn ein Außenvolumen des analytischen Magazins, also ein Volumen ohne Berücksichtigung von optionalen Löchern oder anderen optionalen Öffnungen in dem analytischen Magazin, 5 $cm^3$ nicht überschreitet, vorzugsweise 3 $cm^3$ und besonders bevorzugt 2 $cm^3$. Beispielsweise kann das Außenvolumen 1,94 $cm^3$ betragen. Besonders bevorzugt ist es, wenn ein Leervolumen des analytischen Magazins, also ein gesamtes Volumen von optional vorhandenen Öffnungen in dem analytischen Magazin, 0,8 $cm^3$ nicht überschreitet, vorzugsweise 0,5 $cm^3$ und besonders bevorzugt 0,4 $cm^3$. Beispielsweise kann das Leervolumen 0,39 $cm^3$ betragen. Bei den Öffnungen kann es sich beispielsweise um innere Öffnungen eines scheibenförmigen analytischen Magazins handeln, in welche beispielsweise ein Antrieb eingreifen kann. Nicht umfasst von diesem Leervolumen soll der Innenraum im Inneren des Gehäuses sein, beispielsweise die Kammern. Unter einem Nettovolumen wird dabei im Rahmen der vorliegenden Erfindung allgemein das Außenvolumen abzüglich des Leervolumens verstanden. Bei einem kreisscheibenförmigen Magazin ergibt sich somit ein Volumen, das sich im Wesentlichen aus dem Durchmesser und der Höhe der Kreisscheibe ergibt, und bei einem ringscheibenförmigen Magazin ein Nettovolumen, das sich gegenüber dem Nettovolumen einer entsprechenden Kreisscheibe durch Abzug des Volumens einer zentralen Ausnehmung ergibt. Dementsprechend ist es bevorzugt, wenn das Nettovolumen des analytischen Magazins, also das Außenvolumen abzüglich des Leervolumens, 5 $cm^3$ nicht überschreitet, besonders bevorzugt 3 $cm^3$ und insbesondere 2 $cm^3$ nicht überschreitet. Beispielsweise kann das Nettovolumen 1,55 $cm^3$ betragen.

Unter einer Packungsdichte wird allgemein im Rahmen der vorliegenden Erfindung eine Anzahl analytische Hilfsmittel pro Nettovolumen des analytischen Magazins verstanden.

Wie oben dargelegt, kann jedes analytische Hilfsmittel jedoch mehrere Teil-Hilfsmittel umfassen, welche miteinander zusammenwirken können, beispielsweise jeweils als ein Test. Vorzugsweise umfasst jedes analytische Hilfsmittel als Teil-Hilfsmittel ein Testelement mit mindestens einer Testchemie. Zusätzlich kann dann jedes analytische Hilfsmittel als weiteres Teil-Hilfsmittel mindestens eine Lanzette umfassen, welche zur Probengenerierung einer Probe an Körperflüssigkeit dient, die dann auf das zugehörige Testelement aufgebracht wird. Sind in einem analytischen Hilfsmittel mehrere analytische Teil-Hilfsmittel enthalten, so zählen zusammengehörige analytische Teil-Hilfsmittel für die Berechnung der Packungsdichte jedoch nach wie vor als ein analytisches analytisches Hilfsmittel, beispielsweise indem ein Testelement und eine zugehörige Lanzette als ein gemeinsames analytisches Hilfsmittel gezählt werden. Beispielsweise kann jeweils genau ein analytisches Hilfsmittel mit mindestens einem Testelement und optional mindestens einer Lanzette in jeweils einer Kammer aufgenommen sein. Wie oben dargelegt, sind jedoch auch andere Ausgestaltungen möglich.

So kann das analytische Magazin beispielsweise als Kreisringscheibe ausgestaltet sein, mit einem Außendurchmesser von weniger als 100 mm, beispielsweise 50 mm, und einem Innendurchmesser einer Ausnehmung der Kreisringscheibe von weniger als 50 mm, beispielsweise 22,5 mm. Das analytische Magazin kann beispielsweise eine Dicke von weniger als 5,0 mm aufweisen, beispielsweise eine Dicke von 3,1 mm. Das analytische Magazin kann vorzugsweise mehr als 20 analytische Hilfsmittel umfassen, beispielsweise mindestens 50 analytische Hilfsmittel und sogar 100 analytische Hilfsmittel oder mehr. Beispielsweise können 50 analytische Hilfsmittel mit jeweils einem Testelement und optional jeweils zusätzlich einer Lanzette vorgesehen sein, wobei jeweils ein Testelement und eine zugehörige Lanzette als ein analytisches Hilfsmittel, auch "Test" genannt, zählen. Beispielsweise kann jeweils ein derartiges analytisches Hilfsmittel in jeweils einer Kammer aufgenommen sein. Dementsprechend kann die Packungsdichte beispielsweise mindestens 50 analytische Hilfsmittel / 5 $cm^3$ = 1 analytisches Hilfsmittel / 0,1 $cm^3$ betragen, vorzugsweise mindestens 50 analytische Hilfsmittel / 3 $cm^3$ = 1 analytisches Hilfsmittel / 0,06 $cm^3$ und besonders bevorzugt mindestens 50 analytische Hilfsmittel / 2 $cm^3$ = 1 analytisches Hilfsmittel / 0,04 $cm^3$. Beispielsweise kann die Packungsdichte bei 50 analytische Hilfsmittel / 1,55 $cm^3$ = 1 analytisches Hilfsmittel / 0,031 $cm^3$ liegen. Auch eine andere Ausgestaltung des analytischen Magazins als eine Ausgestaltung als Kreisscheibe ist jedoch möglich, beispielsweise in einer oder mehreren der oben beschriebenen Geometrien. Auch eine Ausgestaltung als Bandmagazin ist beispielsweise möglich, wobei beispielsweise eine Kammer des Bandmagazins einen Gutwickel mit noch nicht benutzten analytischen Hilfsmitteln umfasst und eine andere Kammer des Bandmagazins einen Schlechtwickel, auf welchem bereits benutzte analytische Hilfsmittel aufnehmbar sind.

Wenn eine stabile Testchemie verwendet wird, so kann grundsätzlich auf eine feuchtigkeitsundurchlässige Ausgestaltung der Trennung der einzelnen Kammern auch vollständig verzichtet werden. In einem dritten, ebenfalls nebengeordneten Aspekt, welcher einzeln oder auch in Kombination mit dem oben genannten ersten Aspekt und/oder dem oben genannten zweiten Aspekt in einer oder mehreren der beschriebenen Ausgestaltungen realisiert werden kann, welcher jedoch auch unabhängig realisierbar ist, wird daher wiederum ein analytisches Magazin vorgeschlagen, welches insbesondere, jedoch nicht notwendigerweise, gemäß einer oder mehrerer der oben beschriebenen Ausgestaltungen ausgebildet sein kann. Auch andere Ausgestaltungen sind jedoch möglich, insbesondere Ausgestaltungen, bei welchen das analytische Magazin nicht gemäß dem ersten Aspekt als Wendemagazin bzw. als Magazin mit zwei möglichen Orientierungen ausgestaltet ist und/oder Ausgestaltungen, bei welchen nicht die im zweiten Aspekt genannten Dimensionierungen vorliegen. Für bevorzugte Ausgestaltungen des analytischen Magazins gemäß dem dritten Aspekt kann dabei wiederum auf die obige Beschreibung der bevorzugten zusätzlichen Merkmale des ersten Aspekts und des zweiten Aspekts verwiesen werden, wie sie insbesondere auch in den Unteransprüchen aufgeführt sind. Diese zusätzlichen Merkmale können auch unabhängig von den übrigen Merkmalen des ersten Aspekts und/oder des zweiten Aspekts im Rahmen des dritten Aspekts realisiert werden.

Das analytische Magazin weist in dem dritten Aspekt wiederum mindestens zwei Kammern auf, in denen analytische Hilfsmittel aufnehmbar sind. Die analytischen Hilfsmittel sind in mindestens einer der Kammern aufgenommen. Die analytischen Hilfsmittel umfassen jeweils mindestens ein Testelement mit mindestens einer Testchemie zum Nachweis mindestens eines Analyten in einer flüssigen Probe, insbesondere einer Körperflüssigkeit. Die Testchemie ist dabei wiederum derart ausgestaltet, dass diese zumindest weitgehend stabil gegenüber Umwelteinflüssen, insbesondere gegenüber Feuchtigkeit, ist. In diesem dritten Aspekt wird vorgeschlagen, die Kammern derart voneinander getrennt auszugestalten, dass ein Austausch von Feuchtigkeit zwischen den Kammern, beispielsweise zwischen benachbarten Kammern, möglich ist. Beispielsweise können die Kammern Kammerwände aufweisen, wobei in oder neben den Kammerwänden Spalte oder andere Öffnungen vorgesehen sind, vorzugsweise mit einer Öffnungsweite von nicht mehr als 20 Mikrometern, insbesondere von nicht mehr als 10 Mikrometern. Diese Öffnungsweiten ermöglichen einerseits einen Austausch von Luftfeuchtigkeit zwischen den Kammern, halten jedoch in der Regel gröbere Verunreinigungen oder Keime zurück. Alternativ oder zusätzlich können für die Wände der Kammern und/oder das Gehäuse Materialien eingesetzt werden, welche eine Permeabilität für Wasserdampf aufweisen. Dabei ist in diesem dritten Aspekt der vorliegenden Erfindung das analytische Magazin, insbesondere mindestens einer der Kammern des analytischen Magazins, eingerichtet, um die analytischen Hilfsmittel oder Teil-Hilfsmittel dieser analytischen Hilfsmittel nach einer Verwendung wieder in mindestens einer der Kammern zu remagazinieren. Bezüglich des Begriffs der Remagazinierung und der möglichen Ausgestaltungen des analytischen Magazins zum Zweck der Remagazinierung kann auf die obige Beschreibung des ersten Aspekts der Erfindung verwiesen werden. Insbesondere können wiederum Halteelemente vorgesehen sein, um ein analytisches Hilfsmittel oder, was im Rahmen dieses Aspekts gleichbedeutend sein soll, einen Teil-Hilfsmittel wie beispielsweise eine Lanzette oder einen Microsampler, nach der Remagazinierung in einer geöffneten Kammer zu halten, beispielsweise formschlüssig und/oder kraftschlüssig. Bei einer Remagazinierung macht sich die gegenüber Umwelteinflüssen zumindest weit gehend stabile Testchemie besonders vorteilhaft bemerkbar, da in vielen Fällen bei einer Remagazinierung aktiv Feuchtigkeit, beispielsweise in Form einer flüssigen Probe, in die Kammer eingebracht wird. Durch die Verwendung der gegenüber Umwelteinflüssen stabilen Testchemie lässt sich dieser Problematik im Vergleich zu herkömmlichen Testchemien vermeiden.

Der oben beschriebene zweite Aspekt und/oder der oben beschriebene dritte Aspekt, wie auch der beschriebene erste Aspekt, lassen sich auf verschiedene Weisen vorteilhaft weiter ausgestalten.

Insbesondere kann, wie oben dargestellt, ein analytisches Hilfsmittel jeweils ein Testelement mit einer Testchemie, insbesondere der gegenüber Umwelteinflüssen stabilen Testchemie, und eine Lanzette umfassen. Diese Teil-Hilfsmittel können gemeinsam in ein und derselben Kammer gelagert sein, beispielsweise jeweils ein Paar aus einer Lanzette und einem Testelement in einer Kammer, oder mehrere derartiger Paare in einer gemeinsamen Kammer. Durch Verwendung der gegenüber Umwelteinflüssen, insbesondere Luftfeuchtigkeit und/oder Betastrahlung stabilen Testchemie, kann eine primäre Forderung nach einer separaten Verpackung von Lanzetten und Testelementen entfallen. Darüber hinaus kann auch die Notwendigkeit, die Testchemie wasserdampfdicht zu verpacken und/oder ein Trockenmittel in der die Kammern und/oder das analytische Magazin einzufügen, entfallen. Dadurch werden neue Konzepte insbesondere für ein kombiniertes analytisches Magazin mit Lanzetten und Testelementen möglich. Eine voneinander getrennte Aufbewahrung der Lanzetten und der Testelemente ist nicht erforderlich. Zudem kommen, wie unten noch näher ausgeführt wird, andere Materialien für die Magazinteile infrage, da nicht mehr gleichzeitig die Forderungen nach Strahlenstabilität und Dampfdichtigkeit zusammen erfüllt werden müssen.

Da auf eine Barriere zwischen den analytischen Hilfsmitteln, insbesondere zwischen Lanzetten und Testelementen, verzichtet werden kann, kann eine erhebliche kompaktere Anordnung von Lanzettenelementen und Testelementen erfolgen, beispielsweise in derselben Kammer des analytischen Magazins. Die Lanzettenspitze kann beispielsweise bei der Lagerung in der Kammer dicht neben der Testchemie liegen, die sich nach einer Probengewinnung auch als Position für einen Übertrag der Probe auf die Testchemie eignet. Dadurch kann ein Mechanismus zur Betätigung des analytischen Hilfsmittels erheblich einfacher gestaltet werden als bei üblichen analytischen Magazinen, da beispielsweise auf eine zusätzliche Bewegung zum Zweck einer Beseitigung einer zweiten Barriere, als einer Barriere zwischen Lanzette und Testelement, verzichtet werden kann und da keine zusätzliche Position mit der Lanzette angefahren werden muss. Im Unterschied zu bisherigen Konzepten mit getrennter Verpackung von Lanzette und Testchemie muss die Testchemie auch nicht notwendigerweise bewegt werden, um diese aus ihrer Verpackung zu entfernen. In diesem Fall muss in der Regel lediglich noch die Lanzette an einen entsprechenden Aktor angedockt und bewegt werden.

Hohe Packungsdichten lassen sich insbesondere dadurch erreichen, dass die analytischen Hilfsmittel sehr dicht beieinander gelagert werden, ohne dass eine hermetische Trennung zwischen den analytischen Hilfsmitteln erfolgen muss. Diesbezüglich macht sich die Verwendung der stabilen Testchemie besonders vorteilhaft bemerkbar. So können beispielsweise analytische Magazine verwendet werden, welche ein Gehäuse aufweisen, welches eine Wandstärke von maximal 1,2 mm aufweist. Unter einer Wandstärke ist dabei eine Dicke des Gehäuses zwischen einer ein analytisches Hilfsmittel umfassenden Kammer und der Umgebung oder einer benachbarten Kammer zu verstehen, insbesondere an einer dünnsten Stelle des Gehäuses. Besonders bevorzugt ist es, wenn die Wandstärke nicht mehr als 1,0 mm und insbesondere nicht mehr als 0,8 mm beträgt. Beispielsweise kann die Wandstärke von 0,3 mm bis 0,8 mm gewählt werden.

Wiederum alternativ oder zusätzlich kann in dem analytischen Magazin auf die Verwendung eines Trockenmittels wie beispielsweise Aktivkohle verzichtet werden. Dementsprechend ist es besonders bevorzugt, wendet das analytische Magazin Trockenmittel-frei ausgestaltet ist. Auch auf diese Weise lässt sich Bauraum einsparen. Unter einem Trockenmittel wird dabei allgemein eine für eine größere Menge an Wasser aufnahmefähige Substanz, beispielsweise eine im Wesentlichen bevorzugt Wasser absorbierende Substanz, verstanden. In üblichen analytischen Magazinen ist mindestens eine Kammer mit einem Trockenmittel vorgesehen. Es kann jedoch ein analytisches Magazin bereitgestellt werden, bei welchem in ungebrauchtem Zustand ausschließlich verschlossene Kammern mit analytischen Hilfsmitteln vorliegen, nicht jedoch eine geöffnete Kammer mit einem Trockenmittel.

Weiterhin lässt sich, insbesondere bei Verwendung einer zumindest weitgehend gegenüber Umwelteinflüssen stabilen Testchemie, auch auf bessere und präzisere Verbindungstechniken zur Herstellung des Gehäuses zurückgreifen. So ist es besonders bevorzugt, wie unten noch näher ausgeführt wird, wenn das analytische Magazin ein Gehäuse mit mindestens zwei Teilen aufweist, insbesondere mindestens zwei Magazinhälften. Die beiden Teile müssen dabei nicht notwendigerweise gleich ausgestaltet sein. Das Gehäuse, insbesondere die mindestens zwei Teile gemeinsam, können die mindestens zwei Kammern bilden. Besonders bevorzugt ist es, wenn die mindestens zwei Teile miteinander durch ein Verfahren ohne Verwendung eines Klebemittels verbunden sind. Insbesondere bieten sich hier stoffschlüssige Verbindungsverfahren ohne Klebemittel an. Besonders bevorzugt aufgrund der hohen Präzision und der geringen Verursachung von Verunreinigungen ist die Verwendung mindestens eines Laserschweißverfahrens. Mit einem Laserschweißverfahren lassen sich gleichmäßige Schweißnähte mit einer geringen Breite und hohen Präzision erzielen, wobei das Verfahren gleichzeitig thermisch gut kontrollierbar und lokalisierbar sowie praktisch verunreinigungsfrei durchführbar ist.

Insbesondere bei Verwendung eines Laserschweißverfahrens bietet es sich an, ein erstes der mindestens zwei Teile transparent auszugestalten und ein zweites der mindestens zwei Teile absorbierend auszugestalten. Dabei können vorzugsweise die mindestens zwei Teile denselben Grundwerkstoff aufweisen, jedoch jeweils mit einer unterschiedlichen Absorption für Licht im sichtbaren und/oder infraroten und/oder ultravioletten Spektralbereich. Beispielsweise können die mindestens zwei Teile eine unterschiedliche Absorption in einem Spektralbereich zwischen 500 und 1200 nm aufweisen, insbesondere in einem Spektralbereich von 700 bis 1100 nm oder 700 bis 1000 nm, in welchem

übliche, für das Laserschweißen verwendete Laser emittieren, beispielsweise Halbleiterlaser und/oder Nd:YAG-Laser. Um eine unterschiedliche Transparenz beziehungsweise Absorption der mindestens zwei Teile zu erreichen, kann ein Grundwerkstoff der Teile unterschiedlich angefärbt werden, beispielsweise indem der Grundwerkstoff (beispielsweise Polycarbonat, PC) eines der Teile mit einem Farbstoff versetzt wird, um eine Transparenz zu vermindern.

Wird ein Schweißverfahren zur Verbindung der mindestens zwei Teile verwendet, so können allgemein die Schweißnähte beispielsweise eine Breite von maximal 0,5 mm aufweisen, insbesondere von maximal 0,3 mm und besonders bevorzugt von maximal 0,2 mm. Auch dies trägt zur Erhöhung der Packungsdichte bei. Insbesondere macht sich hier wiederum vorteilhaft bemerkbar, wenn die Testchemie zumindest weitgehend gegenüber Umwelteinflüssen stabil ist, da in diesem Fall ein Feuchtigkeitsübertritt von einer Kammer in eine andere Kammer weitgehend irrelevant ist.

Allgemein lassen sich für das Gehäuse, insbesondere für die mindestens zwei Teile des Gehäuses, beispielsweise für die zwei Magazinhälften, eine Vielzahl von Materialien einsetzen. Insbesondere können thermoplastische Kunststoffe eingesetzt werden. Ein besonderer Vorteil der Erfindung, insbesondere bei Verwendung einer gegenüber Umwelteinflüssen zumindest weitgehend stabilen Testchemie, besteht jedoch darin, dass diese Kunststoffe keine besonderen Anforderungen hinsichtlich einer Undurchlässigkeit gegenüber Feuchtigkeit erfüllen müssen. So können beispielsweise auch Materialien verwendet werden, welche eine Permeabilität gegenüber Wasserdampf aufweisen. Dementsprechend kann die Auswahl und Gestaltung der Kunststoffe beispielsweise nach anderen Kriterien vorgenommen werden, beispielsweise nach einer Verarbeitbarkeit in einem speziellen Formgebungsprozess, beispielsweise beim Spritzgießen. Auch kann auf kostengünstige Werkstoffe zurückgegriffen werden. Beispielsweise lassen sich einer oder mehrere der folgenden Kunststoffe einsetzen: PC (Polycarbonat); ABS (Acrylnitril-Butadien-Styrol); COC (Cyclo-Olefin-Copolymere); PMMA (Polymethylmethacrylat); PS (Polystyrol); PET (Polyethylenterephthalat). Diese Materialien weisen hinsichtlich ihrer Verarbeitungseigenschaften und/oder hinsichtlich ihrer Kosten Vorteile auf, sind jedoch grundsätzlich nur schlecht verwendbar, wenn auch die Forderung nach einer Dampfdichtigkeit erfüllt sein muss.

PC weist beispielsweise eine hohe Beständigkeit gegenüber ionisierender Strahlung auf und eine hohe Transparenz für ein breites Spektrum. Es handelt sich um einen kostengünstigen Massenwerkstoff, welcher jedoch eine vergleichsweise hohe Durchlässigkeit für Wasserdampf aufweist. Da diese Durchlässigkeit jedoch grundsätzlich, insbesondere bei Verwendung der stabilen Testchemie, weitgehend irrelevant ist, und da die Verarbeitungseigenschaften dieses Werkstoffs in der Praxis besonders gut sind, ist dieser Werkstoff im Rahmen der vorliegenden Erfindung besonders bevorzugt.

ABS ist sehr gut zu verarbeiten und insbesondere sehr gut spritzgießbar, so dass auch die Verwendung dieses Werkstoffs vorteilhaft ist. Auch dieser Werkstoff weist eine vergleichsweise gute Transparenz für ein breites Lichtspektrum auf sowie geringe Kosten.

COC weist zwar eine hohe Transparenz in einem weiten Spektralbereich vom ultravioletten Licht bis hin zum infraroten Spektralbereich auf und stellt eine gute Dampfsperre bereit, ist jedoch vergleichsweise teuer und nur mäßig stabil gegen überionisierender Strahlung.

PMMA weist eine lediglich geringe oder gar keine Eigenfluoreszenz im ultravioletten Spektralbereich auf sowie eine gute Transparenz für ein breites Lichtspektrum. Die hohe Dampfdurchlässigkeit dieses Werkstoffs ist, insbesondere bei Verwendung der stabilen Testchemie, tolerierbar, und es handelt sich um einen kostengünstigen Werkstoff. Auch dieses Material kann daher im Rahmen der vorliegenden Erfindung vorteilhaft eingesetzt werden.

PS ist gut zu verarbeiten, insbesondere durch ein Spritzgießverfahren. Es weist eine gute Transparenz für ein breites Lichtspektrum auf. Zudem handelt es sich um einen kostengünstigen Massenkunststoff. Insgesamt ist also auch dieses Material im Rahmen der vorliegenden Erfindung gut einsetzbar.

Mit dieser erweiterten und nicht mehr durch die Forderung nach Dampfundurchlässigkeit eingeschränkten Materialauswahl ist es grundsätzlich möglich, die vorzugsweise mindestens zwei Teile des Gehäuses des analytischen Magazins mittels Laserschweißens zu verbinden, anstelle eines herkömmlichen eines Ultraschallschweißens und/oder eines Klebeverfahrens, welches für undurchsichtige Werkstoffe übrig bliebe. Gleiche Werkstoffe lassen sich beispielsweise mittels eines Lasers sehr einfach verschweißen, beispielsweise PC mit PC und/oder COC mit COC usw., insbesondere wenn ein Teil lichtabsorbierend für die Laserwellenlänge ist, beispielsweise durch eine entsprechende Anfärbung und/oder Dotierung, und wobei das andere Teil transparent oder transparenter ausgestaltet ist. Zwar lassen sich grundsätzlich auch opake Teile so durchstrahlen, dass eine Schweißung möglich ist, aber die Schweißnähte werden dann in der Regel gröber und brauchen mehr Platz. Bei hoher Transparenz und glatten Oberflächen der miteinander zu verbindenden Teile lassen sich hingegen sehr schmale Schweißnähte erzielen, beispielsweise Schweißnähte mit der oben genannten Breite von 0,3 mm. Diese geringen Schweißnähte erlauben es, das analytische Magazin sehr klein zu gestalten, beispielsweise mit den oben beschriebenen bevorzugten Packungsdichten. Weiterhin lässt sich beim Laserschweißen die Bildung von Staub vermeiden, welche üblicherweise bei anderen Schweißverfahren auftritt, beispielsweise beim Ultraschallschweißen. Weiterhin treten keine Vibrationen auf, welche Strukturen oder Teile des analytischen Magazins in einer Resonanz versetzen könnten. Es sind auch keine Zusatzwerkstoffe nötig, wie beispielsweise Klebstoffe, welche das Innere der Kammern und/oder die analytischen Hilfsmitteln verunreinigen könnten, so dass beispielsweise eine Hydrophilie von Lanzetten oder Microsamplern gefährdet wäre.

Auch werden insbesondere durch Verwendung des Laserschweißens und/oder der bevorzugten Kunststoffe, neue Verfahren zum Verschließen und/oder Versiegeln des analytischen Magazins, beispielsweise zur Versiegelung eines fertigen analytischen Magazins, möglich. Bislang werden in vielen Fällen analytische Magazine mit heißklebefähigen Folien thermisch verschlossen. Die dabei verwendeten Heißkleber können jedoch unter Umständen die analytischen Hilfsmittel beeinflussen. So können beispielsweise Dämpfe des Heißklebers Lanzetten und/oder Microsampler beeinflussen, beispielsweise deren Hydrophilie beeinträchtigen. Insbesondere unter Verwendung des Laserschweißverfahrens und/oder der vorgeschlagenen Materialien, ist es jedoch möglich, alternativ oder zusätzlich zu beispielsweise Metallfolien, z.B. Aluminiumfolien, zur Versiegelung des analytischen Magazins eine oder mehrere Kunststofffolien zu verwenden, welche beispielsweise mittels eines Laser angeschweißt statt angeklebt werden können.

Insgesamt bietet die neu gewonnene Freiheit hinsichtlich der Auswahl der Materialien somit eine Grundlage für ein erheblich kompakteres System. Dies ist nicht nur dadurch bedingt, dass das analytische Magazin mit einem erheblich geringeren Bauraum und/oder einer erheblich höheren Packungsdichte ausgestaltet werden kann. Das analytische Magazin kann auch in seiner Herstellung und/oder seiner Handhabung stark vereinfacht und kostengünstiger ausgestaltet werden.

Wie oben dargestellt, wird neben dem analytischen Magazin in einer oder mehreren der oben beschriebenen Ausführungsformen weiterhin ein analytisches System zum Nachweis mindestens eines Analyten in einer Körperflüssigkeit vorgeschlagen. Das analytische System kann beispielsweise das oben genannte Messsystem sein oder ein derartiges Messsystem umfassen. Das analytische System ist eingerichtet, um mindestens ein analytisches Magazin in mindestens zwei unterschiedlichen Orientierungen aufzunehmen. Das analytische Magazin kann insbesondere gemäß einer oder mehreren der oben beschriebenen Ausführungsvarianten ausgestaltet sein.

Dabei ist jedoch das oben beschriebene Merkmal des analytischen Magazins, dass dieses eingerichtet ist, um die analytischen Hilfsmittel nach der Remagazinierung in den Kammern zu haltern, im Rahmen des vorgeschlagenen analytischen Systems nicht notwendigerweise erforderlich, sondern das analytische System kann auch derart realisiert werden, dass das analytische Magazin die analytischen Hilfsmittel nach der Remagazinierung nicht in den Kammern haltert. Auf eine Halterung in den Kammern nach der Remagazinierung, beispielsweise durch einen Kraftschluss, insbesondere einen Reibschluss, kann in diesem Fall verzichtet werden, auch wenn eine derartige Halterung nach wie vor bevorzugt ist. Ist keine derartige Halterung vorgesehen, so können die analytischen Hilfsmittel beispielsweise durch Komponenten des analytischen Systems, beispielsweise Wände einer Aufnahme für das analytische Magazin, in den Kammern gehaltert werden.

Das analytische System kann beispielsweise eine Aufnahme aufweisen, in welche das analytische Magazin in den mindestens zwei Orientierungen aufnehmbar ist. Wie oben dargestellt, kann das analytische System beispielsweise mindestens eine Schnittstelle bereitstellen, beispielsweise eine Schnittstelle mit mechanischen und/oder elektrischen und/oder optischen Funktionen, wobei beispielsweise dieselbe Schnittstelle mit den verschiedenen Teil-Magazinen in den unterschiedlichen Orientierungen zusammenwirken kann, beispielsweise jeweils mit demjenigen Teil-Magazin, welches sich in der aktuellen Orientierung des analytischen Magazins in einer Applikationsposition bzw. Applikationsorientierung befindet und in der aktuellen Orientierung analytische Hilfsmittel an das analytische Magazin bereitstellen kann. Wie oben dargestellt, kann die mechanische Schnittstelle beispielsweise ein Weitertakten des analytischen Magazins bewirken und/oder mindestens einen Aktor zur Ankopplung an das in der Applikationsposition befindliche analytische Hilfsmittel bereitstellen und/oder ein Messsystem zur Durchführung einer Messung mit einem Testelement des in der Applikationsposition bzw. Applikationsebene befindlichen analytischen Hilfsmittels bereitstellen. Bezüglich weiterer möglicher Ausgestaltungen des analytischen Systems und/oder des analytischen Magazins kann auf die obige Beschreibung verwiesen werden.

Das analytische System kann weiterhin eingerichtet sein, um mittels der in dem analytischen Magazin aufgenommenen analytischen Hilfsmittel mindestens eine Probennahmebewegung durchzuführen. Das analytische System ist weiterhin eingerichtet, um eine Remagazinierung der analytischen Hilfsmittel durchzuführen. Beispielsweise kann das analytische System eingerichtet sein, um zunächst an ein in einer Applikationsposition befindliches analytisches Hilfsmittel, was auch ein Teil-Hilfsmittel umfassen kann, anzukoppeln, mit diesem eine Probennahmebewegung durchzuführen, anschließend eine Remagazinierung durchzuführen und anschließend wiederum von diesem analytischen Hilfsmittel abzukoppeln. Für weitere Ausführungsformen und Optionen kann auf die obige Beschreibung verwiesen werden.

Das analytische System kann insbesondere mindestens eine Applikationsposition und/oder Applikationsebene aufweisen. Unter einer Applikationsposition bzw. Applikationsebene wird dabei eine Position bzw. Ebene verstanden, in welcher mindestens eine Kammer des analytischen Magazins bereitgestellt werden kann, damit diese Kammer und/oder das in dieser Kammer aufgenommene analytische Hilfsmittel für den analytischen Nachweis, beispielsweise die Probennahmebewegung und/oder die Analyse der Probe, verwendet werden kann. Dabei können, wie oben dargestellt, auch mehrere Applikationspositionen vorgesehen sein, beispielsweise mehrere Applikationspositionen in einer Applikationsebene. Beispielsweise können, wie oben dargestellt, die Probennahmebewegung und die Messung der Probe an unterschiedlichen Orten erfolgen.

Das analytische System kann insbesondere eingerichtet sein, um in mindestens zwei unterschiedlichen Orientierungen des analytischen Magazins auf mindestens eine Kammer und/oder mindestens ein analytisches Hilfsmittel in der Applikationsposition zuzugreifen und den mindestens einen Analyten mittels dieser Kammer und/oder mittels des analytischen Hilfsmittels in der Applikationsposition nachzuweisen. Somit kann beispielsweise eine Ankopplung für eine Probennahmebewegung und/oder eine Remagazinierung erfolgen und/oder eine Ankopplung für Messzwecke, beispielsweise mittels einer elektrischen Messung und/oder einer optischen Messung oder ähnlicher Messungen, welche beispielsweise auf die Art und Funktionsweise der Testchemie angepasst sein können.

Oben wurde bereits dargestellt, dass das analytische Magazin insbesondere derart ausgestaltet sein kann, dass dieses mindestens ein Anzeigeelement umfasst, welches mindestens eine Information über eine aktuelle Orientierung an das analytische System liefern kann. Das analytische System kann entsprechend ausgerüstet sein, um eine oder mehrere der folgenden Informationen zu erfassen: Eine Orientierung des analytischen Magazins; eine Anzahl von in der aktuellen Orientierung bereits verwendeten analytischen Hilfsmitteln des analytischen Magazins; eine Anzahl von in der aktuellen Orientierung noch verbleibenden Hilfsmitteln des analytischen Magazin; eine Information über bereits verwendete Orientierungen des analytischen Magazins; eine Information über noch verbleibende Orientierungen des analytischen Magazins. Diese Erfassung kann beispielsweise mittels eines oder mehrerer der oben beschriebenen Anzeigeelemente erfolgen. Das mindestens eine Anzeigeelement kann beispielsweise unveränderlich ausgestaltet sein, beispielsweise mittels entsprechender starrer Elemente, welche eine Orientierung anzeigen. Alternativ oder zusätzlich kann jedoch auch mindestens ein Anzeigeelement vorgesehen sein, welches variabel ausgestaltet ist, beispielsweise in Form eines variablen mechanischen und/oder elektronischen Informationsträgers. Auf diese Weise kann beispielsweise vor und/oder nach jeweils einer Benutzung einer Kammer und/oder eines analytischen Hilfsmittels das Anzeigeelement, welches als Informationsträger wirkt, verändert werden, um beispielsweise eine Information der oben beschriebenen Art zu aktualisieren. Verschiedene Ausführungsbeispiele werden unten näher beschrieben. Das analytische System kann insbesondere weiterhin eingerichtet sein, um die mindestens eine Information der genannten Art an einen Benutzer zu übermitteln, beispielsweise mittels einer optischen und/oder akustischen und/oder haptischen Anzeige.

Das analytische System kann insbesondere eingerichtet sein, um einen Benutzer zu einem Wechsel einer Orientierung des analytischen Magazins aufzufordern. Der Wechsel der Orientierung kann beispielsweise automatisch erfolgen. Besonders bevorzugt ist jedoch ein manueller Wechsel der Orientierung. Das analytische System kann insbesondere, beispielsweise aufgrund der oben beschriebenen mindestens einen Information, erkennen, dass in einer aktuellen Orientierung keine unbenutzten analytischen Hilfsmittel mehr zur Verfügung stehen. Entsprechend kann das analytische System beispielsweise einen Benutzer auffordern, die Orientierung des analytischen Magazins zu wechseln, beispielsweise indem ein Systemgehäuse des analytischen Systems geöffnet wird, das analytische Magazin entnommen wird, gedreht wird und wieder ins Gehäuse eingefügt wird.

Das analytische Magazin und das analytische System gemäß einer oder mehreren der oben beschriebenen Ausführungsformen weisen gegenüber bekannten analytischen Magazinen und bekannten analytischen Systemen eine Reihe von Vorteilen auf. So lassen sich insbesondere analytische Systeme realisieren, welche dem beschriebenen Integrationsgedanken, bei welchem mindestens zwei Systemfunktionen miteinander kombiniert werden, genüge leisten. Insbesondere lassen sich die Systemfunktionen einer Probennahme und Probenanalyse mittels ein und desselben analytischen Magazins realisieren. Gleichzeitig werden jedoch die beschriebenen Anforderungen auf gute Weise erfüllt, insbesondere die Anforderungen an ein Microsampler-Magazin. So lassen sich Analyten innerhalb des Magazins qualitativ und/oder quantitativ nachweisen, beispielsweise auf optischem und/oder elektrochemischem Wege. Gleichzeitig kann aufgrund einer möglichen Einbringung einer Testchemie ins Innere der Kammern eine Beständigkeit der Testchemie gewährleistet werden. Da vorzugsweise die Testchemien jeweils in unterschiedlichen Kammern angeordnet sind, lassen sich Querkontaminationen zwischen einzelnen Microsamplern vermeiden.

Gleichzeitig lassen sich die genannten analytischen Magazine auf einfache und kostengünstige Weise ausgestalten. Es lässt sich weiterhin eine besonders kompakte Form der Magazinierung gewährleisten, beispielsweise mittels einer doppelten Gehäuseform. Die kompakte Form der Magazinierung erlaubt es beispielsweise, analytische Hilfsmittel, insbesondere Disposables, übereinander zu magazinieren. Beispielsweise kann bei einer Magazinierung in einem Scheibenmagazin ein Radius der Scheibe auf 25 analytische Hilfsmittel abgestimmt sein. Aufgrund einer doppelten Gehäuseform wird jedoch eine Magazinierung von 50 Disposables möglich, beispielsweise unter Verwendung eines entsprechend kleineren oder konstanten Durchmessers der Scheibe. Nach dem Verbrauch der 25 Disposables kann ein Benutzer entsprechend aufgefordert werden, das analytische Magazin herauszunehmen und umzudrehen. Dabei kann eine geräteseitige Schnittstelle, welche ein Ankoppeln des Systems an das analytische Magazin erlaubt, für alle analytischen Hilfsmittel, beispielsweise für alle 50 analytischen Hilfsmittel unverändert bleiben.

Durch dieses unveränderte Verbleiben einer optionalen Schnittstelle des analytischen Systems, welche zur Ankopplung des analytischen Magazins und/oder der analytischen Hilfsmittel an das analytische System dient, ergeben sich eine Vielzahl von Vorteilen. Insbesondere ergeben sich diese Vorteile bei der Verwendung der genannten Microsampler, bei denen beispielsweise eine Vermessung des Testfeldes nach einer Remagazinierung im Magazingehäuse erfolgen kann. Derartige Systeme erfordern in der Regel eine genaue Positionierung der im Magazingehäuse aufgenommenen, beispielsweise fixierten analytischen Hilfsmittel, beispielsweise der Testelemente, relativ zu einem Messsystem, beispielsweise einer Messoptik. Zudem muss diese Positionierung stets reproduzierbar gewährleistet sein. Aufgrund der Verwendung des analytischen Magazins, beispielsweise des oben beschriebenen Wendemagazins, können diese Schnittstellen und/oder das mindestens eine Messsystem jedoch für alle Orientierungen identisch ausgestaltet werden und/oder unverändert im analytischen System erhalten bleiben, insbesondere für alle analytischen Hilfsmittel. Hieraus ergeben sich besondere Vorteils insbesondere für analytische Systeme, die auf einer Remagazinierung beruhen.

Kurze Beschreibung der Figuren

Weitere Einzelheiten und Merkmale ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktion einander entsprechende Elemente.

Im Einzelnen zeigt:

| | |
|---|---|
| Figur 1 | ein erstes Ausführungsbeispiel eines erfindungsgemäßen analytischen Magazins in Form eines Scheibenmagazins; |
| Figuren 2A bis 2D | ein zweites Ausführungsbeispiel eines erfindungsgemäßen Scheibenmagazins; |
| Figur 3 | ein Ausführungsbeispiel eines linearen analytischen Magazins; und |
| Figur 4 | ein Ausführungsbeispiel eines analytischen Systems. |

Ausführungsbeispiele

In Figur 1 ist ein erstes Ausführungsbeispiel eines erfindungsgemäßen analytischen Magazins 110 in einer perspektivischen Schnittdarstellung gezeigt. Das analytische Magazin 110 ist dabei, wie im Folgenden erläutert, exemplarisch als Wendemagazin gemäß dem ersten Aspekt ausgestaltet. Unabhängig von dieser Ausgestaltung kann das analytische Magazin 110 gemäß Figur 1 auch bei Ausführungsbeispiel für den zweiten und dritten Aspekt dienen.

Die Darstellung in Figur 1 zeigt weiterhin einen Teil eines Gehäuses 112 eines analytischen Systems 114, mit einer Fingeröffnung 116, durch welche eine Probennahmebewegung erfolgen kann. Das analytische Magazin 110 ist im dargestellten Ausführungsbeispiel als Wendemagazin in Form eines runden Scheibenmagazins ausgestaltet und weist zwei Hilfsmittelebenen 118, 120 auf. Diese beiden Hilfsmittelebenen 118, 120 sind dabei jeweils parallel übereinander angeordnet und verlaufen parallel zur Ebene der Scheibe des analytischen Magazins 110. Das analytische Magazin 110 weist ein Gehäuse 122 mit einer großen, runden Mittelöffnung 124 auf. In dem Gehäuse 122 sind in jeder Hilfsmittelebene 118, 120 eine Mehrzahl von Kammern 126 aufgenommen. Die Kammern 126 sind dabei in dem gezeigten Ausführungsbeispiel radial und äquidistant in dem Gehäuse 122 angeordnet. Das Gehäuse 122 ist dabei in der Darstellung gemäß Figur 1 geöffnet gezeigt, so dass die Kammern 126 der ersten Hilfsmittelebene 118 nach oben weisen und geöffnet sind. Die Kammern 126 der zweiten Hilfsmittelebene 120 liegen jeweils um eine halbe Einheit verdreht unterhalb der Kammern 126 der ersten Hilfsmittelebene 118. Das analytische Magazin 110 kann somit aus zwei identischen Halbebenen zusammengesetzt sein, welche gegeneinander montiert sind und jeweils um eine halbe Einheit, d.h. einen halben Winkelabstand der Kammer 126 gegeneinander verdreht sind.

In den Kammern 126 sind im dargestellten Ausführungsbeispiel analytische Hilfsmittel 128 aufgenommen. Diese analytischen Hilfsmittel 128 sind dabei jeweils aus Teil-Hilfsmittel 130, zusammengesetzt. Ein erstes Teil-Hilfsmittel 130 umfasst dabei pro Kammer 126 jeweils einen in den Kammern 126 radial gelagerten Microsampler 132. Jeder Microsampler 132 umfasst wiederum eine Lanzette 134 und ein von den Lanzetten 134 radial nach innen verlaufendes Kapillarelement 136. An ihrem der Mittelöffnung 124 zuweisenden Ende weisen die Microsampler 132 jeweils Kopplungselemente 138 auf, welche im dargestellten Ausführungsbeispiel in Form von Ösen ausgestaltet sind, in welche wiederum ein (in der Figur 1 nicht dargestellter) Aktor des analytischen Systems 114 eingreifen kann, um eine Probennahmebewegung durchzuführen.

Dabei sind die Kammern 126 in radialer Richtung gekrümmt ausgestaltet. Die in den Kammern 126 gelagerten Microsampler 132 werden daher um eine Achse senkrecht zu ihrer Längserstreckung und parallel zur Ebene des analytischen Magazins 110 gebogenen. Die Microsampler 132 sind vorzugsweise, wie unten noch näher ausgeführt wird, aus einem metallischen Werkstoff, beispielsweise einem Metallblech, gefertigt. Dadurch weisen die Microsampler 132 eine gewisse Flexibilität auf und werden bei der Krümmung durch ihre Rückstellkräfte gegen die Kammerwände der

Kammern 126 gedrückt. Hierdurch entsteht ein Reibschluss, welcher die Microsampler 132 in den Kammern 126 hält und welcher für eine unten näher erläuterte Probennahmebewegung von einem Aktor des analytischen Systems 114 überwunden werden muss. Nach einer unten ebenfalls noch näher erläuterten Remagazinierung werden die Microsampler 132 durch den Reibschluss wieder in den Kammern 126 gehaltert. Wie oben beschrieben, können, alternativ oder zusätzlich, auch andere Halterungsmechanismen eingesetzt werden. Die bezüglich des erfindungsgemäßen analytischen Magazins 110 vorgesehene Halterung, welche jedoch hinsichtlich des analytischen Systems 114 optional ist, bietet insbesondere den Vorteil, dass analytische Hilfsmittel 128 und/oder Teil-Hilfsmittel, wie beispielsweise die Microsampler 132, nach der Remagazinierung nicht aus den Kammern 126 herausfallen können, was beispielsweise zu einer Infektionsgefahr, zu hygienischen Problemen oder zu Sicherheitsproblemen führen könnte.

Weiterhin weisen die analytischen Hilfsmittel 128 im dargestellten Ausfuhrungsbeispiel jeweils ein Testelement 140 auf. Dieses Testelement 140 umfasst eine Testchemie 142 in Form eines Testfeldes 144, welches im dargestellten Ausführungsbeispiel in das Gehäuse 122 integriert ist. Jedes Testfeld 144 weist dem Innenraum der jeweils zugehörigen Kammer 126 zu. Wie unten näher erläutert wird, kann das Testfeld 144 beispielsweise dadurch hergestellt werden, dass auf eine Öffnung in dem Gehäuse 122 des analytischen Magazins 110 ein Testfeld 144 eingelegt wird, so dass dessen Oberfläche dem Inneren der Kammer 126 zuweist. Dabei können für mehrere oder alle Kammern 126 auch gemeinsame Testfelder verwendet werden, deren Begrenzung jeweils durch die Öffnungen in den Kammern 126 definiert werden. Die Testelemente 140 bilden weitere Teil-Hilfsmittel 130 der analytischen Hilfsmittel 128.

Das analytische System 114 ist eingerichtet, um mittels des analytischen Magazins 110 bzw. der darin aufgenommenen analytischen Hilfsmittel 128 mindestens einen Analyten in einer Körperflüssigkeit nachzuweisen. Dabei erfolgt ein integrierter Nachweis mittels der Microsampler 132, welcher im Folgenden exemplarisch beschrieben werden soll. So ist in Figur 4 in stark schematisierter Ausführungsform ein analytisches System 114 gezeigt, in welchem ein analytisches Magazin 110, beispielsweise gemäß dem in Figur 1 dargestellten Typ, aufgenommen ist. Das analytische System 114 kann zu diesem Zweck beispielsweise eine oder mehrere Aufnahmen aufweisen, in welchen das analytische Magazin 110 in den unterschiedlichen Orientierungen aufnehmbar ist und vorzugsweise nur in diesen Orientierungen. Die Aufnahme kann beispielsweise mindestens einen Aufnahmeschacht und/oder mindestens eine Vertiefung aufweisen und ist vorzugsweise durch einen Benutzer von außen zu öffnen, um das analytische Magazin 110 einzulegen und/oder die Orientierung des analytischen Magazins 110 zu ändern, beispielsweise dieses zu drehen. Das analytische System 114 ist in dem in Figur 4 dargestellten Fall eingerichtet, um die analytischen Hilfsmittel 128 der ersten Hilfsmittelebene 118 zu verwenden. Das analytische System 114 kann mehrere Schnittstellen aufweisen, um das analytische Magazin 110 bzw. die darin befindlichen analytischen Hilfsmittel 128 zu nutzen.

So kann beispielsweise eine Positionierungsvorrichtung 146 vorgesehen sein, welche eingerichtet ist, um das analytische Magazin 110 entsprechend zu positionieren. Diese Positionierungsvorrichtung 146 kann beispielsweise ein oder mehrere Motoren oder ähnliches umfassen, welche an entsprechenden Transportelementen des analytischen Magazins 110 angreifen können. Weiterhin kann das analytische System 114 einen oder mehrere Aktoren 148, 150 umfassen, welche beispielsweise vollständig oder teilweise in der Mittelöffnung 124 angeordnet sein können. Alternativ oder zusätzlich können auch weitere Systemkomponenten des analytischen Systems 114 vollständig oder teilweise in dieser Mittelöffnung 124 angeordnet sein, so dass insgesamt die Baugröße des analytischen Systems 114 vermindert werden kann.

Die Aktoren 148, 150 können beispielsweise ein oder mehrere Aktorstangen 152 und/oder Stößel und/oder Pins umfassen, welche eingerichtet sind, um über die Kopplungselemente 138 an ein in einer Applikationsposition 154 befindliches analytisches Hilfsmittel 128 und/oder Teil-Hilfsmittel 130 beispielsweise einen Microsampler 132, anzukoppeln.

Ein Analysezyklus kann beispielsweise die folgenden Schritte umfassen. Zunächst kann mittels der Positionierungsvorrichtung 146 die Positionierung einer neuen, bislang unbenutzten Kammer 126 in der Applikationsposition 154 erfolgen. Anschließend erfolgt eine Ankopplung des Aktors 148 bzw. der Aktorstange 152 an das in dieser Kammer 126 befindliche analytische Hilfsmittel 128, beispielsweise indem ein Haken der Aktorstange 152 in die Öse des Kopplungselements 138 einhakt. Für diesen Zweck kann beispielsweise die Aktorstange 152 eine Versiegelung an der der Mittelöffnung 124 zuweisenden Seite der Kammer 126 in der Applikationsposition 154 durchstoßen. Anschließend erfolgt eine Probennahmebewegung, wobei eine Kraft des Aktors 148 den Reibschluss des Microsamplers 132 in der Kammer 126, durch welchen der Microsampler 132 gehaltert wird, überwindet. Dabei wird der Microsampler 132 hin zur Fingeröffnung 116 und durch diese hindurch bewegt, hin zu einer Hautoberfläche eines Probanden. Dieser Teil der Probennahmebewegung kann auch als Stechbewegung bezeichnet werden. Die Lanzette 134 des analytischen Hilfsmittels 128 durchdringt dadurch die Haut des Probanden und erzeugt eine Probe von Blut und/oder interstitieller Flüssigkeit. Bereits beim Eindringen und/oder im eingedrungenen Zustand und/oder in einer sich anschließenden Rückbewegung der Lanzette 134, welche wiederum durch den Aktor 148 bzw, die Aktorstange 152 angetrieben wird, sammelt das Kapillarelement 136 die Probe auf und transportiert diese in Richtung der Kapillare, beispielsweise des Kapillarspalts dieses Kapillarelements 136. Es erfolgt eine Rückbewegung des Microsamplers 132, wobei dieser wieder in der Kammer 126 in der Applikationsposition 154 remagaziniert wird. Dabei wird der Microsampler 132 durch die Krümmung der

Kammer 126 wieder gebogen und erneut gegen die Kammerwände der Kammer 126 gepresst, so dass erneut der oben beschriebene Reibschluss entsteht und der Microsampler 132 nach der Remagazinierung wieder in der Kammer 126 gehaltert wird. Alternativ kann der Reibschluss auch erst nach der Remagazinierung auftreten, wobei vor Verwendung des analytischen Hilfsmittels 128 der Microsampler 132 beispielsweise locker in der Kammer 126 aufgenommen sein kann. In diesem Fall kann sich die Position des Microsamplers 132 beispielsweise nach der Remagazinierung von einer Position des Microsampler 132 vor Durchführung der Probennahmebewegung unterscheiden, so dass beispielsweise erst nach der Remagazinierung die oben beschriebene Krümmung und damit der Reibschluss auftritt.

Bereits während des Sammelvorgangs und/oder anschließend an den Sammelvorgang erfolgt eine Übertragung der aufgenommenen Probe auf das Testfeld 144 im Inneren der Kammer 126. Zu diesem Zweck kann im Inneren der Kammer 126 der Microsampler 132 durch den zweiten Aktor 150 und/oder eine in diesem Aktor 150 umfasste Aktorstange 156 zusätzlich auf das Testfeld 144 gedrückt werden, um einen Übertrag der Probe auf das Testfeld 144 zu verbessern. Alternativ oder zusätzlich kann dieser Übertrag jedoch auch durch eine einfache Annäherung zwischen Microsampler 132 und Testfeld 144 bewirkt werden, beispielsweise durch eine entsprechende Formung der Wände der Kammern 126 und/oder durch eine entsprechende Bewegung der Aktorstange 152. Zu diesem Zweck können weitere Öffnungen vorgesehen sein. Die Außenseite der Kammern 126 kann zusätzlich versiegelt sein, um die analytischen Hilfsmittel 128 im Inneren der Kammern 126 zusätzlich zu schützen.

Nach Übertragung der Probe auf das Testfeld 144 kann beispielsweise die in diesem Testfeld 144 umfasste Testchemie 142 eine Farbreaktion und/oder eine andere nachweisbare Reaktion durchfuhren, welche mittels eines Messsystems 158 des analytischen Systems 114 nachgewiesen bzw. ausgewertet werden kann. Das analytische System 114 kann darüber hinaus weitere Komponenten umfassen. So kann beispielsweise eine Steuerung 160 enthalten sein, welche beispielsweise die beschriebenen Systemkomponenten ansteuern und/oder auswerten kann. Diese Steuerung 160 kann beispielsweise auch über eine Schnittstelle 162, beispielsweise eine oder mehrere Eingabe-/Ausgabe-Schnittstellen eine Kommunikation mit einem Benutzer ermöglichen, so dass ein Benutzer beispielsweise Informationen übermittelt erhalten kann und/oder dass analytische System 114 bedienen kann. Darüber hinaus kann das analytische Magazin 110 ein oder mehrere Anzeigeelemente 164 umfassen, welche beispielsweise mittels eines oder mehrerer Orientierungssensoren 166 des analytischen Systems 114 erfasst werden können. Auf diese Weise kann beispielsweise eine Winkelstellung des analytischen Magazins 110 erfasst werden und/oder eine Orientierung des analytischen Magazins 110.

Das analytische Magazin 110 ist, wie oben beschrieben, derart ausgestaltet, dass die analytischen Hilfsmittel 128 im dargestellten Ausführungsbeispiel in den unterschiedlichen Hilfsmittelebenen 118, 120 versetzt zueinander angeordnet sind. Dementsprechend liegt, wie in Figur 1 erkennbar ist, jeweils ein Testelementfenster 168 einer der beiden Hilfsmittelebenen 118, 120 zwischen zwei Kammern 126 der jeweils anderen der Hilfsmittelebene 118, 120. Auf diese Weise ist sichergestellt, dass mittels des Messsystems 150 jeweils, ungestört von den benachbarten Kammern 126 der jeweils anderen Hilfsmittelebene 118, 120, die Rückseite des Testfeldes 144 in der Applikationsposition 154 beobachtet werden kann. Die versetzte Anordnung der Kammern 126 erlaubt also einen beiderseitigen Zugriff auf die jeweils aktiven Testelemente 140 in der Applikationsposition 154, nämlich beispielsweise von der einen Seite her durch das Messsystem 158, beispielsweise eine auswertende Optik, und von der Gegenseite her durch die Aktorstange 156, beispielsweise einen entsprechenden Dorn, welcher nach dem Einstieg und der Probennahme durch den Microsampler 132 diesen in Kontakt mit der Testchemie 142 bringen kann. Die anderen analytischen Hilfsmittel 128 im analytischen Magazin 110 werden dabei vorzugsweise nicht freigelegt und bleiben durch ihre entsprechenden Versiegelungen bis zur Verwendung steril.

Die Microsampler 132 sind in dem Ausführungsbeispiel gemäß Figur 1 in den Kammern 126 aufgenommen, welche beispielsweise nach oben bzw. unten offen ausgestaltet sein können. Diese Eigenschaft kann wichtig sein bei einem Ankoppelmechanismus, welcher außerhalb einer Magazinebene untergebracht ist. Zur Stichbewegung kann, wie oben beschrieben, die Aktorstange 152, beispielsweise ein Aktorstößel, in radialer Richtung relativ zur Magazinscheibe des analytischen Magazins 110 an den Microsampler 132 ankoppeln und diesen innerhalb der Aussparung radial vor- und zurückschieben, um die oben beschriebene Probennahmebewegung durchzuführen. Zur Sicherstellung der Lagerungsdauer kann die Außenfläche des Magazins 110 durchgängig oder zumindest teilweise mit einer dünnen Versiegelung, beispielsweise in Form einer dünnen Dichtfolie, abgesiegelt werden, welche beispielsweise von der Aktorstange 152 und/oder der Lanzette 134 des Microsamplers 132 beispielsweise leicht durchtrennt werden kann.

Die Dichtfolie kann beispielsweise auch die Testelementfenster 168, welche als Messfenster wirken, überdecken. Um diese zu öffnen, kann ein zusätzlicher Aufreißmechanismus vorgesehen sein, welcher vor jedem Messvorgang das jeweilige Testelementfenster 168 freilegt. Ein derartiger Mechanismus ist in den Figuren nicht dargestellt, kann jedoch zusätzlich vorgesehen sein. Dieser Mechanismus kann beispielsweise beim Weitertakten des analytischen Magazins 110 wirksam werden. Hierzu kann beispielsweise eine versenkbare Klinge in die Vertiefung der Testelementfenster 168 eintauchen und die Folie infolge der Magazindrehung beiseite schieben. In einer anderen, alternativ oder zusätzlich einsetzbaren Ausführungsvariante, kann eine Suchbewegung des Aktorstößels in Form der Aktorstange 152 nach dem Kopplungselement 138 beispielsweise in Form des Pilotlochs und/oder der Öse im Microsampler 132, beispielsweise auch dazu genutzt werden, die Folie auf ganzer Länge aufzureißen, so dass der Aktuationsweg vorab freigelegt ist. Hierzu kann beispielsweise die Aktorstange 152 nach vorne durch die Folie hindurch greifen, auf den Microsampler 132, und dann nach hinten fahren, bis die Aktorstange 152 in das Pilotloch des Kopplungselements 138 einrastet.

Das analytische Magazin 110 kann beispielsweise mit Feuchte-unempfindlichen Testelementen 140 ausgestattet sein, beispielsweise gemäß der obigen Beschreibung des Standes der Technik. In diesem Fall können die Testelementfenster 168, beispielsweise die Messfenster, bereits beim Herstellprozess von ihrer Folie befreit werden und das gesamte analytische Magazin 119 zur Gewährleistung der Langzeithaltbarkeit in eine dichte Umverpackung, beispielsweise eine Folientüte eingebracht werden. Mit dem Aufreißen dieser Umverpackung durch einen Benutzer beginnt somit die Aufbrauchfrist zu laufen. Damit dann die Microsampler 132 und/oder andere Teil-Hilfsmittel 130 der analytischen Hilfsmittel 128 bzw. die analytischen Hilfsmittel 128 als Ganze in den Kammern 126 gehalten werden, können die Kammern 126 mit einer Abdeckscheibe versehen werden, welche zwar beispielsweise die Breite der Microsampler 132 überlappen, aber den Eingriff der Aktorstange 152 freilassen bzw. ermöglichen.

Weiterhin können an dem analytischen Magazin 110 Einleghilfen vorgesehen sein, welche auch ganz oder teilweise beispielsweise mit den Anzeigeelementen 164 kombiniert werden können. Diese Einlegehilfen können dem Kunden das richtige Einlegen des als Wendemagazin ausgestalteten analytischen Magazins 110 erleichtern. Hierfür können beispielsweise aus dem Stand der Technik bekannte Lösungen eingesetzt werden, wie beispielsweise Positionierlöcher, Positionieraussparungen, Positionierzapfen und/oder Kombination der genannten und/oder anderer Elemente, welche hier nicht weiter diskutiert werden sollen.

Allgemein wird darauf hingewiesen, dass das analytische Magazin 110 auch ohne die Anzeigeelemente 164 realisierbar ist oder mit anderes ausgestalteten festen oder variablen Anzeigeelementen 164. Da beispielsweise in dem analytischen System 114 ein Antriebsmotor vorgesehen sein kann, welcher das analytische Magazin 110 durchtaktet, kann beispielsweise, alternativ oder zusätzlich zur Verwendung eines Anzeigeelements 164, eine Zählung der Taktungen eine Information über bereits erfolgte Testvorgänge und/oder noch verbleibende Tests liefern. Die Erkennung einer bereits verwendeten Orientierung des analytischen Magazins 110 ist beispielsweise auch durch einen geräteseitigen Sensor, insbesondere eine Lichtschranke oder einen ähnlichen Sensor, vorstellbar.

Wiederum alternativ oder zusätzlich bietet das Anzeigeelement 164 auch die Möglichkeit neuer Verwendungen des analytischen Magazins 110. So kann beispielsweise ein Benutzer das analytische Magazin 110 auch zwischenzeitlich aus dem analytischen System 114 herausnehmen, beispielsweise um für einen Kurzurlaub eine ausreichende Anzahl an frischen analytischen Hilfsmitteln 128, beispielsweise Testelementen 140, bereitzustellen und/oder einzulegen. In diesem Fall könnte das analytische System 114 über das variable Anzeigeelement 164, welches in diesem Fall beispielsweise als variabler mechanischer und/oder elektronischer Informationsträger wirken kann, dem analytischen Magazin 110 und/oder dem analytischen System 114 eine Status-Information mitgeben. Beim Wiedereinlegen des zuvor benutzten analytischen Magazins 110 in das analytische System 114 könnte diese Status-Information zunächst ausgelesen und das analytische Magazin 110 mittels des Anzeigeelements 164 in die Position des nächsten frischen analytischen Hilfsmittels 128, beispielsweise des nächsten frischen Testelements 140, gebracht werden.

Das in Figur 1 dargestellte analytische Magazin 110 umfasst im dargestellten Ausführungsbeispiel zwei im Wesentlichen identische Magazinhälften oder besteht aus zwei im Wesentlichen identischen Magazinhälften, die durch ein Wenden des Magazins mit dem analytischen System 114 nacheinander in Eingriff gebracht werden können. Obwohl Wendemagazine ein ähnliches Bauvolumen einnehmen wie linear vergrößerte Magazine, erlaubt die kompakte Geometrie eine kleinere und/oder ergonomischere Bauform des analytischen Systems 114, welches beispielsweise als Messgerät ausgestaltet sein kann. Beispielsweise können auf diese Weise Volumina von weniger als 130 ml realisiert werden. Hierbei sind analytische Magazine 110 mit einer Beladung von 50 analytischen Hilfsmitteln 128 oder mehr erreichbar.

Ein Anwender kann das analytische Magazin 110 entsprechend beispielsweise einer Markierung, beispielsweise einer aufgedruckten Markierung, insbesondere einer Pfeilmarkierung, in das analytische System 114 einfügen. Beispielsweise kann dabei zunächst die erste Hilfsmittelebene 118 in einer Applikationsebene 170 angeordnet werden. In dieser Orientierung können die in dieser Hilfsmittelebene 118 in dem analytischen Magazin 110 aufgenommenen analytischen Hilfsmittel 128 nacheinander an das analytische System 114 bereitgestellt werden, beispielsweise jeweils durch ein Weitertakten des Magazins 110 um eine Kammer 126 mittels eine Transportmechanismus des analytischen Systems 114. Nach einer, beispielsweise durch die Anzahl der Kammern 126 in der ersten Hilfsmittelebene 118 des analytischen Magazins 110 vorgegebenen Anzahl von Messungen, beispielsweise 25 Messungen, ist die Kapazität dieser ersten Magazinhälfte aufgezehrt. Der Anwender kann dann beispielsweise über die Schnittstelle 162 eine Systeminformation zum Wenden des analytischen Magazins 110 erhalten. Daraufhin öffnet der Anwender das analytische System 114, indem beispielsweise eine entsprechende Klappe oder andere Art von Öffnung am Gehäuse 112 geöffnet wird. Das zur Hälfte verbrauchte analytische Magazin 110 kann dann entnommen und gewendet werden. Ein weiterer Aufdruck und/oder eine sonstige Markierung auf der Rückseite des analytischen Magazins 110 können zur Orientierung beim Wiedereinlegen helfen.

Durch eine geometrische Gestaltung des analytischen Magazins 110 lässt sich das Risiko eines fehlerhaften Einlegens vermeiden. Hierbei sind insbesondere Formen denkbar, welche die Magazin-Symmetrie innerhalb der Einlegeebene durchbrechen. Des Weiteren kann es hilfreich sein, den Benutzungsstatus des analytischen Magazins 110 zu detektieren, um eine entsprechende Anweisung an den Anwender ausgeben zu können. So kann beispielsweise eine Anzahl bereits verbrauchter analytischer Hilfsmittel 128 $N_{used}$ von Bedeutung sein. Daneben kann, alternativ oder zusätzlich, die Erkennung der verwendeten Magazinhälfte wichtig sein. Für diese Informationen kann beispielsweise das Anzeigeelement 164 am analytischen Magazin genutzt werden.

Beispielsweise kann die Steuerung 160 programmtechnisch eingerichtet sein, um derartige Informationen zu erhalten und/oder auszuwerten. Allgemein kann, beispielsweise durch entsprechende elektronische und/oder programmtechnische Umsetzung, beispielsweise ein mathematischer Schalter H eingeführt werden. Aus den Informationen über die bereits verbrauchten Tests $N_{used}$ und die verwendete Magazinhälfte lassen sich beispielsweise die verbliebenen Tests $N_{left}$ berechnen. Das folgende Rechenbeispiel beruht auf einer Magazinkapazität von 2 x 25 Tests:

$$N_{left} = (25 - N_{used}) + H \times 25$$

Der mathematische Schalter H nimmt den Wert H = 0 an, falls die zweite Magazinhälfte (also die zweite Hilfsmittelebene 120) angebrochen ist. Ansonsten nimmt H den Wert H = 1 an. Der Schalterwert H kann beispielsweise mittels eines Sensors ermittelt werden, beispielsweise mittels des Orientierungssensors 166, welcher beispielsweise die Anzeigeelemente 164 auf unterschiedlichen Seiten des analytischen Magazins 110 auslesen kann. Auf diese Weise kann beispielsweise erkannt werden, ob die zweite Magazinhälfte angebrochen ist oder nicht. Der Orientierungssensor 166 kann beispielsweise auf der aktiven und/oder auf der inaktiven Magazinhälfte nach Spuren einer vorherigen Nutzung suchen. So kann der Orientierungssensor 166 nicht nur, wie in Figur 4 angedeutet, auf der Unterseite des analytischen Magazins 110 angeordnet sein. Alternativ oder zusätzlich kann der Orientierungssensor 166 auch auf der gegenüberliegenden Seite angeordnet sein, also auf der von der Applikationsebene 170 abgewandten Seite und dort beispielsweise nach Spuren der vorherigen Nutzung suchen. Für den Orientierungssensor eignen sich beispielsweise herkömmliche Sensoren, wie insbesondere optische Sensoren (beispielsweise Lichtschranken oder ähnliche optische Sensoren) und/oder kapazitive und/oder induktive Sensoren. Auch andere Arten von Sensoren können grundsätzlich eingesetzt werden.

Die Anzeigeelemente 164 können beispielsweise veränderliche Anzeigeelemente 164 umfassen, welche beispielsweise bei einer Nutzung verändert werden können. So kann beispielsweise eine Siegelfolie vorgesehen sein, insbesondere eine metallische Siegelfolie, welche durch eine vorherige Nutzung eingedrückt werden kann. Dann kann beispielsweise ein eingestrahlter Lichtstrahl, beispielsweise ein von den Orientierungssensoren 166 emittierter Lichtstrahl, nicht mehr korrekt reflektiert werden. Analog lassen sich beispielsweise gemessene Kapazitäten (Kondensatorprinzip) und/oder Induktivitäten (Spulenprinzip) verändern und/oder es lassen sich einfache Speicherelemente, vorzugsweise veränderliche Speicherelemente, einsetzen, beispielsweise magnetische Speicherelemente. Auch mechanische Sensoren und/oder Anzeigeelemente 164, wie beispielsweise Microschalter, sind für eine Erkennung geeignet und dem Fachmann grundsätzlich bekannt.

In der Tabelle 1 sind für verschiedene Ausgangssituationen die oben genannten Variablen H, $N_{used}$ und $N_{left}$ exemplarisch dargestellt. Weiterhin sind mögliche Anwenderhinweise dargestellt, die beispielsweise über die Schnittstelle 162, insbesondere über ein oder mehrere Displays, an einen Benutzer ausgegeben werden können. Auch andere Ausgestaltungen sind jedoch grundsätzlich möglich.

| | Ausgangssituation | H | $N_{used}$ | $N_{left}$ | Anwenderhinweis (z.B. auf Display) |
|---|---|---|---|---|---|
| 1. Magazinhälfte | Magazin neu eingelegt | 1 | 0 | 50 | *Neues Magazin erkannt* |
| | 15 Tests verbraucht | 1 | 10 | 35 | -- |
| | 25 Tests verbraucht | 1 | 25 | 25 | *Wenden des Magazins erforderlich* |
| 2. Magazinhälfte | Magazin gewendet und eingesetzt | 0 | 0 | 25 | *Gewendetes Magazin erkannt* |
| | 15 Tests verbraucht | 0 | 10 | 10 | *Achtung, Magazin geht zur Neige* |
| | 25 Tests verbraucht | 0 | 25 | 0 | *Tausch des Magazins erforderlich* |

*Tabelle 1: Beispiel einer Rechenlogik zur Bestimmung des Magazinstatus*

Das analytische Magazin 110 in dem in Figur dargestellten Ausführungsbeispiel ist also als Wendemagazin ausgestaltet und weist zwei Teil-Magazine in Form von Magazinhälften auf, welche im Folgenden mit den Bezugsziffern 172 und 174 bezeichnet werden. Diese Magazinhälften 172, 174 sind im Wesentlichen symmetrisch zueinander ausgestaltet. Beispielsweise geht bei dem analytischen Magazin 110 die erste Magazinhälfte 172 nach einer Drehung um eine Achse parallel zu den Hilfsmittelebenen 118, 120 in die zweite Magazinhälfte 174 über, wobei ggf. noch eine geringfügige Drehung um eine Achse senkrecht zu diesen Hilfsmittelebenen 118, 120, um den Versatzwinkel der beiden Hilfsmittelebenen 118, 120 (d.h. um die Hälfte des Winkels der äquidistanten Winkelverteilung der Kammein 126) zu ergänzen ist.

Das als Wendemagazin ausgestaltete analytische Magazin 110 soll die Sterilität jedes analytischen Hilfsmittels 128, insbesondere die Sterilität jedes Microsamplers 132, bis hin zum Stich gewährleisten. Aus diesem Grund können zumindest die Microsampler 132 in die Kammern 126 eingebracht werden, welche insbesondere als separate Kammern und vorzugsweise als keimundurchlässige Kammern ausgestaltet ist. Diese Aufgabe lässt sich durch entsprechende Verbindungstechniken der einzelnen Teile des analytischen Magazins 110 insbesondere des Gehäuses 122 des analytischen Magazins 110 realisieren. Hierbei können insbesondere Verbindungsverfahren eingesetzt werden, wie beispielsweise Klebeverfahren und/oder Schweißverfahren, insbesondere Ultraschallschweißen und/oder Laserschweißen. Vorzugsweise sollten die dabei verbleibende Spalte, beispielsweise Spalte zwischen benachbarten Kammern 126 und/oder Spalte im Gehäuse 122, Breiten von nicht mehr als $10 \mu m$ aufweisen, um Keime vom Durchtritt abzuhalten. Eine weitere Aufgabe des als Wendemagazin ausgestalteten analytischen Magazins 110 kann in einer Sicherstellung der Aufbrauchfrist für die angebrochene Verpackung gelten. Obige Spaltmaße sind in der Regel nicht geeignet, Feuchtigkeit aus dem Inneren des Gehäuses 122 zurückzuhalten. Aus diesem Grund ist es bevorzugt, für das Wendemagazin eine feuchteverträgliche, stabile Testchemie 142 einzusetzen, welche jedoch mittlerweile verfügbar ist. Beispielsweise kann hierbei eine als carbaNAD-Rezeptur bekannte Testchemie 142 zur optischen Testauswertung eingesetzt werden, welche beispielsweise aus WO 2007/012494 A1 bekannt ist. Alternativ oder zusätzlich können jedoch grundsätzlich dichte Gehäuse 122 verwendet werden und/oder Gehäuse, bei welchen die Kammern 126 untereinander und gegen die Außenatmosphäre durch zusätzliche Versiegelungen abgedichtet sind.

Das analytische Magazin 110 und das analytische System 114 sind voranstehend unter Bezugnahme auf die spezielle Darstellung in Figur 1 beschrieben. Das dargestellte Prinzip lässt sich jedoch problemlos auf andere Typen von Wendemagazinen übertragen, wie beispielsweise die im Folgenden beschriebenen analytischen Magazine 110 in den Figuren 2A bis 3, welche sich im wesentlichen in ihrer äußeren Geometrie unterscheiden. Prinzipiell sind die technischen Merkmale jedoch austauschbar und nicht an die spezielle Magazinform gekoppelt, so dass das oben Gesagte für die nachfolgend beschriebenen Ausführungsbeispiele in analoger Weise gelten kann.

So ist in den Figuren 2A bis 2D ein zweites Ausführungsbeispiel eines erfindungsgemäßen analytischen Magazins 110 in verschiedenen Darstellungen gezeigt. So zeigt Figur 2A eine perspektivische Darstellung in Ansicht schräg

von oben, Figur 2B eine Draufsicht, Figur 2C eine Schnittdarstellung und Figur 2D eine Explosionsdarstellung des analytischen Magazins 110. Wie aus diesen Figuren hervorgeht, handelt es sich bei dem analytischen Magazin 110 in dem zweiten Ausführungsbeispiel ebenfalls um ein rundes Wendemagazin, welches im Wesentlichen die Form einer runden Scheibe aufweist.

Das analytische Magazin 110 ist dabei in den Figuren in geschlossener Darstellung gezeigt und weist wiederum ein Gehäuse 122 mit einzelnen Kammern 126 (in den Figuren durch das Gehäuse 122 verdeckt) auf Bezüglich der einzelnen Bauelemente kann weitgehend auf die obige Beschreibung der Figur 1 verwiesen werden. Die Kammern 126 sind wieder äquidistant in radialer Ausrichtung in dem Gehäuse 122 in zwei Hilfsmittelebenen 118, 120 verteilt, wobei zwischen den beiden Hilfsmittelebenen 118, 120 wiederum ein Verwinkelversatz um die Hälfte eines Verteilungswinkels erfolgt. Dies ist insbesondere aus der Darstellung in Figur 2A erkennbar.

Im Unterschied zum analytischen Magazin 110 gemäß dem in Figur 1 dargestellten Ausführungsbeispiel liegt im Ausführungsbeispiel gemäß den Figuren 2A bis 2D der Microsampler 132 nicht gestreckt in den Kammern 126, sondern ist vorzugsweise an einem Ende aus seiner Ebene herausgebogen. Die Kammern 126 werden jeweils vorzugsweise aus zwei Hartkomponenten gebildet, welche diese Biegung hervorrufen. Der Eingriff in den Microsampler 132, welcher eine Probennahmebewegung und eine Remagazinierung ermöglicht, beispielsweise mittels der Aktorstange 152, erfolgt mittels eines hakenförmigen Stößels der Aktorstange 152, welcher von hinten, d.h. von der Mittelöffnung 124 her, in die Kammer 126 einfährt. Der hakenförmige Stößel schiebt den Microsampler 132 radial nach außen, wobei sich dieser, der Führungskontur der Kammer 126 folgend, in eine gestreckte Lage begibt und so das Pilotloch des (in den Figuren nicht näher bezeichneten) Kopplungselements 138 auf den hakenförmigen Stößel der Aktorstange 152 auffädelt. Beim Rückzug, also bei der Rückholbewegung der Probennahmebewegung und/oder bei der Remagazinierung, wird der Microsampler 132 wieder aus der Ebene herausgebogen und gibt so den hakenförmigen Stößel der Aktorstange 152 wieder frei. Auch andere An- und/oder Abkopplungsmechanismen zur Durchführung der Probennahmebewegung und/oder zur Remagazinierung sind jedoch grundsätzlich möglich.

Die in den Figuren mit der Bezugsziffer 176 bezeichneten Öffnungen am Außenumfang und am Innenumfang des ringförmigen analytischen Magazins 110 sind vorzugsweise mit einer Dichtfolie abgeschlossen, die innen von der Aktorstange 152 und außen vom Microsampler 132 durchstochen werden kann.

In der perspektivischen Darstellung schräg von oben in Figur 2A ist die Verdrehung zwischen den Magazinhälften 172 und 174 um eine halbe Einheit gut erkennbar. Die als Messfenster wirkenden Testelementfenster 168 sind offen dargestellt. Derartig offene Messfenster sind insbesondere wiederum in Verbindung mit einer gegen Umwelteinflüsse stabilen Testchemie 142 von Vorzug.

In der geschnittenen Darstellung gemäß Figur 2C ist die gedoppelte Anordnung der Magazinhälften 172, 174 erkennbar. Durch die Verdrehung der beiden Magazinhälften 172, 174 um eine halbe Einheit ist der Microsampler 132 an unterschiedlichen Positionen bezüglich der Magazinachse zu sehen. Diese Anordnung erlaubt es wiederum, die Kammem 126 auf beiden Seiten der Scheibe so ineinander zu verschachteln, dass trotz geringer Dicke der Scheibe die entstehenden Wandstärken zwischen den Kammern funktional oder produktionstechnisch hinreichend groß sind.

Die in Figur 2C unten dargestellte Kammer 126 befindet sich beispielsweise in der Applikationsposition 154 (vergleiche auch Figur 4). In dieser Darstellung ist der Microsampler 132 in dieser Kammer 126 in der Applikationsposition 154 zumindest teilweise aus der Kammer 126 herausgeschoben. Die Aktorstange 152 eines Aktors 148, welche dies bewirkt, ist in der Darstellung nicht gezeigt. Der Explosionsdarstellung in Figur 2D ist ein bevorzugter Aufbau des analytischen Magazins 110 zu entnehmen. Der Darstellung ist zu entnehmen, dass das Gehäuse 122 im Wesentlichen drei Komponenten umfasst, welche beispielsweise, wie oben dargestellt, miteinander verschweißt werden können. So umfasst das Gehäuse 122 einen oberen Fensterring 178, welcher in einer obersten Teilebene angeordnet ist, sowie einen unteren Fensterring 180, welcher in einer untersten Teilebene angeordnet ist. In einer Mittelebene ist ein Microlanzetten-Ring 182 angeordnet, in welchem Kammern 126 für 2 x 25 Microsampler 132 bzw. Microlanzetten angeordnet sind. Diese sind gegeneinander versetzt jeweils auf der oberen und unteren Seite des Microlanzetten-Rings 182 untergebracht. Die Elemente 178, 180 und 182 können beispielsweise als Kunststoff-Bauteile ausgestaltet sein, beispielsweise als Spritzgieß-Bauteile, beispielsweise aus einem oder mehreren thermoplastischen Kunststoffen.

An den Microlanzetten-Ring 182 schließt sich nach oben bzw. nach unten jeweils in weiteren Ebenen zwischen Microlanzetten-Ring 182 und den Fensterringen 178, 180 jeweils ein identischer Chemie-Ring 184 an. Diese Chemieringe 184 können beispielsweise eine Trägerfolie aufweisen, beispielsweise eine dünne Kunststofffolie, welche auf der den Kammern 126 zuweisenden Seite mit der Testchemie 142 bedeckt ist. Der Chemie-Ring 184 bildet somit ein durchgehendes Testfeld 144 für alle Kammern 126 einer Hilfsmittelebene 118, 120, wobei die den Kammern 126 zuweisenden Bereiche dieses Testfeldes 144 jeweils einzelne Testfelder 144 und somit Teil-Hilfsmittel 130 eines analytischen Hilfsmittels 128 bilden.

Um den Transfer der Probe von dem Kapillarelement 136 der Microsampler 132 (in den Figuren nicht dargestellt) auf die Testchemie 142 der Testfelder 144 in den Kammern 126 zu übertragen, kann wiederum, ähnlich zur Darstellung in Figur 4, ein Andrück-Mechanismus vorgesehen sein. Dieses Andrücken kann beispielsweise wiederum durch eine separate Andrücköffnung erfolgen. Bevorzugt ist es jedoch, das Andrücken derart vorzunehmen, dass dieses von der

Seite der Testelementfenster 168 her erfolgt, also von der gleichen Seite, von der aus auch die Messung vorgenommen wird. Eine geringe Folienstärke der Trägerfolie der Chemie-Ringe 184 erlaubt es, diese in ihrem Testelementfenster 168 aufgespannte Membran mit geringer Kraft auf den in seiner Kammer 126 liegenden Microsampler 132 zu drücken, damit ein Kontakt zwischen der gesammelten Probe und der Testchemie 142 stattfindet.

Das analytische Magazin 110 wird nach außen hin durch die beiden, beispielsweise im Wesentlichen identisch ausgestalteten, Fensterringe 178 und 180 abgeschlossen. Es können jedoch auch zusätzliche Dichtelemente vorgesehen sein. Beispielsweise können am äußeren und am inneren Umfang Dichtfolien aufgebracht sein, welche die Kammeröffnungen 176 (siehe Figur 2A) verschließen. Diese sind in den Figuren nicht dargestellt.

Der Vorteil der Anordnung gemäß den Figuren 2A bis 2D liegt insbesondere in einer einfachen Herstellung der verschiedenen Elemente des Gehäuses 122. Sowohl der Microlanzetten-Ring 182 als auch die Fensterringe 178, 180 können als einfache Spritzgussbauteile hergestellt sein, welche sich in großer Stückzahl und mit hoher Konstanz fertigen lassen. Die Chemie-Ringe 184 können beispielsweise aus einer gängigen Beschichtungsfolie ausgestanzt werden. Alle Ringe lassen sich beispielsweise über eine zentrale Achse zentrieren, ausrichten und montieren.

Die einzelnen Ringelemente des Gehäuses 122 sind vorzugsweise derart ausgelegt, dass diese sich mit gängigen Herstellungsverfahren verbinden lassen und dabei möglichst kleine Spalte erzeugen. Insbesondere lassen sich wiederum Spaltbreiten herstellen, welche nicht größer sind als 10$\mu$m, Spalte unterhalb des genannten Durchmessers bzw. der genannten Breite zeichnen sich durch ihre Keimundurchlässigkeit aus und schließen somit eine Kreuzkontamination zwischen benutzten und unbenutzten Kammern 126 bzw. Microsamplern 132 zumindest weitgehend aus. Typische Verbundverfahren wie Kleben, Ultraschallschweißen, Laserschweißen, Kaltverstemmen oder ähnliche genannte Techniken sind im Stand der Technik grundsätzlich bekannt und können auch im Rahmen der vorliegenden Erfindung eingesetzt werden. Darüber hinaus können auch zusätzliche Hilfsstrukturen auf den Magazinteilen des Gehäuses 122 aufgebracht werden, wenn diese für das jeweilige Verbundverfahren von Vorteil sind.

In Figur 3 ist ein drittes Ausführungsbeispiel eines analytischen Magazins 110 dargestellt, welches wiederum beispielsweise in einem analytischen System in ähnlicher Ausgestaltung wie dem in Figur 4 gezeigten analytischen System 114 eingesetzt werden kann. Es handelt sich bei dem hier in perspektivischer Darstellung gezeigten analytischen Magazin 110 um ein lineares Wendemagazin.

Die zuvor gezeigten Ausführungsformen im Wesentlichen unter Bezugnahme auf analytische Hilfsmittel 128 mit optischer Testchemie 142 beschrieben. Alternativ oder zusätzlich zu einer optischen Auswertung können jedoch auch andere Nachweisverfahren eingesetzt werden. So kann das in Figur 3 gezeigte analytische Magazin 110 beispielsweise für analytische Hilfsmittel 128 eingesetzt werden, welche nach einem elektrochemischen Messverfahren ausgewertet werden. Die optische Auswertung und somit das Testelementfenster 168 kann somit entfallen.

Die analytischen Hilfsmittel 128 umfassen wiederum Microsampler 132 mit Lanzetten 134, welche den in Figur 3 nach vorne weisenden Kammeröffnungen 176 zuweisen. Beispielsweise sind diese Microsampler 132 auf streifenförmigen Trägern angeordnet. Ein Kapillarelement 136 der Microsampler 132 kann aufgenommenes Blut auf Testelemente 140 mit einer Testchemie 142 in Form von Testfeldern 144 übertragen, welche über elektrische Kontakte 186, beispielsweise an einem der Lanzette 134 gegenüberliegenden Ende der streifenförmigen analytischen Hilfsmittel 128, angeordnet sein können.

Eine Ankopplungsmechanik, welche an in Figur 3 nicht näher dargestellten Kopplungselementen 138 der analytischen Hilfsmittel 128 ankoppelt, um die Probennahmebewegung und/oder die Remagazinierung zu bewirken, kann auch eine elektrische Verbindung zu dem analytischen Hilfsmittel 128 herstellen. Alternativ können auch zusätzliche Elemente zum Herstellen dieser elektrischen Verbindungen vorgesehen sein. Über die elektrische Kopplung kann ein Strom, welcher an elektrochemischen Kontakten der Testelemente 140 anliegt, ausgewertet werden. Das Messsystem 158 in Figur 4 kann beispielsweise für derartige Messungen eingerichtet sein. Derartige elektrochemische Messungen sind aus dem Stand der Technik bekannt, beispielsweise dem oben genannten Stand der Technik, und sollen hier nicht näher vertieft werden. Beispielhaft ist ein analytisches Hilfsmittel 128 in Figur 3 in Form eines elektrochemischen Microsamplers in vertikaler Ausrichtung in dem analytischen Magazin 110 dargestellt. Auch andere Anordnungen sind jedoch grundsätzlich möglich.

Die Ausführungsform in Figur 3 zeigt weiterhin, dass ein Versatz zwischen den Kammern 126 in der oberen und der unteren Magazinhälfte 172, 174 nicht unbedingt erforderlich ist. In dieser Ausführungsform sind exemplarisch beide Magazinhälften 172, 174 spiegelsymmetrisch zueinander ausgeführt. Dies hat insbesondere den Vorteil, dass eine Magazinpositionierung beispielsweise allein über das Gehäuse 122 des analytischen Magazins 110 erfolgen kann und dass beispielsweise auch zusätzliche Positionierhilfen entfallen können.

Ein Wenden des analytischen Magazins 110, d.h. ein Wechsel einer Orientierung, zum Wechsel der jeweils verwendeten Magazinhälfte 172, 174 kann dabei auf verschiedene Weisen erfolgen. Die Anordnung der analytischen Hilfsmittel 128 in den Kammern 126 kann dieser bestimmten Art des Wechsels der Orientierung bzw. des Wendens angepasst sein. So können beispielsweise in beiden Magazinhälften 172, 174 bzw. in beiden Hilfsmittelebenen 118, 120 die analytischen Hilfsmittel 128 gleich orientiert sein, beispielsweise mit einer zur vorderen Kammeröffnung 176 in Figur 3 hinweisenden Lanzette 134. In diesem Fall erfolgt ein Wenden um eine Achse, welche parallel zu den analytischen

Hilfsmittel 128 und parallel zu den Hilfsmittelebenen 118, 120 orientiert ist, beispielsweise eine senkrecht zur schmalen Vorderseite in Figur 3 verlaufende Achse. Das analytische Magazin 110 kann somit über eine schmale Kante gewendet werden.

Alternativ lassen sich die analytischen Hilfsmittel 128 in den Magazinhälften 172, 174 auch in unterschiedlicher Weise anordnen, beispielsweise indem in der zweiten Magazinhälfte 174 die Lanzetten 134 in Figur 3 nach hinten weisen. In diesem Fall kann das analytische Magazin 110 beispielsweise beim Wenden um eine senkrecht zu den analytischen Hilfsmitteln 128 und parallel zu den Hilfsmittelebenen 118, 120 verlaufende Achse gedreht werden, beispielsweise eine parallel zur Längserstreckung des linearen Wendemagazins verlaufende Achse.

Ein analytisches System 114, welches mit dem analytischen Magazin 110 gemäß Figur 3 zusammenwirkt, kann grundsätzlich analog zur Darstellung in Figur 4 ausgestaltet sein. Insbesondere können wiederum Aktoren 148, gegebenenfalls mit einer oder mehreren Aktorstangen 152 und/oder anderen Arten von Kopplungselementen, vorgesehen sein, welche an die analytischen Hilfsmittel 128 ankoppeln können. Beispielsweise kann wiederum eine Applikationsposition 154 vorgesehen sein, in welcher die Kopplung erfolgt. Mittels dieser Aktoren 148 kann dann wiederum eine Probennahmebewegung erfolgen, einschließlich einer entsprechenden Remagazinierung. Entsprechend können die analytischen Hilfsmittel 128 wiederum Kopplungselemente 138 umfassen, beispielsweise wiederum Pilotöffnungen, Ösen oder ähnliches, an welche der Aktor 148 und/oder eine Aktorstange 152 des Aktors 148 mechanisch ankoppeln kann. Wie oben beschrieben, kann die mechanische Ankopplung auch optional mit einer elektrischen Ankopplung kombiniert werden.

Bezugszeichenliste

| | |
|---|---|
| 110 | analytisches Magazin |
| 112 | Gehäuse (System) |
| 114 | analytisches System |
| 116 | Fingeröffnung |
| 118 | 1. Hilfsmittelebene |
| 120 | 2. Hilfsmittelebene |
| 122 | Gehäuse (Magazin) |
| 124 | Mittelöffnung |
| 126 | Kammern |
| 128 | analytische Hilfsmittel |
| 130 | Teil-Hilfsmittel |
| 132 | Microsampler |
| 134 | Lanzette |
| 136 | Kapillarelement |
| 138 | Kopplungselement |
| 140 | Testelement |
| 142 | Testchemie |
| 144 | Testfeld |
| 146 | Positionierungsvorrichtung |
| 148 | Aktor |
| 150 | Aktor |
| 152 | Aktorstange |
| 154 | Applikationsposition |
| 156 | Aktorstange |
| 158 | Messsystem |
| 160 | Steuerung |
| 162 | Schnittstelle |
| 164 | Anzeigeelement |
| 166 | Orientierungssensor |
| 168 | Testelementfenster |
| 170 | Applikationsebene |
| 172 | erste Magazinhälfte |
| 174 | zweite Magazinhälfte |
| 176 | Kammeröffnungen |
| 178 | oberer Fensterring |

180 unterer Fensterring
182 Microlanzetten-Ring
184 Chemie-Ring
186 elektrische Kontakte

**Patentansprüche**

1. Analytisches Magazin (110), wobei das analytische Magazin (110) mindestens zwei Kammern (126) aufweist, in denen analytische Hilfsmittel (128) aufnehmbar sind, wobei die analytischen Hilfsmittel (128) in den Kammern (126) aufgenommen sind, wobei die analytischen Hilfsmittel (128) jeweils mindestens einen Microsampler (132) zum Erzeugen mindestens einer Öffnung in einer Haut eines Probanden und mindestens ein Testelement (140) mit mindestens einer Testchemie (142) zum Nachweis mindestens eines Analyten in einer Körperflüssigkeit umfassen, wobei die Testchemie (142) zumindest weitgehend stabil ist gegenüber Feuchtigkeit, wobei die Testchemie (142) ein Enzym und ein stabiles Coenzym umfasst, wobei das Enzym und das stabile Coenzym gemeinsam gelagert sind, wobei die Stabilität dergestalt ist, dass bei einer Lagerung bei 32 °C, einer relativen Luftfeuchtigkeit von 85% bei Normaldruck über eine Zeitdauer von drei Wochen eine Aktivität der Testchemie (142) des analytischen Hilfsmittels (128) um weniger als 50% abnimmt, wobei das analytische Magazin (110) eine Packungsdichte der analytischen Hilfsmittel (128) von mehr als 5 analytischen Hilfsmitteln (128) pro $cm^3$ aufweist, wobei das Magazin als Wendemagazin ausgestaltet ist, welches in mindestens zwei Orientierungen in ein analytisches System (114) einfügbar ist.

2. Analytisches Magazin (110) nach dem vorhergehenden Anspruch, wobei das analytische Magazin (110) ein Gehäuse (112) aufweist, wobei das Gehäuse (112) eine Wandstärke von nicht mehr als 1,2 mm aufweist.

3. Analytisches Magazin (110) nach einem der vorhergehenden Ansprüche, wobei das analytische Magazin (110) Trockenmittel-frei ausgestaltet ist.

4. Analytisches Magazin (110) nach einem der vorhergehenden Ansprüche, wobei das analytische Magazin (110) ein Gehäuse (122) mit mindestens zwei Teilen aufweist, insbesondere mindestens zwei Magazinhälften (172, 174), wobei die mindestens zwei Teile miteinander durch ein Laserschweißverfahren verbunden sind, wobei mindestens eine Schweißnaht mit einer Schweißnahtbreite von maximal 0,5 mm vorgesehen ist.

5. Analytisches Magazin (110) nach einem der vorhergehenden Ansprüche, wobei das analytische Magazin (110) ein Gehäuse (122) aufweist, insbesondere ein Gehäuse (122) mit mindestens zwei Teilen, wobei das Gehäuse (122) einen Werkstoff aufweist, ausgewählt aus den folgenden Werkstoffen: ein Polycarbonat; ein AcrylnitrilButadien-Styrol; ein Cyclo-Olefin-Copolymer; ein Polymethylmethacrylat; ein Polystyrol; ein Polyethylenterephthalat.

6. Analytisches Magazin (110) nach einem der vorhergehenden Ansprüche, wobei das analytische Magazin (110) eingerichtet ist, um in den Orientierungen jeweils eine Mehrzahl analytischer Hilfsmittel (128) an das analytische System (114) bereitzustellen, wobei mittels der analytischen Hilfsmittel (128) mindestens eine Probennahmebewegung durchführbar ist, wobei die analytischen Hilfsmittel (128) mindestens ein Transferelement zum Aufnehmen einer Probe einer Körperflüssigkeit und/oder zum Transferieren der Probe umfassen, wobei das analytische Magazin (110) eingerichtet ist, um eine Remagazinierung der analytischen Hilfsmittel (128) zu ermöglichen, wobei das analytische Magazin (110) eingerichtet ist, um die analytischen Hilfsmittel (128) nach der Remagazinierung in den Kammern (126) zu haltern, wobei das analytische Magazin (110) mindestens zwei im Wesentlichen gleiche Teil-Magazine (172, 174) umfasst, wobei jedes der Teil-Magazine (172, 174) eine Mehrzahl gleichartiger analytischer Hilfsmittel (128) umfasst.

7. Analytisches Magazin (110) nach dem vorhergehenden Anspruch, wobei das analytische Magazin (110) zumindest teilweise mit einer Symmetrie ausgestattet ist, insbesondere einer Symmetrie um mindestens eine Symmetrieachse.

8. Analytisches Magazin (110) nach einem der beiden vorhergehenden Ansprüche, wobei das analytische Magazin (110) mindestens ein Anzeigeelement (164) umfasst, vorzugsweise eine der Anzahl möglicher Orientierungen entsprechende Anzahl von Anzeigeelementen (164), wobei das Anzeigeelement (164) ausgestaltet ist, um von dem analytischen System (114) erfasst zu werden und mindestens eine Information über eine aktuelle Orientierung an das analytische System (114) zu liefern.

**9.** Analytisches Magazin (110) nach einem der drei vorhergehenden Ansprüche, wobei das analytische Magazin (110) eine Form einer runden Scheibe aufweist, wobei die analytischen Hilfsmittel (128) in dem analytischen Magazin (110) in radialer Anordnung ausgerichtet sind, wobei die analytischen Hilfsmittel (128) in unterschiedlichen Hilfsmittelebenen (118, 120) winkelversetzt zueinander angeordnet sind.

**10.** Analytisches Magazin (110) nach einem der vier vorhergehenden Ansprüche, wobei das analytische Magazin (110) eingerichtet ist, um in allen Orientierungen einen Zugang zu einer in einer Applikationsposition (154) des analytischen Systems (114) befindlichen Kammer (126) und/oder mindestens eines in dieser Kammer (126) aufgenommenen analytischen Hilfsmittels (128) aus mindestens zwei Richtungen, insbesondere von einander gegenüberliegenden Richtungen aus, zu ermöglichen.

**11.** Analytisches Magazin (110) nach einem der fünf vorhergehenden Ansprüche, wobei die analytischen Hilfsmittel (128) zumindest teilweise eingerichtet sind, um bei der Probennahmebewegung eine Probe einer Körperflüssigkeit aufzunehmen und zu einem Testelement (140) zu transferieren, wobei der Transfer der Probe insbesondere zumindest teilweise bei der Remagazinierung des analytischen Hilfsmittels (128) erfolgt.

**12.** Analytisches Magazin (110) nach einem der sechs vorhergehenden Ansprüche, wobei die Testchemie (142) zumindest teilweise in ein Gehäuse (122) des analytischen Magazins (110) integriert ist, insbesondere eine Wand der Kammern (126).

**13.** Analytisches Magazin (110) nach einem der sieben vorhergehenden Ansprüche, wobei die Kammern (126) derart gestaltet sind, dass die Lanzetten (134) in den Kammern (126) gebogen werden und durch eine Rückstellkraft gegen eine Kammerwand gepresst werden.

## Claims

**1.** Analytical magazine (110), wherein the analytical magazine (110) has at least two chambers (126), in which analytical aids (128) can be accommodated, wherein the analytical aids (128) are accommodated in the chambers (126), wherein the analytical aids (128) in each case comprise at least one microsampler (132) for making at least one opening in a test subject's skin and at least one test element (140) with at least one test chemistry (142) for detecting at least one analyte in a body fluid, wherein the test chemistry (142) is at least largely stable against moisture, wherein the test chemistry (142) comprises an enzyme and a stable coenzyme, wherein the enzyme and the stable coenzyme are stored together, wherein the stability is such that when stored at 32°C, a relative humidity of the air of 85% at normal pressure for a period of three weeks, an activity of the test chemistry (142) of the analytical aid (128) decreases by less than 50%, wherein the analytical magazine (110) has a packing density of the analytical aids (128) of more than 5 analytical aids (128) per $cm^3$, wherein the magazine is configured as a reversing magazine that can be inserted into an analytical system (114) in at least two orientations.

**2.** Analytical magazine (110) according to the preceding claim, wherein the analytical magazine (110) has a housing (112), wherein the housing (112) has a wall thickness of not more than 1.2 mm.

**3.** Analytical magazine (110) according to one of the preceding claims, wherein the analytical magazine (110) is of drying agent-free design.

**4.** Analytical magazine (110) according to one of the preceding claims, wherein the analytical magazine (110) has a housing (122) with at least two parts, in particular at least two magazine halves (172, 174), wherein the at least two parts are joined together by a laser welding process, wherein at least one weld seam is provided with a weld seam width of max. 0.5 mm.

**5.** Analytical magazine (110) according to one of the preceding claims, wherein the analytical magazine (110) has a housing (122), in particular a housing (122) with at least two parts, wherein the housing (122) has a material selected from the following materials: a polycarbonate; an acrylonitrile-butadiene-styrene; a cyclo-olefin copolymer; a polymethylmethacrylate; a polystyrene; a polyethylene terephthalate.

**6.** Analytical magazine (110) according to one of the preceding claims, wherein the analytical magazine (110) is arranged to supply, in the orientations, in each case a plurality of analytical aids (128) to the analytical system (114), wherein by means of the analytical aids (128) at least one sampling movement can be executed, wherein the

analytical aids (128) comprise at least one transfer element for receiving a sample of a body fluid and/or for transferring the sample, wherein the analytical magazine (110) is arranged to make a remagazining of the analytical aids (128) possible, wherein the analytical magazine (110) is arranged to hold the analytical aids (128) in place in the chambers (126) after remagazining, wherein the analytical magazine (110) comprises at least two essentially identical partial magazines (172, 174), wherein each of the partial magazines (172, 174) comprises a plurality of similar analytical aids (128).

**7.** Analytical magazine (110) according to the preceding claim, wherein the analytical magazine (110) is provided at least partially with a symmetry, in particular a symmetry about at least one axis of symmetry.

**8.** Analytical magazine (110) according to one of the two preceding claims, wherein the analytical magazine (110) comprises at least one indicating element (164), preferably a number of indicating elements (164) corresponding to the number of possible orientations, wherein the indicating element (164) is designed to be detected by the analytical system (114) and to supply at least one piece of information about a current orientation to the analytical system (114).

**9.** Analytical magazine (110) according to one of the three preceding claims, wherein the analytical magazine (110) has a shape of a round disk, wherein the analytical aids (128) are aligned in a radial arrangement in the analytical magazine (110), wherein the analytical aids (128) are arranged in different levels of aids (118, 120) with an angular offset relative to one another.

**10.** Analytical magazine (110) according to one of the four preceding claims, wherein the analytical magazine (110) is arranged to permit access in all orientations to a chamber (126) located in an application position (154) of the analytical system (114) and/or at least one analytical aid (128) accommodated in said chamber (126) from at least two directions, in particular from directions opposite to one another.

**11.** Analytical magazine (110) according to one of the five preceding claims, wherein the analytical aids (128) are arranged at least partially to collect a sample of a body fluid during the sampling movement and transfer it to a test element (140), wherein sample transfer takes place in particular at least partially during remagazining of the analytical aid (128).

**12.** Analytical magazine (110) according to one of the six preceding claims, wherein the test chemistry (142) is integrated at least partially in a housing (122) of the analytical magazine (110), in particular a wall of the chambers (126).

**13.** Analytical magazine (110) as claimed in one of the seven preceding claims, wherein the chambers (126) are configured such that the lancets (134) can be bent in the chambers (126) and are pressed against a chamber wall by a restoring force.

## Revendications

**1.** Magasin d'analyse (110), le magasin d'analyse (110) présentant au moins deux chambres (126) dans lesquelles des agents auxiliaires d'analyse (128) peuvent être repris, les agents auxiliaires d'analyse (128) étant repris dans les chambres (126), chacun des agents auxiliaires d'analyse (128) comprenant au moins un micro-échantillonneur (132) qui forme au moins une ouverture dans la peau d'un échantillon et au moins un élément de test (140) doté d'au moins une chimie de test (142) permettant de détecter au moins un analyte dans un liquide corporel, la chimie de test (142) étant au moins largement stable vis-à-vis de l'humidité, la chimie de test (142) comprenant une enzyme et une coenzyme stable, l'enzyme et la coenzyme stable étant conservées ensemble, la stabilité étant telle que lors d'un entreposage à 32°C à une humidité relative de l'air de 85 % sous pression normale et une durée de trois semaines, l'activité de la chimie de test (142) de l'agent auxiliaire d'analyse (128) diminue de moins de 50 %, le magasin d'analyse (110) présentant une densité d'empilement des agents auxiliaires d'analyse (128) supérieure à 5 agents auxiliaires d'analyse (128) par $cm^3$, le magasin étant configuré comme magasin rotatif qui peut être inséré dans un système analytique (114) dans au moins deux orientations.

**2.** Magasin d'analyse (110) selon la revendication précédente, dans lequel le magasin d'analyse (110) présente un boîtier (112), le boîtier (112) présentant une paroi d'une épaisseur non supérieure à 1,2 mm.

**3.** Magasin d'analyse (110) selon l'une des revendications précédentes, dans lequel le magasin d'analyse (110) ne

présente pas d'agent de séchage.

4. Magasin d'analyse (110) selon l'une des revendications précédentes, dans lequel le magasin d'analyse (110) présente un boîtier (122) doté d'au moins deux parties, en particulier d'au moins deux moitiés (172, 174) de magasin, les deux ou plusieurs parties étant reliées les unes aux autres par un procédé de soudage au laser, au moins un cordon de soudure d'une largeur d'au plus 0,5 mm étant prévu.

5. Magasin d'analyse (110) selon l'une des revendications précédentes, dans lequel le magasin d'analyse (110) présente un boîtier (122), en particulier un boîtier (122) doté d'au moins deux parties, le boîtier (122) présentant un matériau sélectionné parmi les matériaux suivants : un polycarbonate, un acrylonitrile-butadiène-styrène, un copolymère de cyclooléfine, un poly(méthacrylate de méthyle), un polystyrène et un poly(téréphtalate d'éthylène).

6. Magasin d'analyse (110) selon l'une des revendications précédentes, dans lequel le magasin d'analyse (110) est conçu pour présenter au système analytique (114) plusieurs agents auxiliaires d'analyse (128) dans chacune des orientations, au moins un déplacement de prise d'échantillon pouvant être réalisé au moyen des agents auxiliaires d'analyse (128), les agents auxiliaires d'analyse (128) comprenant au moins un élément de transfert qui reprend un échantillon de liquide corporel et/ou qui transfère l'échantillon, le magasin d'analyse (110) étant conçu pour permettre de replacer les agents auxiliaires d'analyse (128) dans lemagasin, le magasin d'analyse (110) étant conçu pour maintenir les agents auxiliaires d'analyse (128) dans les chambres (126) après qu'ils ont été replacés dans le magasin, le magasin d'analyse (110) comprenant au moins deux parties (172, 174) de magasin essentiellement identiques, chacune des deux parties (172, 174) comprenant plusieurs agents auxiliaires d'analyse (128) identiques.

7. Magasin d'analyse (110) selon la revendication précédente, dans lequel le magasin d'analyse (110) est configuré au moins en partie avec une symétrie et en particulier une symétrie autour d'au moins un axe de symétrie.

8. Magasin d'analyse (110) selon l'une des deux revendications qui précèdent, dans lequel le magasin d'analyse (110) comporte au moins un élément d'affichage (164), de préférence un nombre d'éléments d'affichage (164) qui correspond au nombre des orientations possibles, l'élément d'affichage (164) étant configuré pour être saisi par le système analytique (114) et fournir au système analytique (114) au moins une information sur l'orientation effective.

9. Magasin d'analyse (110) selon l'une des trois revendications précédentes, dans lequel le magasin d'analyse (110) présente la forme d'un disque circulaire, les agents auxiliaires d'analyse (128) étant orientés radialement dans le magasin d'analyse (110), les agents auxiliaires d'analyse (128) étant disposés à un décalage angulaire mutuel dans différents plans (118, 120) d'auxiliaires.

10. Magasin d'analyse (110) selon l'une des quatre revendications qui précèdent, dans lequel le magasin d'analyse (110) est conçu pour permettre dans toutes les orientations un accès à une chambre (126) située en une position d'application (154) du système analytique (114) et/ou du ou des agents auxiliaires d'analyse (128) repris dans cette chambre (126) dans au moins deux directions et en particulier dans des directions mutuellement opposées.

11. Magasin d'analyse (110) selon l'une des cinq revendications qui précèdent, dans lequel au moins certains des agents auxiliaires d'analyse (128) sont conçus pour, lors du déplacement de prise d'échantillon, reprendre un échantillon de liquide corporel et le transférer vers un élément de test (140), le transfert de l'échantillon s'effectuant au moins en partie lors du replacement des agents auxiliaires d'analyse (128) dans le magasin.

12. Magasin d'analyse (110) selon l'une des six revendications qui précèdent, dans lequel au moins une partie de la chimie de test (142) est intégrée dans un boîtier (122) du magasin d'analyse (110), en particulier dans une paroi des chambres (126).

13. Magasin d'analyse (110) selon l'une des sept revendications qui précèdent, dans lequel les chambres (126) sont configurées de telle sorte que les lancettes (134) sont courbées dans les chambres (126) et sont repoussées par une force de rappel contre une paroi de la chambre.

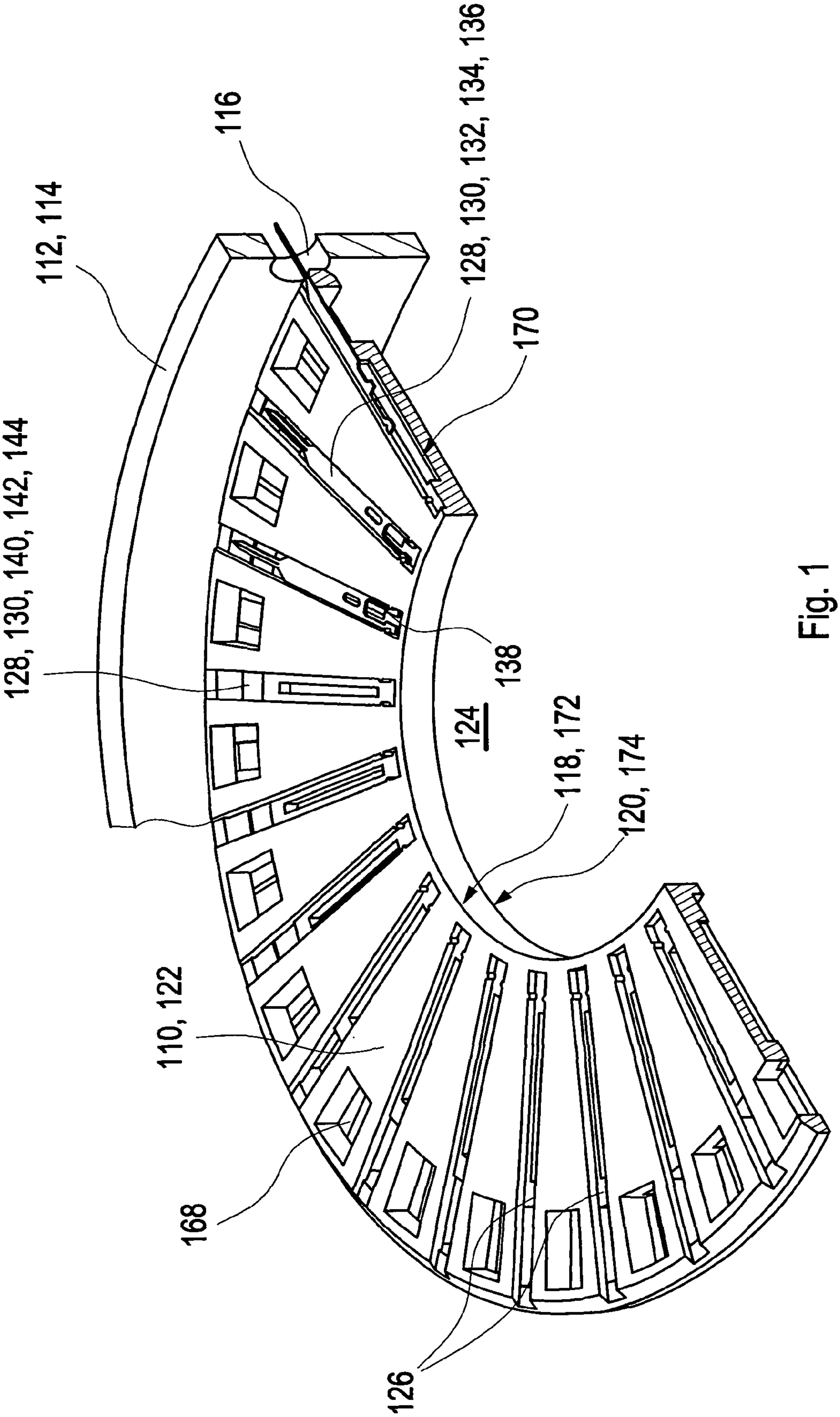
112, 114
116
128, 130, 132, 134, 136
170
128, 130, 140, 142, 144
138
128, 130
124
118, 172
120, 174
110, 122
168
126

Fig. 1

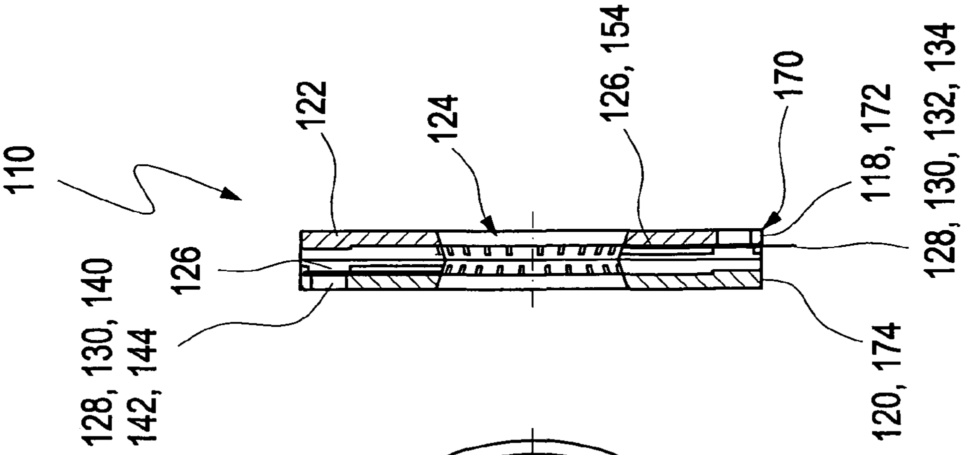


Fig. 2 C

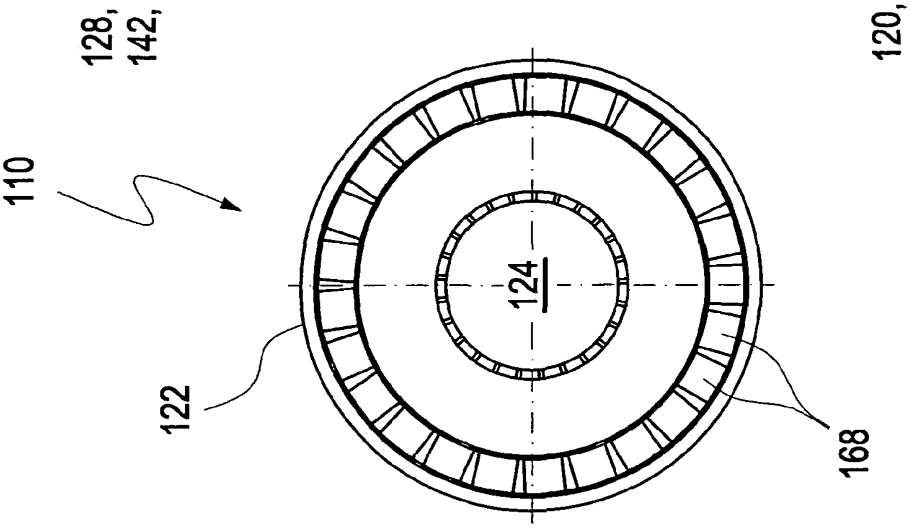


Fig. 2 B

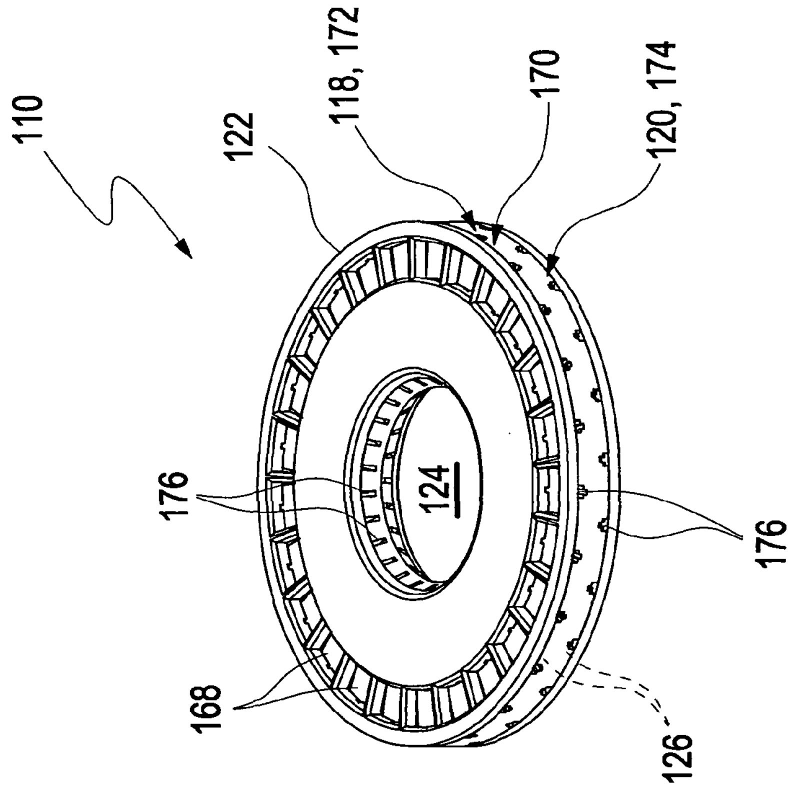


Fig. 2 A

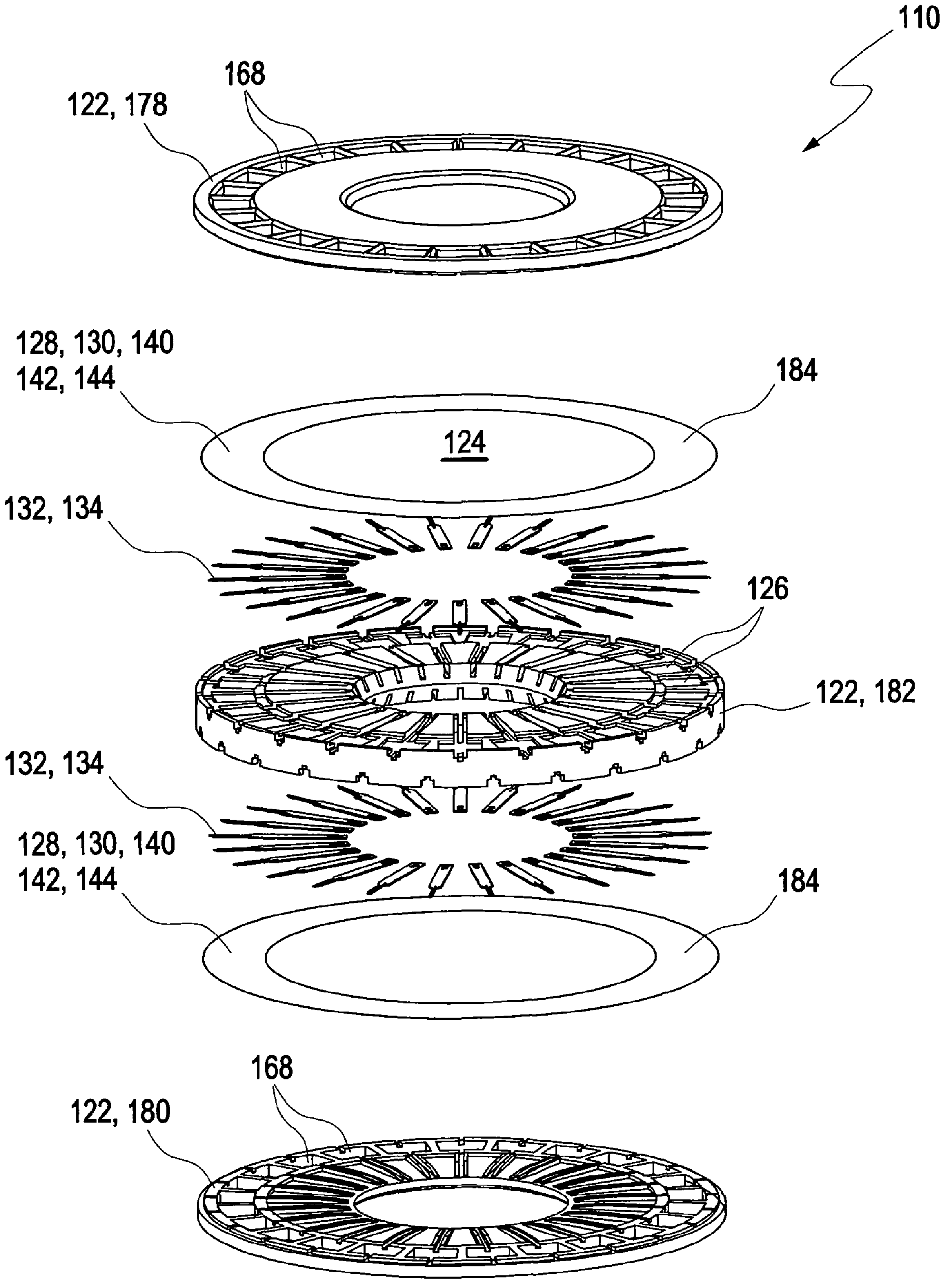
110
168
122, 178
128, 130, 140
142, 144
184
124
132, 134
126
122, 182
132, 134
128, 130, 140
142, 144
184
168
122, 180

Fig. 2 D

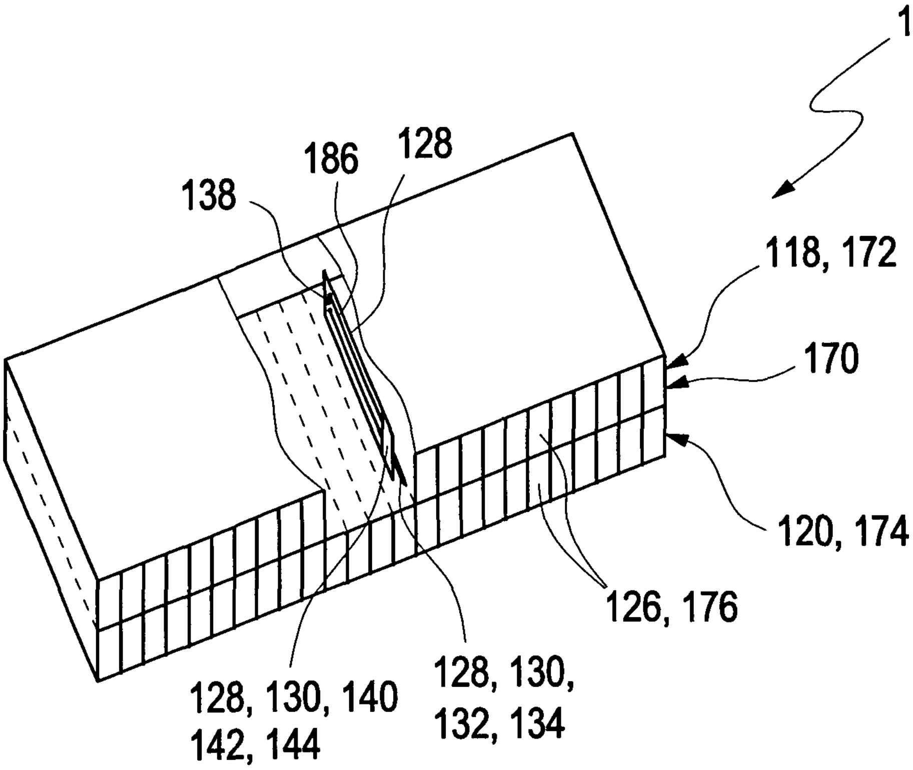
110
186
128
138
118, 172
170
120, 174
126, 176
128, 130, 140
142, 144
128, 130,
132, 134

Fig. 3

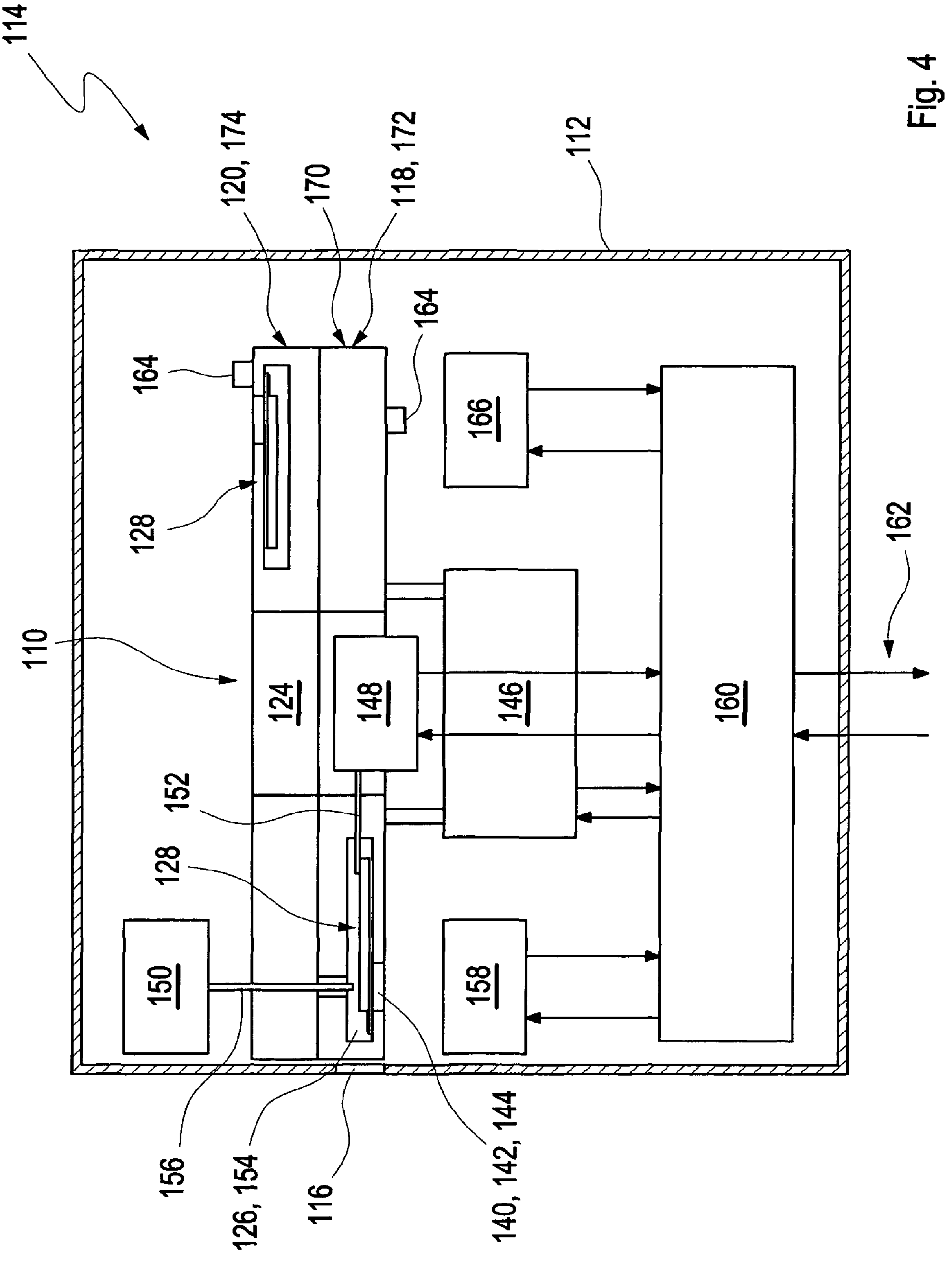
114
120, 174
170
118, 172
112
164
164
166
128
162
110
124
148
146
160
152
128
150
158
156
126, 154
116
140, 142, 144

Fig. 4

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20060008389 A
- US 20070292314 A
- US 20060184064 A
- US 20030212347 A
- US 20020087056 A
- US 20040138543 A
- US 20070129650 A
- US 6036924 A
- US 20030191415 A
- US 20020188224 A
- US 20080103415 A
- EP 1360935 A1
- DE 19819407 A1
- US 20060264996 A
- WO 2007012494 A1
- US 20060008389 A1
- EP 08003054
- EP 2009001206 W
- WO 2007012494 A
- US 11460366 B
- US 20050214891 A


### In der Beschreibung aufgeführte Nicht-Patentliteratur


- **J. HÖNES et al.** *Diabetes Technology & Therapeutics,* 2008, vol. 10 (1), S-10-S-26
- Methoden der enzymatischen Analyse. **H. U. BERGMEYER.** Verlag Chemie. 1970, 417
- **BANAUCH et al.** A glucose dehydrogenase for the determination of glucose concentrations in body fluids. *Z. Klin. Chem. Klin. Biochem.,* Marz 1975, vol. 13 (3), 101-7